# EUROPEAN PATENT SPECIFICATION

(11) **EP 2 655 610 B1**
(45) Date of publication and mention of the grant of the patent: **16.11.2016**
(21) Application number: 11807927.6
(22) Date of filing: 23.12.2011
(51) Int. Cl.: C12N 9/36, C12N 15/62, A61K 38/00

(54) **ANTIMICROBIAL AGENTS**
ANTIMIKROBIELLE MITTEL
AGENTS ANTIMICROBIENS

(30) Priority: 23.12.2010 EP 10196890
(43) Date of publication of application: 30.10.2013
(73) Proprietor: Lysando AG, 9497 Triesenberg (LI); Katholieke Universiteit Leuven, 3000 Leuven (BE)
(72) Inventor: BRIERS, Yves, Nederokkerzeel 1910 (BE); LAVIGNE, Rob, 2170 Merksem (BE); WALMAGH, Maarten, 3540 Herk-de-Stad (BE); MILLER, Stefan, 93055 Regensburg (DE)
(74) Representative: Rückerl, Florian
(86) International application number: PCT/EP2011/073908
(87) International publication number: WO 2012/085259

(56) References cited:
- WO-A1-2010/149795
- WO-A1-2011/023702
- WO-A1-2011/134998
- WO-A2-2007/022768
- WO-A2-2010/011960
- WO-A2-2010/023207
- WO-A2-2010/149792
- US-B1- 7 572 602
- ARIMA H ET AL: "Bactericidal action of lysozymes attached with various sizes of hydrophobic peptides to the C-terminal using genetic modification", FEBS LETTERS, ELSEVIER, AMSTERDAM, NL, vol. 415, no. 1, 22 September 1997 (1997-09-22), pages 114-118, XP004261147, ISSN: 0014-5793, DOI: DOI:10.1016/S0014-5793(97)01071-5
- IBRAHIM H R ET AL: "Enhanced bactericidal action of lysozyme to Escherichia coli by inserting a hydrophobic pentapeptide into its C terminus", JOURNAL OF BIOLOGICAL CHEMISTRY, THE AMERICAN SOCIETY OF BIOLOGICAL CHEMISTS, INC., BALTIMORE, MD, US, vol. 269, no. 7, 18 February 1994 (1994-02-18), pages 5059-5063, XP002579297, ISSN: 0021-9258
- DONOVAN D M ET AL: "Peptidoglycan hydrolase fusions maintain their parental specificities", APPLIED AND ENVIRONMENTAL MICROBIOLOGY, AMERICAN SOCIETY FOR MICROBIOLOGY, US, vol. 72, no. 4, 1 April 2006 (2006-04-01), pages 2988-2996, XP002582772, ISSN: 0099-2240, DOI: DOI:10.1128/AEM.72.4.2988-2996.2006
- VAN DER LINDEN DANITSJA S ET AL: "Synergistic effects of ovine-derived cathelicidins and other antimicrobials against Escherichia coli O157:H7 and Staphylococcus aureus 1056 MRSA", BIOTECHNOLOGY LETTERS, SPRINGER NETHERLANDS, DORDRECHT, vol. 31, no. 8, 1 August 2009 (2009-08-01) , pages 1265-1267, XP002604248, ISSN: 1573-6776, DOI: DOI:10.1007/S10529-009-0010-9 [retrieved on 2009-04-26]
- DATABASE UniProt [Online] 1 April 1990 (1990-04-01), "RecName: Full=Lysozyme; EC=3.2.1.17; AltName: Full=CP-1 lysin; AltName: Full=Endolysin; AltName: Full=Muramidase;", XP002655449, retrieved from EBI accession no. UNIPROT:P15057 Database accession no. P15057 & GARCIA E ET AL: "Molecular evolution of lytic enzymes of streptococcus pneumoniae and its bacteriophages", PROCEEDINGS OF THE NATIONAL ACADEMY OF SCIENCES, NATIONAL ACADEMY OF SCIENCES, WASHINGTON, DC; US, vol. 85, 1 February 1988 (1988-02-01), pages 914-918, XP002973729, ISSN: 0027-8424, DOI: DOI:10.1073/PNAS.85.3.914

### SEQUENCE LISTING

<110> Lysando Holding AG and Katholieke Universiteit Leuven
<120> ANTIMICROBIAL AGENTS
<130> LYS-007 PCT
<140> not yet known
   <141> 2011-12-23
<160> 164
<170> PatentIn version 3.3
<210> 1
   <211> 339
   <212> PRT
   <213> Streptococcus pneumoniae Phage Cpl-1
<400> 1
   Met Val Lys Lys Asn Asp Leu Phe Val Asp Val Ser Ser His Asn Gly
   1               5                   10                  15
   Tyr Asp Ile Thr Gly Ile Leu Glu Gln Met Gly Thr Thr Asn Thr Ile
               20                  25                  30
   Ile Lys Ile Ser Glu Ser Thr Thr Tyr Leu Asn Pro Cys Leu Ser Ala
           35                  40                  45
   Gln Val Glu Gln Ser Asn Pro Ile Gly Phe Tyr His Phe Ala Arg Phe
       50                  55                  60
   Gly Gly Asp Val Ala Glu Ala Glu Arg Glu Ala Gln Phe Phe Leu Asp
   65                  70                  75                  80
   Asn Val Pro Met Gln Val Lys Tyr Leu Val Leu Asp Tyr Glu Asp Asp
                   85                  90                  95
   Pro Ser Gly Asp Ala Gln Ala Asn Thr Asn Ala Cys Leu Arg Phe Met
               100                 105                 110
   Gln Met Ile Ala Asp Ala Gly Tyr Lys Pro Ile Tyr Tyr Ser Tyr Lys
           115                 120                 125
   Pro Phe Thr His Asp Asn Val Asp Tyr Gln Gln Ile Leu Ala Gln Phe
       130                 135                 140
   Pro Asn Ser Leu Trp Ile Ala Gly Tyr Gly Leu Asn Asp Gly Thr Ala
   145                 150                 155                 160
   Asn Phe Glu Tyr Phe Pro Ser Met Asp Gly Ile Arg Trp Trp Gln Tyr
                   165                 170                 175
   Ser Ser Asn Pro Phe Asp Lys Asn Ile Val Leu Leu Asp Asp Glu Glu
               180                 185                 190
   Asp Asp Lys Pro Lys Thr Ala Gly Thr Trp Lys Gln Asp Ser Lys Gly
           195                 200                 205
   Trp Trp Phe Arg Arg Asn Asn Gly Ser Phe Pro Tyr Asn Lys Trp Glu
       210                 215                 220
   Lys Ile Gly Gly Val Trp Tyr Tyr Phe Asp Ser Lys Gly Tyr Cys Leu
   225                 230                 235                 240
   Thr Ser Glu Trp Leu Lys Asp Asn Glu Lys Trp Tyr Tyr Leu Lys Asp
                   245                 250                 255
   Asn Gly Ala Met Ala Thr Gly Trp Val Leu Val Gly Ser Glu Trp Tyr
               260                 265                 270
   Tyr Met Asp Asp Ser Gly Ala Met Val Thr Gly Trp Val Lys Tyr Lys
           275                 280                 285
   Asn Asn Trp Tyr Tyr Met Thr Asn Glu Arg Gly Asn Met Val Ser Asn
       290                 295                 300
   Glu Phe Ile Lys Ser Gly Lys Gly Trp Tyr Phe Met Asn Thr Asn Gly
   305                 310                 315                 320
   Glu Leu Ala Asp Asn Pro Ser Phe Thr Lys Glu Pro Asp Gly Leu Ile
                   325                 330                 335
   Thr Val Ala
<210> 2
   <211> 341
   <212> PRT
   <213> Listeria monocytogenes Phage A511
<400> 2
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
               35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Arg Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys
               340
<210> 3
   <211> 495
   <212> PRT
   <213> Staphylococcus aureus Phage K
<400> 3
   Met Ala Lys Thr Gln Ala Glu Ile Asn Lys Arg Leu Asp Ala Tyr Ala
   1               5                   10                  15
   Lys Gly Thr Val Asp Ser Pro Tyr Arg Val Lys Lys Ala Thr Ser Tyr
               20                  25                  30
   Asp Pro Ser Phe Gly Val Met Glu Ala Gly Ala Ile Asp Ala Asp Gly
           35                  40                  45
   Tyr Tyr His Ala Gln Cys Gln Asp Leu Ile Thr Asp Tyr Val Leu Trp
       50                  55                  60
   Leu Thr Asp Asn Lys Val Arg Thr Trp Gly Asn Ala Lys Asp Gln Ile
   65                  70                  75                  80
   Lys Gln Ser Tyr Gly Thr Gly Phe Lys Ile His Glu Asn Lys Pro Ser
                   85                  90                  95
   Thr Val Pro Lys Lys Gly Trp Ile Ala Val Phe Thr Ser Gly Ser Tyr
               100                 105                 110
   Glu Gln Trp Gly His Ile Gly Ile Val Tyr Asp Gly Gly Asn Thr Ser
           115                 120                 125
   Thr Phe Thr Ile Leu Glu Gln Asn Trp Asn Gly Tyr Ala Asn Lys Lys
       130                 135                 140
   Pro Thr Lys Arg Val Asp Asn Tyr Tyr Gly Leu Thr His Phe Ile Glu
   145                 150                 155                 160
   Ile Pro Val Lys Ala Gly Thr Thr Val Lys Lys Glu Thr Ala Lys Lys
                   165                 170                 175
   Ser Ala Ser Lys Thr Pro Ala Pro Lys Lys Lys Ala Thr Leu Lys Val
               180                 185                 190
   Ser Lys Asn His Ile Asn Tyr Thr Met Asp Lys Arg Gly Lys Lys Pro
           195                 200                 205
   Glu Gly Met Val Ile His Asn Asp Ala Gly Arg Ser Ser Gly Gln Gln
       210                 215                 220
   Tyr Glu Asn Ser Leu Ala Asn Ala Gly Tyr Ala Arg Tyr Ala Asn Gly
   225                 230                 235                 240
   Ile Ala His Tyr Tyr Gly Ser Glu Gly Tyr Val Trp Glu Ala Ile Asp
                   245                 250                 255
   Ala Lys Asn Gln Ile Ala Trp His Thr Gly Asp Gly Thr Gly Ala Asn
               260                 265                 270
   Ser Gly Asn Phe Arg Phe Ala Gly Ile Glu Val Cys Gln Ser Met Ser
           275                 280                 285
   Ala Ser Asp Ala Gln Phe Leu Lys Asn Glu Gln Ala Val Phe Gln Phe
       290                 295                 300
   Thr Ala Glu Lys Phe Lys Glu Trp Gly Leu Thr Pro Asn Arg Lys Thr
   305                 310                 315                 320
   Val Arg Leu His Met Glu Phe Val Pro Thr Ala Cys Pro His Arg Ser
                   325                 330                 335
   Met Val Leu His Thr Gly Phe Asn Pro Val Thr Gln Gly Arg Pro Ser
               340                 345                 350
   Gln Ala Ile Met Asn Lys Leu Lys Asp Tyr Phe Ile Lys Gln Ile Lys
           355                 360                 365
   Asn Tyr Met Asp Lys Gly Thr Ser Ser Ser Thr Val Val Lys Asp Gly
       370                 375                 380
   Lys Thr Ser Ser Ala Ser Thr Pro Ala Thr Arg Pro Val Thr Gly Ser
   385                 390                 395                 400
   Trp Lys Lys Asn Gln Tyr Gly Thr Trp Tyr Lys Pro Glu Asn Ala Thr
                   405                 410                 415
   Phe Val Asn Gly Asn Gln Pro Ile Val Thr Arg Ile Gly Ser Pro Phe
   420 425 430
   Leu Asn Ala Pro Val Gly Gly Asn Leu Pro Ala Gly Ala Thr Ile Val
   435 440 445
   Tyr Asp Glu Val Cys Ile Gln Ala Gly His Ile Trp Ile Gly Tyr Asn
   450 455 460
   Ala Tyr Asn Gly Asn Arg Val Tyr Cys Pro Val Arg Thr Cys Gln Gly
   465 470 475 480
   Val Pro Pro Asn Gln Ile Pro Gly Val Ala Trp Gly Val Phe Lys
   485 490 495
<210> 4
   <211> 245
   <212> PRT
   <213> Staphylococcus simulans
<400> 4
   Met Ala His Glu His Ser Ala Gln Trp Leu Asn Asn Tyr Lys Lys Gly
   1 5 10 15
   Tyr Gly Tyr Gly Pro Tyr Pro Leu Gly Ile Asn Gly Gly Met His Tyr
   20 25 30
   Gly Val Asp Phe Phe Met Asn Ile Gly Thr Pro Val Lys Ala Ile Ser
   35 40 45
   Ser Gly Lys Ile Val Glu Ala Gly Trp Ser Asn Tyr Gly Gly Gly Asn
   50 55 60
   Gln Ile Gly Leu Ile Glu Asn Asp Gly Val His Arg Gln Trp Tyr Met
   65 70 75 80
   His Leu Ser Lys Tyr Asn Val Lys Val Gly Asp Tyr Val Lys Ala Gly
   85 90 95
   Gln Ile Ile Gly Trp Ser Gly Ser Thr Gly Tyr Ser Thr Ala Pro His
   100 105 110
   Leu His Phe Gln Arg Met Val Asn Ser Phe Ser Asn Ser Thr Ala Gln
   115 120 125
   Asp Pro Met Pro Phe Leu Lys Ser Ala Gly Tyr Gly Lys Ala Gly Gly
   130 135 140
   Thr Val Thr Pro Thr Pro Asn Thr Gly Trp Lys Thr Asn Lys Tyr Gly
   145 150 155 160
   Thr Leu Tyr Lys Ser Glu Ser Ala Ser Phe Thr Pro Asn Thr Asp Ile
   165 170 175
   Ile Thr Arg Thr Thr Gly Pro Phe Arg Ser Met Pro Gln Ser Gly Val
   180 185 190
   Leu Lys Ala Gly Gln Thr Ile His Tyr Asp Glu Val Met Lys Gln Asp
   195 200 205
   Gly His Val Trp Val Gly Tyr Thr Gly Asn Ser Gly Gln Arg Ile Tyr
   210 215 220
   Leu Pro Val Arg Thr Trp Asn Lys Ser Thr Asn Thr Leu Gly Val Leu
   225 230 235 240
   Trp Gly Thr Ile Lys
   245
<210> 5
   <211> 286
   <212> PRT
   <213> Propionibacterium acnes
<400> 5
   Met Val Arg Tyr Ile Pro Ala Ala His His Ser Ala Gly Ser Asn Asn
   1 5 10 15
   Pro Val Asn Arg Val Val Ile His Ala Thr Cys Pro Asp Val Gly Phe
   20 25 30
   Pro Ser Ala Ser Arg Lys Gly Arg Ala Val Ser Thr Ala Asn Tyr Phe
   35 40 45
   Ala Ser Pro Ser Ser Gly Gly Ser Ala His Tyr Val Cys Asp Ile Gly
   50 55 60
   Glu Thr Val Gln Cys Leu Ser Glu Ser Thr Ile Gly Trp His Ala Pro
   65 70 75 80
   Pro Asn Pro His Ser Leu Gly Ile Glu Ile Cys Ala Asp Gly Gly Ser
   85 90 95
   His Ala Ser Phe Arg Val Pro Gly His Ala Tyr Thr Arg Glu Gln Trp
   100 105 110
   Leu Asp Pro Gln Val Trp Pro Ala Val Glu Arg Ala Ala Val Leu Cys
           115                 120                 125
   Arg Arg Leu Cys Asp Lys Tyr Asn Val Pro Lys Arg Lys Leu Ser Ala
       130                 135                 140
   Ala Asp Leu Lys Ala Gly Arg Arg Gly Val Cys Gly His Val Asp Val
   145                 150                 155                 160
   Thr Asp Ala Trp His Gln Ser Asp His Asp Asp Pro Gly Pro Trp Phe
                   165                 170                 175
   Pro Trp Asp Lys Phe Met Ala Val Val Asn Gly Gly Ser Gly Asp Ser
               180                 185                 190
   Gly Glu Leu Thr Val Ala Asp Val Lys Ala Leu His Asp Gln Ile Lys
           195                 200                 205
   Gln Leu Ser Ala Gln Leu Thr Gly Ser Val Asn Lys Leu His His Asp
       210                 215                 220
   Val Gly Val Val Gln Val Gln Asn Gly Asp Leu Gly Lys Arg Val Asp
   225                 230                 235                 240
   Ala Leu Ser Trp Val Lys Asn Pro Val Thr Gly Lys Leu Trp Arg Thr
                   245                 250                 255
   Lys Asp Ala Leu Trp Ser Val Trp Tyr Tyr Val Leu Glu Cys Arg Ser
               260                 265                 270
   Arg Leu Asp Arg Leu Glu Ser Ala Val Asn Asp Leu Lys Lys
           275                 280                 285
<210> 6
   <211> 260
   <212> PRT
   <213> Pseudomonas aeruginosa phage KZ
<400> 6
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys
               260
<210> 7
   <211> 292
   <212> PRT
   <213> Pseudomonas aeruginosa phage EL
<400> 7
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Leu Asp Lys Ala Phe Glu Val Tyr Lys Glu Arg Tyr Arg Thr
               100                 105                 110
   Pro Thr Tyr Asp Ile Ala Trp Ser Gly Lys Val Ser Pro Ala Phe Thr
           115                 120                 125
   Ala Lys Val Lys Asp Trp Cys Gly Val His Val Pro Asn His Arg Ala
       130                 135                 140
   Pro His Trp Leu Met Ala Cys Met Ala Phe Glu Thr Gly Gln Thr Phe
   145                 150                 155                 160
   Ser Pro Ser Ile Lys Asn Ala Ala Gly Ser Glu Ala Tyr Gly Leu Ile
                   165                 170                 175
   Gln Phe Met Ser Pro Ala Ala Asn Asp Leu Asn Val Pro Leu Ser Val
               180                 185                 190
   Ile Arg Ser Met Asp Gln Leu Thr Gln Leu Asp Leu Val Phe Lys Tyr
           195                 200                 205
   Phe Glu Met Trp Met Lys Arg Gly Lys Arg Tyr Thr Gln Leu Glu Asp
       210                 215                 220
   Phe Tyr Leu Thr Ile Phe His Pro Ala Ser Val Gly Lys Lys Ala Asp
   225                 230                 235                 240
   Glu Val Leu Phe Leu Gln Gly Ser Lys Ala Tyr Leu Gln Asn Lys Gly
                   245                 250                 255
   Phe Asp Val Asp Lys Asp Gly Lys Ile Thr Leu Gly Glu Ile Ser Ser
   260 265 270
   Thr Leu Tyr Thr Thr Tyr Tyr Lys Gly Leu Leu Pro Glu Asn Arg His
   275 280 285
   Val Ile Ser Tyr
   290
<210> 8
   <211> 181
   <212> PRT
   <213> unknown
<220>
   <223> Salmonella endolysin
<400> 8
   Met Lys Pro Lys Asp Glu Ile Phe Asp Glu Ile Leu Gly Lys Glu Gly
   1 5 10 15
   Gly Tyr Val Asn His Pro Asp Asp Lys Gly Gly Pro Thr Lys Trp Gly
   20 25 30
   Ile Thr Glu Lys Val Ala Arg Ala His Gly Tyr Arg Gly Asp Met Arg
   35 40 45
   Asn Leu Thr Arg Gly Gln Ala Leu Glu Ile Leu Glu Thr Asp Tyr Trp
   50 55 60
   Tyr Gly Pro Arg Phe Asp Arg Val Ala Lys Ala Ser Pro Asp Val Ala
   65 70 75 80
   Ala Glu Leu Cys Asp Thr Gly Val Asn Met Gly Pro Ser Val Ala Ala
   85 90 95
   Lys Met Leu Gln Arg Trp Leu Asn Val Phe Asn Gln Gly Gly Arg Leu
   100 105 110
   Tyr Pro Asp Met Asp Thr Asp Gly Arg Ile Gly Pro Arg Thr Leu Asn
   115 120 125
   Ala Leu Arg Val Tyr Leu Glu Lys Arg Gly Lys Asp Gly Glu Arg Val
   130 135 140
   Leu Leu Val Ala Leu Asn Cys Thr Gln Gly Glu Arg Tyr Leu Glu Leu
   145 150 155 160
   Ala Glu Lys Arg Glu Ala Asp Glu Ser Phe Val Tyr Gly Trp Met Lys
                   165                 170                 175
   Glu Arg Val Leu Ile
               180
<210> 9
   <211> 163
   <212> PRT
   <213> Enterobacteria phage T4
<400> 9
   Met Asn Ile Phe Glu Met Leu Arg Ile Asp Glu Gly Leu Arg Leu Lys
   1               5                   10                  15
   Ile Tyr Lys Asp Thr Glu Gly Tyr Tyr Thr Ile Gly Ile Gly His Leu
               20                  25                  30
   Leu Thr Lys Ser Pro Ser Leu Asn Ala Ala Lys Ser Glu Leu Asp Lys
           35                  40                  45
   Ala Ile Gly Arg Asn Cys Asn Gly Val Ile Thr Lys Asp Glu Ala Glu
       50                  55                  60
   Lys Leu Phe Asn Gln Asp Val Asp Ala Ala Val Arg Gly Ile Leu Arg
   65                  70                  75                  80
   Asn Ala Lys Leu Lys Pro Val Tyr Asp Ser Leu Asp Ala Val Arg Arg
                   85                  90                  95
   Cys Ala Leu Ile Asn Met Val Phe Gln Met Gly Glu Thr Gly Val Ala
               100                 105                 110
   Gly Phe Thr Asn Ser Leu Arg Met Leu Gln Gln Lys Arg Trp Asp Glu
           115                 120                 125
   Ala Ala Val Asn Leu Ala Lys Ser Arg Trp Tyr Asn Gln Thr Pro Asn
       130                 135                 140
   Arg Ala Lys Arg Val Ile Thr Thr Phe Arg Thr Gly Thr Trp Asp Ala
   145                 150                 155                 160
   Tyr Lys Asn
<210> 10
   <211> 280
   <212> PRT
   <213> Acinetobacter baumannii phage
<400> 10
   Met Glu Tyr Asp Met Ile Leu Lys Phe Gly Ser Lys Gly Asp Ala Val
   1               5                   10                  15
   Ala Thr Leu Gln Lys Gln Leu Ala Lys Met Gly Tyr Lys Gly Val Lys
               20                  25                  30
   Asp Lys Pro Leu Ser Val Asp Gly His Phe Gly Glu Ser Thr Glu Phe
           35                  40                  45
   Ala Val Ile Gln Leu Gln Arg Lys Phe Gly Leu Val Ala Asp Gly Lys
       50                  55                  60
   Val Gly Asp Lys Thr Arg Gln Ala Leu Ala Gly Asp Ser Val Ser Lys
   65                  70                  75                  80
   Phe Leu Lys Asp Glu Asp Tyr Lys Lys Ala Ala Ile Arg Leu Lys Val
                   85                  90                  95
   Pro Glu Leu Val Ile Arg Val Phe Gly Ala Val Glu Gly Leu Gly Val
               100                 105                 110
   Gly Phe Leu Pro Asn Gly Lys Ala Lys Ile Leu Phe Glu Arg His Arg
           115                 120                 125
   Met Tyr Phe Tyr Leu Cys Gln Ala Leu Gly Lys Thr Phe Ala Asn Ser
       130                 135                 140
   Gln Val Lys Ile Thr Pro Asn Ile Val Asn Thr Leu Thr Gly Gly Tyr
   145                 150                 155                 160
   Lys Gly Asp Ala Ala Glu Tyr Thr Arg Leu Ser Met Ala Ile Asn Ile
                   165                 170                 175
   His Lys Glu Ser Ala Leu Met Ser Thr Ser Trp Gly Gln Phe Gln Ile
               180                 185                 190
   Met Gly Glu Asn Trp Lys Asp Leu Gly Tyr Ser Ser Val Gln Glu Phe
           195                 200                 205
   Val Asp Gln Gln Gln Leu Asn Glu Gly Asn Gln Leu Glu Ala Phe Ile
       210                 215                 220
   Arg Phe Ile Glu Trp Lys Pro Gly Leu Leu Glu Ala Leu Arg Lys Gln
   225                 230                 235                 240
   Asp Trp Asp Thr Val Phe Thr Leu Tyr Asn Gly Lys Asn Tyr Lys Lys
                   245                 250                 255
   Leu Gly Tyr Gln Ala Lys Phe Gln Lys Glu Trp Asp His Leu Glu Pro
               260                 265                 270
   Ile Tyr Arg Glu Lys Thr Ala Ala
           275                 280
<210> 11
   <211> 152
   <212> PRT
   <213> E. coli phage K1F
<400> 11
   Met Val Ser Lys Val Gln Phe Asn Pro Arg Ser Arg Thr Asp Ala Ile
   1               5                   10                  15
   Phe Val His Cys Ser Ala Thr Lys Pro Glu Met Asp Ile Gly Val Glu
               20                  25                  30
   Thr Ile Arg Met Trp His Lys Gln Gln Ala Trp Leu Asp Val Gly Tyr
           35                  40                  45
   His Phe Ile Ile Lys Arg Asp Gly Thr Val Glu Glu Gly Arg Pro Val
       50                  55                  60
   Asn Val Val Gly Ser His Val Lys Asp Trp Asn Ser Arg Ser Val Gly
   65                  70                  75                  80
   Val Cys Leu Val Gly Gly Ile Asn Ala Lys Gly Gln Phe Glu Ala Asn
                   85                  90                  95
   Phe Thr Pro Ala Gln Met Asn Ser Leu Arg Asn Lys Leu Asp Asp Leu
               100                 105                 110
   Lys Val Met Tyr Pro Gln Ala Glu Ile Arg Ala His His Asp Val Ala
           115                 120                 125
   Pro Lys Ala Cys Pro Ser Phe Asp Leu Gln Arg Trp Leu Ser Thr Asn
       130                 135                 140
   Glu Leu Val Thr Ser Asp Arg Gly
   145                 150
<210> 12
   <211> 328
   <212> PRT
   <213> Pseudomonas putida phage
<400> 12
   Met Lys Asn Ser Glu Lys Asn Ala Ser Ile Ile Met Ser Ile Gln Arg
   1               5                   10                  15
   Thr Leu Ala Ser Leu Ser Leu Tyr Gly Gly Arg Ile Asp Gly Leu Phe
               20                  25                  30
   Gly Glu Lys Cys Arg Gly Ala Ile Ile Leu Met Leu Asn Lys Val Tyr
           35                  40                  45
   Pro Asn Phe Ser Thr Asn Lys Leu Pro Ser Asn Thr Tyr Glu Ala Glu
       50                  55                  60
   Ser Val Phe Thr Phe Leu Gln Thr Ala Leu Ala Gly Val Gly Leu Tyr
   65                  70                  75                  80
   Thr Ile Thr Ile Asp Gly Lys Trp Gly Gly Thr Ser Gln Gly Ala Ile
                   85                  90                  95
   Asp Ala Leu Val Lys Ser Tyr Arg Gln Ile Thr Glu Ala Glu Arg Ala
               100                 105                 110
   Gly Ser Thr Leu Pro Leu Gly Leu Ala Thr Val Met Ser Lys His Met
           115                 120                 125
   Ser Ile Glu Gln Leu Arg Ala Met Leu Pro Thr Asp Arg Gln Gly Tyr
       130                 135                 140
   Ala Glu Val Tyr Ile Asp Pro Leu Asn Glu Thr Met Asp Ile Phe Glu
   145                 150                 155                 160
   Ile Asn Thr Pro Leu Arg Ile Ala His Phe Met Ala Gln Ile Leu His
                   165                 170                 175
   Glu Thr Ala Cys Phe Lys Tyr Thr Glu Glu Leu Ala Ser Gly Lys Ala
               180                 185                 190
   Tyr Glu Gly Arg Ala Asp Leu Gly Asn Thr Arg Pro Gly Asp Gly Pro
           195                 200                 205
   Leu Phe Lys Gly Arg Gly Leu Leu Gln Ile Thr Gly Arg Leu Asn Tyr
       210                 215                 220
   Val Lys Cys Gln Val Tyr Leu Arg Glu Lys Leu Lys Asp Pro Thr Phe
   225                 230                 235                 240
   Asp Ile Thr Ser Ser Val Thr Cys Ala Gln Gln Leu Ser Glu Ser Pro
                   245                 250                 255
   Leu Leu Ala Ala Leu Ala Ser Gly Tyr Phe Trp Arg Phe Ile Lys Pro
               260                 265                 270
   Lys Leu Asn Glu Thr Ala Asp Lys Asp Asp Ile Tyr Trp Val Ser Val
           275                 280                 285
   Tyr Val Asn Gly Tyr Ala Lys Gln Ala Asn Pro Tyr Tyr Pro Asn Arg
       290                 295                 300
   Asp Lys Glu Pro Asn His Met Lys Glu Arg Val Gln Met Leu Ala Val
   305                 310                 315                 320
   Thr Lys Lys Ala His Gly Ile Val
                   325
<210> 13
   <211> 165
   <212> PRT
   <213> unknown
<220>
   <223> Salmonella endolysin
<400> 13
   Met Pro Val Ile Asn Thr His Gln Asn Ile Ala Ala Phe Leu Asp Met
   1               5                   10                  15
   Leu Ala Tyr Ser Glu Gly Thr Ala Asn His Pro Leu Thr Lys Asn Arg
               20                  25                  30
   Gly Tyr Asp Val Ile Val Thr Gly Phe Asp Gly Ser Pro Glu Ile Phe
           35                  40                  45
   Thr Asp Tyr Ser Asp His Pro Phe Ala His Gly Arg Pro Pro Lys Val
       50                  55                  60
   Phe Asn Arg Arg Gly Glu Lys Ser Thr Ala Ser Gly Arg Tyr Gln Gln
   65                  70                  75                  80
   Leu Tyr Ile Phe Trp Pro His Tyr Lys Lys Gln Leu Ala Leu Pro Asp
                   85                  90                  95
   Phe Ser Pro Leu Ser Gln Asp Lys Leu Ala Ile Gln Leu Ile Arg Glu
               100                 105                 110
   Arg Gly Ala Ile Asp Asp Ile Arg Ala Gly Arg Ile Glu Arg Ala Val
           115                 120                 125
   Ser Arg Cys Arg Asn Ile Trp Ala Ser Leu Pro Gly Ala Gly Tyr Gly
       130                 135                 140
   Gln Arg Glu His Ser Leu Glu Lys Leu Val Thr Val Trp Arg Thr Ala
   145                 150                 155                 160
   Gly Gly Val Met Ala
                   165
<210> 14
   <211> 165
   <212> PRT
   <213> E.coli Phage P2
<400> 14
   Met Pro Val Ile Asn Thr His Gln Asn Ile Ala Ala Phe Leu Asp Met
   1               5                   10                  15
   Leu Ala Val Ser Glu Gly Thr Ala Asn His Pro Leu Thr Lys Asn Arg
               20                  25                  30
   Gly Tyr Asp Val Ile Val Thr Gly Leu Asp Gly Lys Pro Glu Ile Phe
           35                  40                  45
   Thr Asp Tyr Ser Asp His Pro Phe Ala His Gly Arg Pro Ala Lys Val
       50                  55                  60
   Phe Asn Arg Arg Gly Glu Lys Ser Thr Ala Ser Gly Arg Tyr Gln Gln
   65                  70                  75                  80
   Leu Tyr Leu Phe Trp Pro His Tyr Arg Lys Gln Leu Ala Leu Pro Asp
                   85                  90                  95
   Phe Ser Pro Leu Ser Gln Asp Arg Leu Ala Ile Gln Leu Ile Arg Glu
               100                 105                 110
   Arg Gly Ala Leu Asp Asp Ile Arg Ala Gly Arg Ile Glu Arg Ala Ile
           115                 120                 125
   Ser Arg Cys Arg Asn Ile Trp Ala Ser Leu Pro Gly Ala Gly Tyr Gly
       130                 135                 140
   Gln Arg Glu His Ser Leu Glu Lys Leu Val Thr Val Trp Arg Thr Ala
   145                 150                 155                 160
   Gly Gly Val Pro Ala
   165
<210> 15
   <211> 177
   <212> PRT
   <213> Salmonella typhimurium phage
<400> 15
   Met Asn Pro Ile Ile Asp Gly Ile Ile Ala Leu Glu Gly Gly Tyr Val
   1               5                   10                  15
   Phe Asn Pro Lys Asp Lys Gly Gly Ala Thr His Trp Gly Ile Thr Glu
               20                  25                  30
   Ala Thr Ala Arg Ala His Gly Tyr Ala Gly Asp Met Arg Asp Leu Thr
           35                  40                  45
   His Ala Glu Ala Tyr Ala Ile Leu Glu Glu Asp Tyr Trp Ile Lys Pro
       50                  55                  60
   Gly Phe Asp Val Ile Ser Thr Leu Ser Trp Pro Val Ser Phe Glu Leu
   65                  70                  75                  80
   Cys Asp Ala Ala Val Asn Ile Gly Ala Tyr His Pro Ser Ala Trp Leu
                   85                  90                  95
   Gln Arg Trp Leu Asn Val Phe Asn His Glu Gly Lys Arg Tyr Pro Asp
               100                 105                 110
   Ile His Val Asp Gly Asn Ile Gly Pro Arg Thr Leu Ala Ala Leu Glu
           115                 120                 125
   His Tyr Leu Ala Trp Arg Gly Gln Glu Gly Glu Ala Val Leu Val Lys
       130                 135                 140
   Ala Leu Asn Cys Ser Gln Gly Thr Tyr Tyr Leu Asn Val Ala Glu Lys
   145                 150                 155                 160
   Asn His Asn Asn Glu Gln Phe Ile Tyr Gly Trp Ile Lys Asn Arg Val
                   165                 170                 175
   Thr
<210> 16
   <211> 208
   <212> PRT
   <213> E. coli Phage N4
<400> 16
   Met Ala Ile Ser Lys Lys Lys Val Gly Gly Val Gly Gly Val Ile Ala
   1               5                   10                  15
   Ala Ile Ile Ala Ala Val Phe Ala Val Glu Gly Gly Tyr Val Asn Asp
               20                  25                  30
   Pro Lys Asp Pro Gly Gly Glu Thr Asn His Gly Val Thr Ile Gln Val
           35                  40                  45
   Ala Gln Lys His Lys Gln Glu Leu Glu Ser Met Tyr Asn Trp Asp Gly
       50                  55                  60
   Ser Met Lys Asn Leu Thr Gln Glu Met Ala Ser Ser Ile Tyr Tyr Asn
   65                  70                  75                  80
   Asp Tyr Ile Leu Lys Pro Gly Phe Val Lys Phe Ala Asp Val Ser Pro
                   85                  90                  95
   Ala Val Thr Glu Lys Leu Val Asp Ala Gly Val Asn Thr Gly Pro Ala
               100                 105                 110
   Arg Pro Ser Arg Trp Leu Gln Glu Ser Leu Asn Ala Phe Ser Arg Asn
           115                 120                 125
   Gly Lys Asp Tyr Pro Lys Ile Gln Val Asp Gly Lys Val Gly Ser Gly
       130                 135                 140
   Thr Leu Ser Ala Tyr Lys Ser Leu Gln Asn Lys Arg Gly Lys Val Glu
   145                 150                 155                 160
   Ala Cys Lys Leu Ile Leu Lys Ser Leu Asp Gly Lys Gln Leu Asn Tyr
                   165                 170                 175
   Tyr Leu Ser Leu Asn Met Pro Glu Tyr Thr Thr Gly Trp Ile Ala Asn
               180                 185                 190
   Arg Ile Gly Asn Val Pro Leu Glu Arg Cys Asn Glu Asp Ile Val Asn
           195                 200                 205
<210> 17
   <211> 185
   <212> PRT
   <213> unknown
<220>
   <223> N4-gp61 trunc.
<400> 17
   Met Val Glu Gly Gly Tyr Val Asn Asp Pro Lys Asp Pro Gly Gly Glu
   1               5                   10                  15
   Thr Asn His Gly Val Thr Ile Gln Val Ala Gln Lys His Lys Gln Glu
               20                  25                  30
   Leu Glu Ser Met Tyr Asn Trp Asp Gly Ser Met Lys Asn Leu Thr Gln
           35                  40                  45
   Glu Met Ala Ser Ser Ile Tyr Tyr Asn Asp Tyr Ile Leu Lys Pro Gly
       50                  55                  60
   Phe Val Lys Phe Ala Asp Val Ser Pro Ala Val Thr Glu Lys Leu Val
   65                  70                  75                  80
   Asp Ala Gly Val Asn Thr Gly Pro Ala Arg Pro Ser Arg Trp Leu Gln
                   85                  90                  95
   Glu Ser Leu Asn Ala Phe Ser Arg Asn Gly Lys Asp Tyr Pro Lys Ile
               100                 105                 110
   Gln Val Asp Gly Lys Val Gly Ser Gly Thr Leu Ser Ala Tyr Lys Ser
           115                 120                 125
   Leu Gln Asn Lys Arg Gly Lys Val Glu Ala Cys Lys Leu Ile Leu Lys
       130                 135                 140
   Ser Leu Asp Gly Lys Gln Leu Asn Tyr Tyr Leu Ser Leu Asn Met Pro
   145                 150                 155                 160
   Glu Tyr Thr Thr Gly Trp Ile Ala Asn Arg Ile Gly Asn Val Pro Leu
                   165                 170                 175
   Glu Arg Cys Asn Glu Asp Ile Val Asn
               180                 185
<210> 18
   <211> 259
   <212> PRT
   <213> Pseudomonas aeruginosa phage KZ
<400> 18
   Lys Val Leu Arg Lys Gly Asp Arg Gly Asp Glu Val Cys Gln Leu Gln
   1               5                   10                  15
   Thr Leu Leu Asn Leu Cys Gly Tyr Asp Val Gly Lys Pro Asp Gly Ile
               20                  25                  30
   Phe Gly Asn Asn Thr Phe Asn Gln Val Val Lys Phe Gln Lys Asp Asn
           35                  40                  45
   Cys Leu Asp Ser Asp Gly Ile Val Gly Lys Asn Thr Trp Ala Glu Leu
       50                  55                  60
   Phe Ser Lys Tyr Ser Pro Pro Ile Pro Tyr Lys Thr Ile Pro Met Pro
   65                  70                  75                  80
   Thr Ala Asn Lys Ser Arg Ala Ala Ala Thr Pro Val Met Asn Ala Val
                   85                  90                  95
   Glu Asn Ala Thr Gly Val Arg Ser Gln Leu Leu Leu Thr Phe Ala Ser
               100                 105                 110
   Ile Glu Ser Ala Phe Asp Tyr Glu Ile Lys Ala Lys Thr Ser Ser Ala
           115                 120                 125
   Thr Gly Trp Phe Gln Phe Leu Thr Gly Thr Trp Lys Thr Met Ile Glu
       130                 135                 140
   Asn Tyr Gly Met Lys Tyr Gly Val Leu Thr Asp Pro Thr Gly Ala Leu
   145                 150                 155                 160
   Arg Lys Asp Pro Arg Ile Ser Ala Leu Met Gly Ala Glu Leu Ile Lys
                   165                 170                 175
   Glu Asn Met Asn Ile Leu Arg Pro Val Leu Lys Arg Glu Pro Thr Asp
               180                 185                 190
   Thr Asp Leu Tyr Leu Ala His Phe Phe Gly Pro Gly Ala Ala Arg Arg
           195                 200                 205
   Phe Leu Thr Thr Gly Gln Asn Glu Leu Ala Ala Thr His Phe Pro Lys
       210                 215                 220
   Glu Ala Gln Ala Asn Pro Ser Ile Phe Tyr Asn Lys Asp Gly Ser Pro
   225                 230                 235                 240
   Lys Thr Ile Gln Glu Val Tyr Asn Leu Met Asp Gly Lys Val Ala Ala
                   245                 250                 255
   His Arg Lys
<210> 19
   <211> 6
   <212> PRT
   <213> artificial
<220>
   <223> synthetic sequence
<400> 19
   Lys Arg Lys Lys Arg Lys
   1               5
<210> 20
   <211> 5
   <212> PRT
   <213> artificial
<220>
   <223> synethtic sequence
<220>
   <221> misc_feature
   <222> (3)..(3)
   <223> Xaa can be any naturally occurring amino acid
<400> 20
   Lys Arg Xaa Lys Arg
   1               5
<210> 21
   <211> 5
   <212> PRT
   <213> artificial
<220>
   <223> synthetic sequence
<400> 21
   Lys Arg Ser Lys Arg
   1               5
<210> 22
   <211> 5
   <212> PRT
   <213> artificial
<220>
   <223> synthetic sequence
<400> 22
   Lys Arg Gly Ser Gly
   1               5
<210> 23
   <211> 9
   <212> PRT
   <213> artificial
<220>
   <223> synthetic sequence
<400> 23
   Lys Arg Lys Lys Arg Lys Lys Arg Lys
   1 5
<210> 24
   <211> 9
   <212> PRT
   <213> artificial
<220>
   <223> synthetic sequence
<400> 24
   Arg Arg Arg Arg Arg Arg Arg Arg Arg
   1 5
<210> 25
   <211> 8
   <212> PRT
   <213> artificial
<220>
   <223> synthetic sequence
<400> 25
   Lys Lys Lys Lys Lys Lys Lys Lys
   1 5
<210> 26
   <211> 10
   <212> PRT
   <213> artificial
<220>
   <223> synthetic sequence
<400> 26
   Lys Arg Lys Lys Arg Lys Lys Arg Lys Lys
   1 5 10
<210> 27
   <211> 12
   <212> PRT
   <213> artificial
<220>
   <223> synthetic sequence
<400> 27
   Lys Arg Lys Lys Arg Lys Lys Arg Lys Lys Arg Lys
   1 5 10
<210> 28
   <211> 14
   <212> PRT
   <213> artificial
<220>
   <223> synthetic sequence
<400> 28
   Lys Arg Lys Lys Arg Lys Lys Arg Lys Lys Arg Lys Lys Arg
   1 5 10
<210> 29
   <211> 16
   <212> PRT
   <213> artificial
<220>
   <223> synthetic sequence
<400> 29
   Lys Lys Lys Lys Lys Lys Lys Lys Lys Lys Lys Lys Lys Lys Lys Lys
   1 5 10 15
<210> 30
   <211> 18
   <212> PRT
   <213> artificial
<220>
   <223> synthetic sequence
<400> 30
   Lys Arg Lys Lys Arg Lys Lys Arg Lys Lys Arg Lys Lys Arg Lys Lys
   1 5 10 15
   Arg Lys
<210> 31
   <211> 19
   <212> PRT
   <213> artificial
<220>
   <223> synthetic sequence
<400> 31
   Lys Arg Lys Lys Arg Lys Lys Arg Lys Lys Arg Lys Lys Arg Lys Lys
   1 5 10 15
   Arg Lys Lys
<210> 32
   <211> 19
   <212> PRT
   <213> artificial
<220>
   <223> synthetic sequence
<400> 32
   Arg Arg Arg Arg Arg Arg Arg Arg Arg Arg Arg Arg Arg Arg Arg Arg
   1               5                   10                  15
   Arg Arg Arg
<210> 33
   <211> 19
   <212> PRT
   <213> artificial
<220>
   <223> synthetic sequence
<400> 33
   Lys Lys Lys Lys Lys Lys Lys Lys Lys Lys Lys Lys Lys Lys Lys Lys
   1               5                   10                  15
   Lys Lys Lys
<210> 34
   <211> 20
   <212> PRT
   <213> artificial
<220>
   <223> synthetic sequence
<400> 34
   Lys Arg Lys Lys Arg Lys Lys Arg Lys Arg Ser Lys Arg Lys Lys Arg
   1               5                   10                  15
   Lys Lys Arg Lys
               20
<210> 35
   <211> 21
   <212> PRT
   <213> artificial
<220>
   <223> synthetic sequence
<400> 35
   Lys Arg Lys Lys Arg Lys Lys Arg Lys Arg Ser Lys Arg Lys Lys Arg
   1               5                   10                  15
   Lys Lys Arg Lys Lys
               20
<210> 36
   <211> 21
   <212> PRT
   <213> artificial
<220>
   <223> synthetic sequence
<400> 36
   Lys Arg Lys Lys Arg Lys Lys Arg Lys Lys Arg Lys Lys Arg Lys Lys
   1               5                   10                  15
   Arg Lys Lys Arg Lys
               20
<210> 37
   <211> 22
   <212> PRT
   <213> artificial
<220>
   <223> synthetic sequence
<400> 37
   Lys Arg Lys Lys Arg Lys Lys Arg Lys Arg Gly Ser Gly Lys Arg Lys
   1               5                   10                  15
   Lys Arg Lys Lys Arg Lys
               20
<210> 38
   <211> 24
   <212> PRT
   <213> artificial
<220>
   <223> synthetic sequence
<400> 38
   Lys Arg Lys Lys Arg Lys Lys Arg Lys Arg Gly Ser Gly Ser Gly Lys
   1               5                   10                  15
   Arg Lys Lys Arg Lys Lys Arg Lys
   20
<210> 39
   <211> 25
   <212> PRT
   <213> artificial
<220>
   <223> synthetic sequence
<400> 39
   Lys Arg Lys Lys Arg Lys Lys Arg Lys Lys Arg Lys Lys Arg Lys Lys
   1 5 10 15
   Arg Lys Lys Arg Lys Lys Arg Lys Lys
   20 25
<210> 40
   <211> 31
   <212> PRT
   <213> artificial
<220>
   <223> synthetic sequence
<400> 40
   Lys Arg Lys Lys Arg Lys Lys Arg Lys Arg Ser Lys Arg Lys Lys Arg
   1 5 10 15
   Lys Lys Arg Lys Arg Ser Lys Arg Lys Lys Arg Lys Lys Arg Lys
   20 25 30
<210> 41
   <211> 38
   <212> PRT
   <213> artificial
<220>
   <223> synthetic sequence
<400> 41
   Lys Arg Lys Lys Arg Lys Lys Arg Lys Arg Gly Ser Gly Ser Gly Lys
   1 5 10 15
   Arg Lys Lys Arg Lys Lys Arg Lys Gly Ser Gly Ser Gly Lys Arg Lys
   20 25 30
   Lys Arg Lys Lys Arg Lys
   35
<210> 42
   <211> 39
   <212> PRT
   <213> artificial
<220>
   <223> synthetic sequence
<400> 42
   Lys Arg Lys Lys Arg Lys Lys Arg Lys Lys Arg Lys Lys Arg Lys Lys
   1               5                   10                  15
   Arg Lys Lys Arg Lys Lys Arg Lys Lys Arg Lys Lys Arg Lys Lys Arg
               20                  25                  30
   Lys Lys Arg Lys Lys Arg Lys
           35
<210> 43
   <211> 42
   <212> PRT
   <213> artificial
<220>
   <223> synthetic sequence
<400> 43
   Lys Arg Lys Lys Arg Lys Lys Arg Lys Arg Ser Lys Arg Lys Lys Arg
   1               5                   10                  15
   Lys Lys Arg Lys Arg Ser Lys Arg Lys Lys Arg Lys Lys Arg Lys Arg
               20                  25                  30
   Ser Lys Arg Lys Lys Arg Lys Lys Arg Lys
           35                  40
<210> 44
   <211> 37
   <212> PRT
   <213> Homo sapiens
<400> 44
   Leu Leu Gly Asp Phe Phe Arg Lys Ser Lys Glu Lys Ile Gly Lys Glu
   1 5 10 15
   Phe Lys Arg Ile Val Gln Arg Ile Lys Asp Phe Leu Arg Asn Leu Val
   20 25 30
   Pro Arg Thr Glu Ser
   35
<210> 45
   <211> 29
   <212> PRT
   <213> unknown
<220>
   <223> SMAP-29 sheep
<400> 45
   Arg Gly Leu Arg Arg Leu Gly Arg Lys Ile Ala His Gly Val Lys Lys
   1 5 10 15
   Tyr Gly Pro Thr Val Leu Arg Ile Ile Arg Ile Ala Gly
   20 25
<210> 46
   <211> 13
   <212> PRT
   <213> unknown
<220>
   <223> Indolicidine bovine
<400> 46
   Ile Leu Pro Trp Lys Trp Pro Trp Trp Pro Trp Arg Arg
   1 5 10
<210> 47
   <211> 18
   <212> PRT
   <213> unknown
<220>
   <223> Protegrin Porcine
<400> 47
   Arg Gly Gly Arg Leu Cys Tyr Cys Arg Arg Arg Phe Cys Val Cys Val
   1 5 10 15
   Gly Arg
<210> 48
   <211> 31
   <212> PRT
   <213> unknown
<220>
   <223> Cecropin P1 Mammal (pig)
<400> 48
   Ser Trp Leu Ser Lys Thr Ala Lys Lys Leu Glu Asn Ser Ala Lys Lys
   1               5                   10                  15
   Arg Ile Ser Glu Gly Ile Ala Ile Ala Ile Gln Gly Gly Pro Arg
               20                  25                  30
<210> 49
   <211> 23
   <212> PRT
   <213> unknown
<220>
   <223> Magainin frog
<400> 49
   Gly Ile Gly Lys Phe Leu His Ser Ala Lys Lys Phe Gly Lys Ala Phe
   1               5                   10                  15
   Val Gly Glu Ile Met Asn Ser
               20
<210> 50
   <211> 25
   <212> PRT
   <213> unknown
<220>
   <223> Pleurocidin fish
<400> 50
   Gly Trp Gly Ser Phe Phe Lys Lys Ala Ala His Val Gly Lys His Val
   1               5                   10                  15
   Gly Lys Ala Ala Leu Thr His Tyr Leu
               20                  25
<210> 51
   <211> 36
   <212> PRT
   <213> Aedes aegypti
<400> 51
   Gly Gly Leu Lys Lys Leu Gly Lys Lys Leu Glu Gly Ala Gly Lys Arg
   1 5 10 15
   Val Phe Asn Ala Ala Glu Lys Ala Leu Pro Val Val Ala Gly Ala Lys
   20 25 30
   Ala Leu Arg Lys
   35
<210> 52
   <211> 40
   <212> PRT
   <213> Drosophila melanogaster
<400> 52
   Gly Trp Leu Lys Lys Ile Gly Lys Lys Ile Glu Arg Val Gly Gln His
   1 5 10 15
   Thr Arg Asp Ala Thr Ile Gln Gly Leu Gly Ile Pro Gln Gln Ala Ala
   20 25 30
   Asn Val Ala Ala Thr Ala Arg Gly
   35 40
<210> 53
   <211> 21
   <212> PRT
   <213> unknown
<220>
   <223> Buforin II vertebrate
<400> 53
   Thr Arg Ser Ser Arg Ala Gly Leu Gln Phe Pro Val Gly Arg Val His
   1 5 10 15
   Arg Leu Leu Arg Lys
   20
<210> 54
   <211> 39
   <212> PRT
   <213> unknown
<220>
   <223> Sarcotoxin IA Fly
<400> 54
   Gly Trp Leu Lys Lys Ile Gly Lys Lys Ile Glu Arg Val Gly Gln His
   1               5                   10                  15
   Thr Arg Asp Ala Thr Ile Gln Gly Leu Gly Ile Ala Gln Gln Ala Ala
               20                  25                  30
   Asn Val Ala Ala Thr Ala Arg
           35
<210> 55
   <211> 17
   <212> PRT
   <213> Apis mellifera
<400> 55
   Ala Asn Arg Pro Val Tyr Ile Pro Pro Pro Arg Pro Pro His Pro Arg
   1               5                   10                  15
   Leu
<210> 56
   <211> 24
   <212> PRT
   <213> unknown
<220>
   <223> Ascaphine 5 Frog
<400> 56
   Gly Ile Lys Asp Trp Ile Lys Gly Ala Ala Lys Lys Leu Ile Lys Thr
   1               5                   10                  15
   Val Ala Ser His Ile Ala Asn Gln
               20
<210> 57
   <211> 22
   <212> PRT
   <213> unknown
<220>
   <223> Nigrocine 2 Frog
<400> 57
   Gly Leu Leu Ser Lys Val Leu Gly Val Gly Lys Lys Val Leu Cys Gly
   1               5                   10                  15
   Val Ser Gly Leu Val Cys
               20
<210> 58
   <211> 24
   <212> PRT
   <213> unknown
<220>
   <223> Pseudin 1 Rana Frog
<400> 58
   Gly Leu Asn Thr Leu Lys Lys Val Phe Gln Gly Leu His Glu Ala Ile
   1               5                   10                  15
   Lys Leu Ile Asn Asn His Val Gln
               20
<210> 59
   <211> 18
   <212> PRT
   <213> unknown
<220>
   <223> Ranalexin Frog
<400> 59
   Phe Leu Gly Gly Leu Ile Val Pro Ala Met Ile Cys Ala Val Thr Lys
   1               5                   10                  15
   Lys Cys
<210> 60
   <211> 26
   <212> PRT
   <213> unknown
<220>
   <223> Melittin bee
<400> 60
   Gly Ile Gly Ala Val Leu Lys Val Leu Thr Thr Gly Leu Pro Ala Leu
   1               5                   10                  15
   Ile Ser Trp Ile Lys Arg Lys Arg Gln Gln
               20                  25
<210> 61
   <211> 25
   <212> PRT
   <213> unknown
<220>
   <223> Lycotoxin 1 Spider
<400> 61
   Ile Trp Leu Thr Ala Leu Lys Phe Leu Gly Lys His Ala Ala Lys Lys
   1 5 10 15
   Leu Ala Lys Gln Gln Leu Ser Lys Leu
   20 25
<210> 62
   <211> 19
   <212> PRT
   <213> unknown
<220>
   <223> Parasin 1 Fish
<400> 62
   Lys Gly Arg Gly Lys Gln Gly Gly Lys Val Arg Ala Lys Ala Lys Thr
   1 5 10 15
   Arg Ser Ser
<210> 63
   <211> 34
   <212> PRT
   <213> Limulus polyphemus
<400> 63
   Gly Phe Lys Leu Lys Gly Met Ala Arg Ile Ser Cys Leu Pro Asn Gly
   1 5 10 15
   Gln Trp Ser Asn Phe Pro Pro Lys Cys Ile Arg Glu Cys Ala Met Val
   20 25 30
   Ser Ser
<210> 64
   <211> 27
   <212> PRT
   <213> artificial
<220>
   <223> synthetic sequence
<400> 64
   Lys Arg Trp Val Lys Arg Val Lys Arg Val Lys Arg Trp Val Lys Arg
   1 5 10 15
   Val Val Arg Val Val Lys Arg Trp Val Lys Arg
   20 25
<210> 65
   <211> 18
   <212> PRT
   <213> artificial
<220>
   <223> synthetic sequence
<400> 65
   Gly Phe Phe Ile Pro Ala Val Ile Leu Pro Ser Ile Ala Phe Leu Ile
   1               5                   10                  15
   Val Pro
<210> 66
   <211> 5
   <212> PRT
   <213> artificial
<220>
   <223> synthetic sequence
<400> 66
   Phe Phe Val Ala Pro
   1               5
<210> 67
   <211> 39
   <212> PRT
   <213> unknown
<220>
   <223> Buforin I Toad
<400> 67
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
               20                  25                  30
   Leu Leu Arg Lys Gly Asn Tyr
           35
<210> 68
   <211> 13
   <212> PRT
   <213> unknown
<220>
   <223> alpha4-helix of T4 lysozyme
<400> 68
   Pro Asn Arg Ala Lys Arg Val Ile Thr Thr Phe Arg Thr
   1               5                   10
<210> 69
   <211> 25
   <212> PRT
   <213> artificial
<220>
   <223> synthetic sequence
<400> 69
   Gly Lys Pro Gly Trp Leu Ile Lys Lys Ala Leu Val Phe Lys Lys Leu
   1               5                   10                  15
   Ile Arg Arg Pro Leu Lys Arg Leu Ala
               20                  25
<210> 70
   <211> 341
   <212> PRT
   <213> artificial
<220>
   <223> synthetic sequence
<400> 70
   Phe Phe Val Ala Pro Lys Arg Lys Lys Arg Lys Lys Arg Lys Lys Asn
   1               5                   10                  15
   Ser Glu Lys Asn Ala Ser Ile Ile Met Ser Ile Gln Arg Thr Leu Ala
               20                  25                  30
   Ser Leu Ser Leu Tyr Gly Gly Arg Ile Asp Gly Leu Phe Gly Glu Lys
           35                  40                  45
   Cys Arg Gly Ala Ile Ile Leu Met Leu Asn Lys Val Tyr Pro Asn Phe
       50                  55                  60
   Ser Thr Asn Lys Leu Pro Ser Asn Thr Tyr Glu Ala Glu Ser Val Phe
   65                  70                  75                  80
   Thr Phe Leu Gln Thr Ala Leu Ala Gly Val Gly Leu Tyr Thr Ile Thr
                   85                  90                  95
   Ile Asp Gly Lys Trp Gly Gly Thr Ser Gln Gly Ala Ile Asp Ala Leu
               100                 105                 110
   Val Lys Ser Tyr Arg Gln Ile Thr Glu Ala Glu Arg Ala Gly Ser Thr
           115                 120                 125
   Leu Pro Leu Gly Leu Ala Thr Val Met Ser Lys His Met Ser Ile Glu
       130                 135                 140
   Gln Leu Arg Ala Met Leu Pro Thr Asp Arg Gln Gly Tyr Ala Glu Val
   145                 150                 155                 160
   Tyr Ile Asp Pro Leu Asn Glu Thr Met Asp Ile Phe Glu Ile Asn Thr
                   165                 170                 175
   Pro Leu Arg Ile Ala His Phe Met Ala Gln Ile Leu His Glu Thr Ala
               180                 185                 190
   Cys Phe Lys Tyr Thr Glu Glu Leu Ala Ser Gly Lys Ala Tyr Glu Gly
           195                 200                 205
   Arg Ala Asp Leu Gly Asn Thr Arg Pro Gly Asp Gly Pro Leu Phe Lys
       210                 215                 220
   Gly Arg Gly Leu Leu Gln Ile Thr Gly Arg Leu Asn Tyr Val Lys Cys
   225                 230                 235                 240
   Gln Val Tyr Leu Arg Glu Lys Leu Lys Asp Pro Thr Phe Asp Ile Thr
                   245                 250                 255
   Ser Ser Val Thr Cys Ala Gln Gln Leu Ser Glu Ser Pro Leu Leu Ala
               260                 265                 270
   Ala Leu Ala Ser Gly Tyr Phe Trp Arg Phe Ile Lys Pro Lys Leu Asn
           275                 280                 285
   Glu Thr Ala Asp Lys Asp Asp Ile Tyr Trp Val Ser Val Tyr Val Asn
       290                 295                 300
   Gly Tyr Ala Lys Gln Ala Asn Pro Tyr Tyr Pro Asn Arg Asp Lys Glu
   305                 310                 315                 320
   Pro Asn His Met Lys Glu Arg Val Gln Met Leu Ala Val Thr Lys Lys
                   325                 330                 335
   Ala His Gly Ile Val
               340
<210> 71
   <211> 349
   <212> PRT
   <213> artificial
<220>
   <223> synthetic sequence
<400> 71
   Pro Asn Arg Ala Lys Arg Val Ile Thr Thr Phe Arg Thr Lys Arg Lys
   1               5                   10                  15
   Lys Arg Lys Lys Arg Lys Lys Asn Ser Glu Lys Asn Ala Ser Ile Ile
               20                  25                  30
   Met Ser Ile Gln Arg Thr Leu Ala Ser Leu Ser Leu Tyr Gly Gly Arg
           35                  40                  45
   Ile Asp Gly Leu Phe Gly Glu Lys Cys Arg Gly Ala Ile Ile Leu Met
       50                  55                  60
   Leu Asn Lys Val Tyr Pro Asn Phe Ser Thr Asn Lys Leu Pro Ser Asn
   65                  70                  75                  80
   Thr Tyr Glu Ala Glu Ser Val Phe Thr Phe Leu Gln Thr Ala Leu Ala
                   85                  90                  95
   Gly Val Gly Leu Tyr Thr Ile Thr Ile Asp Gly Lys Trp Gly Gly Thr
               100                 105                 110
   Ser Gln Gly Ala Ile Asp Ala Leu Val Lys Ser Tyr Arg Gln Ile Thr
           115                 120                 125
   Glu Ala Glu Arg Ala Gly Ser Thr Leu Pro Leu Gly Leu Ala Thr Val
       130                 135                 140
   Met Ser Lys His Met Ser Ile Glu Gln Leu Arg Ala Met Leu Pro Thr
   145                 150                 155                 160
   Asp Arg Gln Gly Tyr Ala Glu Val Tyr Ile Asp Pro Leu Asn Glu Thr
                   165                 170                 175
   Met Asp Ile Phe Glu Ile Asn Thr Pro Leu Arg Ile Ala His Phe Met
               180                 185                 190
   Ala Gln Ile Leu His Glu Thr Ala Cys Phe Lys Tyr Thr Glu Glu Leu
           195                 200                 205
   Ala Ser Gly Lys Ala Tyr Glu Gly Arg Ala Asp Leu Gly Asn Thr Arg
       210                 215                 220
   Pro Gly Asp Gly Pro Leu Phe Lys Gly Arg Gly Leu Leu Gln Ile Thr
   225                 230                 235                 240
   Gly Arg Leu Asn Tyr Val Lys Cys Gln Val Tyr Leu Arg Glu Lys Leu
                   245                 250                 255
   Lys Asp Pro Thr Phe Asp Ile Thr Ser Ser Val Thr Cys Ala Gln Gln
               260                 265                 270
   Leu Ser Glu Ser Pro Leu Leu Ala Ala Leu Ala Ser Gly Tyr Phe Trp
           275                 280                 285
   Arg Phe Ile Lys Pro Lys Leu Asn Glu Thr Ala Asp Lys Asp Asp Ile
       290                 295                 300
   Tyr Trp Val Ser Val Tyr Val Asn Gly Tyr Ala Lys Gln Ala Asn Pro
   305 310 315 320
   Tyr Tyr Pro Asn Arg Asp Lys Glu Pro Asn His Met Lys Glu Arg Val
   325 330 335
   Gln Met Leu Ala Val Thr Lys Lys Ala His Gly Ile Val
   340 345
<210> 72
   <211> 354
   <212> PRT
   <213> artificial
<220>
   <223> synthetic sequence
<400> 72
   Gly Phe Phe Ile Pro Ala Val Ile Leu Pro Ser Ile Ala Phe Leu Ile
   1 5 10 15
   Val Pro Lys Arg Lys Lys Arg Lys Lys Arg Lys Lys Asn Ser Glu Lys
   20 25 30
   Asn Ala Ser Ile Ile Met Ser Ile Gln Arg Thr Leu Ala Ser Leu Ser
   35 40 45
   Leu Tyr Gly Gly Arg Ile Asp Gly Leu Phe Gly Glu Lys Cys Arg Gly
   50 55 60
   Ala Ile Ile Leu Met Leu Asn Lys Val Tyr Pro Asn Phe Ser Thr Asn
   65 70 75 80
   Lys Leu Pro Ser Asn Thr Tyr Glu Ala Glu Ser Val Phe Thr Phe Leu
   85 90 95
   Gln Thr Ala Leu Ala Gly Val Gly Leu Tyr Thr Ile Thr Ile Asp Gly
   100 105 110
   Lys Trp Gly Gly Thr Ser Gln Gly Ala Ile Asp Ala Leu Val Lys Ser
   115 120 125
   Tyr Arg Gln Ile Thr Glu Ala Glu Arg Ala Gly Ser Thr Leu Pro Leu
   130 135 140
   Gly Leu Ala Thr Val Met Ser Lys His Met Ser Ile Glu Gln Leu Arg
   145 150 155 160
   Ala Met Leu Pro Thr Asp Arg Gln Gly Tyr Ala Glu Val Tyr Ile Asp
                   165                 170                 175
   Pro Leu Asn Glu Thr Met Asp Ile Phe Glu Ile Asn Thr Pro Leu Arg
               180                 185                 190
   Ile Ala His Phe Met Ala Gln Ile Leu His Glu Thr Ala Cys Phe Lys
           195                 200                 205
   Tyr Thr Glu Glu Leu Ala Ser Gly Lys Ala Tyr Glu Gly Arg Ala Asp
       210                 215                 220
   Leu Gly Asn Thr Arg Pro Gly Asp Gly Pro Leu Phe Lys Gly Arg Gly
   225                 230                 235                 240
   Leu Leu Gln Ile Thr Gly Arg Leu Asn Tyr Val Lys Cys Gln Val Tyr
                   245                 250                 255
   Leu Arg Glu Lys Leu Lys Asp Pro Thr Phe Asp Ile Thr Ser Ser Val
               260                 265                 270
   Thr Cys Ala Gln Gln Leu Ser Glu Ser Pro Leu Leu Ala Ala Leu Ala
           275                 280                 285
   Ser Gly Tyr Phe Trp Arg Phe Ile Lys Pro Lys Leu Asn Glu Thr Ala
       290                 295                 300
   Asp Lys Asp Asp Ile Tyr Trp Val Ser Val Tyr Val Asn Gly Tyr Ala
   305                 310                 315                 320
   Lys Gln Ala Asn Pro Tyr Tyr Pro Asn Arg Asp Lys Glu Pro Asn His
                   325                 330                 335
   Met Lys Glu Arg Val Gln Met Leu Ala Val Thr Lys Lys Ala His Gly
               340                 345                 350
   Ile Val
<210> 73
   <211> 361
   <212> PRT
   <213> artificial
<220>
   <223> synthetic sequence
<400> 73
   Gly Lys Pro Gly Trp Leu Ile Lys Lys Ala Leu Val Phe Lys Lys Leu
   1               5                   10                  15
   Ile Arg Arg Pro Leu Lys Arg Leu Ala Lys Arg Lys Lys Arg Lys Lys
               20                  25                  30
   Arg Lys Lys Asn Ser Glu Lys Asn Ala Ser Ile Ile Met Ser Ile Gln
           35                  40                  45
   Arg Thr Leu Ala Ser Leu Ser Leu Tyr Gly Gly Arg Ile Asp Gly Leu
       50                  55                  60
   Phe Gly Glu Lys Cys Arg Gly Ala Ile Ile Leu Met Leu Asn Lys Val
   65                  70                  75                  80
   Tyr Pro Asn Phe Ser Thr Asn Lys Leu Pro Ser Asn Thr Tyr Glu Ala
                   85                  90                  95
   Glu Ser Val Phe Thr Phe Leu Gln Thr Ala Leu Ala Gly Val Gly Leu
               100                 105                 110
   Tyr Thr Ile Thr Ile Asp Gly Lys Trp Gly Gly Thr Ser Gln Gly Ala
           115                 120                 125
   Ile Asp Ala Leu Val Lys Ser Tyr Arg Gln Ile Thr Glu Ala Glu Arg
       130                 135                 140
   Ala Gly Ser Thr Leu Pro Leu Gly Leu Ala Thr Val Met Ser Lys His
   145                 150                 155                 160
   Met Ser Ile Glu Gln Leu Arg Ala Met Leu Pro Thr Asp Arg Gln Gly
                   165                 170                 175
   Tyr Ala Glu Val Tyr Ile Asp Pro Leu Asn Glu Thr Met Asp Ile Phe
               180                 185                 190
   Glu Ile Asn Thr Pro Leu Arg Ile Ala His Phe Met Ala Gln Ile Leu
           195                 200                 205
   His Glu Thr Ala Cys Phe Lys Tyr Thr Glu Glu Leu Ala Ser Gly Lys
       210                 215                 220
   Ala Tyr Glu Gly Arg Ala Asp Leu Gly Asn Thr Arg Pro Gly Asp Gly
   225                 230                 235                 240
   Pro Leu Phe Lys Gly Arg Gly Leu Leu Gln Ile Thr Gly Arg Leu Asn
                   245                 250                 255
   Tyr Val Lys Cys Gln Val Tyr Leu Arg Glu Lys Leu Lys Asp Pro Thr
               260                 265                 270
   Phe Asp Ile Thr Ser Ser Val Thr Cys Ala Gln Gln Leu Ser Glu Ser
           275                 280                 285
   Pro Leu Leu Ala Ala Leu Ala Ser Gly Tyr Phe Trp Arg Phe Ile Lys
       290                 295                 300
   Pro Lys Leu Asn Glu Thr Ala Asp Lys Asp Asp Ile Tyr Trp Val Ser
   305                 310                 315                 320
   Val Tyr Val Asn Gly Tyr Ala Lys Gln Ala Asn Pro Tyr Tyr Pro Asn
                   325                 330                 335
   Arg Asp Lys Glu Pro Asn His Met Lys Glu Arg Val Gln Met Leu Ala
               340                 345                 350
   Val Thr Lys Lys Ala His Gly Ile Val
           355                 360
<210> 74
   <211> 355
   <212> PRT
   <213> artificial
<220>
   <223> synthetic sequence
<400> 74
   Lys Gly Arg Gly Lys Gln Gly Gly Lys Val Arg Ala Lys Ala Lys Thr
   1               5                   10                  15
   Arg Ser Ser Lys Arg Lys Lys Arg Lys Lys Arg Lys Lys Asn Ser Glu
               20                  25                  30
   Lys Asn Ala Ser Ile Ile Met Ser Ile Gln Arg Thr Leu Ala Ser Leu
           35                  40                  45
   Ser Leu Tyr Gly Gly Arg Ile Asp Gly Leu Phe Gly Glu Lys Cys Arg
       50                  55                  60
   Gly Ala Ile Ile Leu Met Leu Asn Lys Val Tyr Pro Asn Phe Ser Thr
   65                  70                  75                  80
   Asn Lys Leu Pro Ser Asn Thr Tyr Glu Ala Glu Ser Val Phe Thr Phe
                   85                  90                  95
   Leu Gln Thr Ala Leu Ala Gly Val Gly Leu Tyr Thr Ile Thr Ile Asp
   100 105 110
   Gly Lys Trp Gly Gly Thr Ser Gln Gly Ala Ile Asp Ala Leu Val Lys
   115 120 125
   Ser Tyr Arg Gln Ile Thr Glu Ala Glu Arg Ala Gly Ser Thr Leu Pro
   130 135 140
   Leu Gly Leu Ala Thr Val Met Ser Lys His Met Ser Ile Glu Gln Leu
   145 150 155 160
   Arg Ala Met Leu Pro Thr Asp Arg Gln Gly Tyr Ala Glu Val Tyr Ile
   165 170 175
   Asp Pro Leu Asn Glu Thr Met Asp Ile Phe Glu Ile Asn Thr Pro Leu
   180 185 190
   Arg Ile Ala His Phe Met Ala Gln Ile Leu His Glu Thr Ala Cys Phe
   195 200 205
   Lys Tyr Thr Glu Glu Leu Ala Ser Gly Lys Ala Tyr Glu Gly Arg Ala
   210 215 220
   Asp Leu Gly Asn Thr Arg Pro Gly Asp Gly Pro Leu Phe Lys Gly Arg
   225 230 235 240
   Gly Leu Leu Gln Ile Thr Gly Arg Leu Asn Tyr Val Lys Cys Gln Val
   245 250 255
   Tyr Leu Arg Glu Lys Leu Lys Asp Pro Thr Phe Asp Ile Thr Ser Ser
   260 265 270
   Val Thr Cys Ala Gln Gln Leu Ser Glu Ser Pro Leu Leu Ala Ala Leu
   275 280 285
   Ala Ser Gly Tyr Phe Trp Arg Phe Ile Lys Pro Lys Leu Asn Glu Thr
   290 295 300
   Ala Asp Lys Asp Asp Ile Tyr Trp Val Ser Val Tyr Val Asn Gly Tyr
   305 310 315 320
   Ala Lys Gln Ala Asn Pro Tyr Tyr Pro Asn Arg Asp Lys Glu Pro Asn
   325 330 335
   His Met Lys Glu Arg Val Gln Met Leu Ala Val Thr Lys Lys Ala His
   340 345 350
   Gly Ile Val
   355
<210> 75
   <211> 361
   <212> PRT
   <213> artificial
<220>
   <223> synthetic sequence
<400> 75
   Ile Trp Leu Thr Ala Leu Lys Phe Leu Gly Lys His Ala Ala Lys Lys
   1               5                   10                  15
   Leu Ala Lys Gln Gln Leu Ser Lys Leu Lys Arg Lys Lys Arg Lys Lys
               20                  25                  30
   Arg Lys Lys Asn Ser Glu Lys Asn Ala Ser Ile Ile Met Ser Ile Gln
           35                  40                  45
   Arg Thr Leu Ala Ser Leu Ser Leu Tyr Gly Gly Arg Ile Asp Gly Leu
       50                  55                  60
   Phe Gly Glu Lys Cys Arg Gly Ala Ile Ile Leu Met Leu Asn Lys Val
   65                  70                  75                  80
   Tyr Pro Asn Phe Ser Thr Asn Lys Leu Pro Ser Asn Thr Tyr Glu Ala
                   85                  90                  95
   Glu Ser Val Phe Thr Phe Leu Gln Thr Ala Leu Ala Gly Val Gly Leu
               100                 105                 110
   Tyr Thr Ile Thr Ile Asp Gly Lys Trp Gly Gly Thr Ser Gln Gly Ala
           115                 120                 125
   Ile Asp Ala Leu Val Lys Ser Tyr Arg Gln Ile Thr Glu Ala Glu Arg
       130                 135                 140
   Ala Gly Ser Thr Leu Pro Leu Gly Leu Ala Thr Val Met Ser Lys His
   145                 150                 155                 160
   Met Ser Ile Glu Gln Leu Arg Ala Met Leu Pro Thr Asp Arg Gln Gly
                   165                 170                 175
   Tyr Ala Glu Val Tyr Ile Asp Pro Leu Asn Glu Thr Met Asp Ile Phe
               180                 185                 190
   Glu Ile Asn Thr Pro Leu Arg Ile Ala His Phe Met Ala Gln Ile Leu
   195 200 205
   His Glu Thr Ala Cys Phe Lys Tyr Thr Glu Glu Leu Ala Ser Gly Lys
   210 215 220
   Ala Tyr Glu Gly Arg Ala Asp Leu Gly Asn Thr Arg Pro Gly Asp Gly
   225 230 235 240
   Pro Leu Phe Lys Gly Arg Gly Leu Leu Gln Ile Thr Gly Arg Leu Asn
   245 250 255
   Tyr Val Lys Cys Gln Val Tyr Leu Arg Glu Lys Leu Lys Asp Pro Thr
   260 265 270
   Phe Asp Ile Thr Ser Ser Val Thr Cys Ala Gln Gln Leu Ser Glu Ser
   275 280 285
   Pro Leu Leu Ala Ala Leu Ala Ser Gly Tyr Phe Trp Arg Phe Ile Lys
   290 295 300
   Pro Lys Leu Asn Glu Thr Ala Asp Lys Asp Asp Ile Tyr Trp Val Ser
   305 310 315 320
   Val Tyr Val Asn Gly Tyr Ala Lys Gln Ala Asn Pro Tyr Tyr Pro Asn
   325 330 335
   Arg Asp Lys Glu Pro Asn His Met Lys Glu Arg Val Gln Met Leu Ala
   340 345 350
   Val Thr Lys Lys Ala His Gly Ile Val
   355 360
<210> 76
   <211> 341
   <212> PRT
   <213> artificial
<220>
   <223> synthetic sequence
<400> 76
   Lys Arg Lys Lys Arg Lys Lys Arg Lys Phe Phe Val Ala Pro Lys Asn
   1               5                   10                  15
   Ser Glu Lys Asn Ala Ser Ile Ile Met Ser Ile Gln Arg Thr Leu Ala
               20                  25                  30
   Ser Leu Ser Leu Tyr Gly Gly Arg Ile Asp Gly Leu Phe Gly Glu Lys
           35                  40                  45
   Cys Arg Gly Ala Ile Ile Leu Met Leu Asn Lys Val Tyr Pro Asn Phe
       50                  55                  60
   Ser Thr Asn Lys Leu Pro Ser Asn Thr Tyr Glu Ala Glu Ser Val Phe
   65                  70                  75                  80
   Thr Phe Leu Gln Thr Ala Leu Ala Gly Val Gly Leu Tyr Thr Ile Thr
                   85                  90                  95
   Ile Asp Gly Lys Trp Gly Gly Thr Ser Gln Gly Ala Ile Asp Ala Leu
               100                 105                 110
   Val Lys Ser Tyr Arg Gln Ile Thr Glu Ala Glu Arg Ala Gly Ser Thr
           115                 120                 125
   Leu Pro Leu Gly Leu Ala Thr Val Met Ser Lys His Met Ser Ile Glu
       130                 135                 140
   Gln Leu Arg Ala Met Leu Pro Thr Asp Arg Gln Gly Tyr Ala Glu Val
   145                 150                 155                 160
   Tyr Ile Asp Pro Leu Asn Glu Thr Met Asp Ile Phe Glu Ile Asn Thr
                   165                 170                 175
   Pro Leu Arg Ile Ala His Phe Met Ala Gln Ile Leu His Glu Thr Ala
               180                 185                 190
   Cys Phe Lys Tyr Thr Glu Glu Leu Ala Ser Gly Lys Ala Tyr Glu Gly
           195                 200                 205
   Arg Ala Asp Leu Gly Asn Thr Arg Pro Gly Asp Gly Pro Leu Phe Lys
       210                 215                 220
   Gly Arg Gly Leu Leu Gln Ile Thr Gly Arg Leu Asn Tyr Val Lys Cys
   225                 230                 235                 240
   Gln Val Tyr Leu Arg Glu Lys Leu Lys Asp Pro Thr Phe Asp Ile Thr
                   245                 250                 255
   Ser Ser Val Thr Cys Ala Gln Gln Leu Ser Glu Ser Pro Leu Leu Ala
               260                 265                 270
   Ala Leu Ala Ser Gly Tyr Phe Trp Arg Phe Ile Lys Pro Lys Leu Asn
           275                 280                 285
   Glu Thr Ala Asp Lys Asp Asp Ile Tyr Trp Val Ser Val Tyr Val Asn
       290                 295                 300
   Gly Tyr Ala Lys Gln Ala Asn Pro Tyr Tyr Pro Asn Arg Asp Lys Glu
   305                 310                 315                 320
   Pro Asn His Met Lys Glu Arg Val Gln Met Leu Ala Val Thr Lys Lys
                   325                 330                 335
   Ala His Gly Ile Val
               340
<210> 77
   <211> 349
   <212> PRT
   <213> artificial
<220>
   <223> synthetic sequence
<400> 77
   Lys Arg Lys Lys Arg Lys Lys Arg Lys Pro Asn Arg Ala Lys Arg Val
   1               5                   10                  15
   Ile Thr Thr Phe Arg Thr Lys Asn Ser Glu Lys Asn Ala Ser Ile Ile
               20                  25                  30
   Met Ser Ile Gln Arg Thr Leu Ala Ser Leu Ser Leu Tyr Gly Gly Arg
           35                  40                  45
   Ile Asp Gly Leu Phe Gly Glu Lys Cys Arg Gly Ala Ile Ile Leu Met
       50                  55                  60
   Leu Asn Lys Val Tyr Pro Asn Phe Ser Thr Asn Lys Leu Pro Ser Asn
   65                  70                  75                  80
   Thr Tyr Glu Ala Glu Ser Val Phe Thr Phe Leu Gln Thr Ala Leu Ala
                   85                  90                  95
   Gly Val Gly Leu Tyr Thr Ile Thr Ile Asp Gly Lys Trp Gly Gly Thr
               100                 105                 110
   Ser Gln Gly Ala Ile Asp Ala Leu Val Lys Ser Tyr Arg Gln Ile Thr
           115                 120                 125
   Glu Ala Glu Arg Ala Gly Ser Thr Leu Pro Leu Gly Leu Ala Thr Val
       130                 135                 140
   Met Ser Lys His Met Ser Ile Glu Gln Leu Arg Ala Met Leu Pro Thr
   145                 150                 155                 160
   Asp Arg Gln Gly Tyr Ala Glu Val Tyr Ile Asp Pro Leu Asn Glu Thr
                   165                 170                 175
   Met Asp Ile Phe Glu Ile Asn Thr Pro Leu Arg Ile Ala His Phe Met
               180                 185                 190
   Ala Gln Ile Leu His Glu Thr Ala Cys Phe Lys Tyr Thr Glu Glu Leu
           195                 200                 205
   Ala Ser Gly Lys Ala Tyr Glu Gly Arg Ala Asp Leu Gly Asn Thr Arg
       210                 215                 220
   Pro Gly Asp Gly Pro Leu Phe Lys Gly Arg Gly Leu Leu Gln Ile Thr
   225                 230                 235                 240
   Gly Arg Leu Asn Tyr Val Lys Cys Gln Val Tyr Leu Arg Glu Lys Leu
                   245                 250                 255
   Lys Asp Pro Thr Phe Asp Ile Thr Ser Ser Val Thr Cys Ala Gln Gln
               260                 265                 270
   Leu Ser Glu Ser Pro Leu Leu Ala Ala Leu Ala Ser Gly Tyr Phe Trp
           275                 280                 285
   Arg Phe Ile Lys Pro Lys Leu Asn Glu Thr Ala Asp Lys Asp Asp Ile
       290                 295                 300
   Tyr Trp Val Ser Val Tyr Val Asn Gly Tyr Ala Lys Gln Ala Asn Pro
   305                 310                 315                 320
   Tyr Tyr Pro Asn Arg Asp Lys Glu Pro Asn His Met Lys Glu Arg Val
                   325                 330                 335
   Gln Met Leu Ala Val Thr Lys Lys Ala His Gly Ile Val
               340                 345
<210> 78
   <211> 39
   <212> PRT
   <213> unknown
<220>
   <223> Buforin I Toad
<400> 78
   Ala Gly Arg Gly Lys Gln Gly Gly Lys Val Arg Ala Lys Ala Lys Thr
   1               5                   10                  15
   Arg Ser Ser Arg Ala Gly Leu Gln Phe Pro Val Gly Arg Val His Arg
   20 25 30
   Leu Leu Arg Lys Gly Asn Tyr
   35
<210> 79
   <211> 34
   <212> PRT
   <213> unknown
<220>
   <223> Dermaseptin 1 Frog
<400> 79
   Ala Leu Trp Lys Thr Met Leu Lys Lys Leu Gly Thr Met Ala Leu His
   1 5 10 15
   Ala Gly Lys Ala Ala Leu Gly Ala Ala Ala Asp Thr Ile Ser Gln Gly
   20 25 30
   Thr Gln
<210> 80
   <211> 12
   <212> PRT
   <213> unknown
<220>
   <223> Bactenecin 1 Cow
<400> 80
   Arg Leu Cys Arg Ile Val Val Ile Arg Val Cys Arg
   1 5 10
<210> 81
   <211> 21
   <212> PRT
   <213> unknown
<220>
   <223> Thanatin Insect
<400> 81
   Gly Ser Lys Lys Pro Val Pro Ile Ile Tyr Cys Asn Arg Arg Thr Gly
   1 5 10 15
   Lys Cys Gln Arg Met
   20
<210> 82
   <211> 19
   <212> PRT
   <213> unknown
<220>
   <223> Brevinin 1T Rana frogs
<400> 82
   Val Asn Pro Ile Ile Leu Gly Val Leu Pro Lys Val Cys Leu Ile Thr
   1               5                   10                  15
   Lys Lys Cys
<210> 83
   <211> 26
   <212> PRT
   <213> unknown
<220>
   <223> Ranateurin 1 Rana frog
<400> 83
   Ser Met Leu Ser Val Leu Lys Asn Leu Gly Lys Val Gly Leu Gly Phe
   1               5                   10                  15
   Val Ala Cys Lys Ile Asn Ile Lys Gln Cys
               20                  25
<210> 84
   <211> 46
   <212> PRT
   <213> unknown
<220>
   <223> Esculentin 1 Rana frogs
<400> 84
   Gly Ile Phe Ser Lys Leu Gly Arg Lys Lys Ile Lys Asn Leu Leu Ile
   1               5                   10                  15
   Ser Gly Leu Lys Asn Val Gly Lys Glu Val Gly Met Asp Val Val Arg
               20                  25                  30
   Thr Gly Ile Lys Ile Ala Gly Cys Lys Ile Lys Gly Glu Cys
           35                  40                  45
<210> 85
   <211> 17
   <212> PRT
   <213> Limulus polyphemus
<400> 85
   Arg Trp Cys Phe Arg Val Cys Tyr Arg Gly Ile Cys Tyr Arg Lys Cys
   1               5                   10                  15
   Arg
<210> 86
   <211> 25
   <212> PRT
   <213> unknown
<220>
   <223> Androctonin Scorpion
<400> 86
   Arg Ser Val Cys Arg Gln Ile Lys Ile Cys Arg Arg Arg Gly Gly Cys
   1               5                   10                  15
   Tyr Tyr Lys Cys Thr Asn Arg Pro Tyr
               20                  25
<210> 87
   <211> 30
   <212> PRT
   <213> Homo sapiens
<400> 87
   Asp Cys Tyr Cys Arg Ile Pro Ala Cys Ile Ala Gly Glu Arg Arg Tyr
   1               5                   10                  15
   Gly Thr Cys Ile Tyr Gln Gly Arg Leu Trp Ala Phe Cys Cys
               20                  25                  30
<210> 88
   <211> 38
   <212> PRT
   <213> unknown
<220>
   <223> beta-defensin cow
<400> 88
   Asn Pro Val Ser Cys Val Arg Asn Lys Gly Ile Cys Val Pro Ile Arg
   1 5 10 15
   Cys Pro Gly Ser Met Lys Gln Ile Gly Thr Cys Val Gly Arg Ala Val
   20 25 30
   Lys Cys Cys Arg Lys Lys
   35
<210> 89
   <211> 18
   <212> PRT
   <213> unknown
<220>
   <223> theta-defensin monkey
<400> 89
   Gly Phe Cys Arg Cys Leu Cys Arg Arg Gly Val Cys Arg Cys Ile Cys
   1 5 10 15
   Thr Arg
<210> 90
   <211> 40
   <212> PRT
   <213> unknown
<220>
   <223> defensin (sapecin A) insect
<400> 90
   Ala Thr Cys Asp Leu Leu Ser Gly Thr Gly Ile Asn His Ser Ala Cys
   1 5 10 15
   Ala Ala His Cys Leu Leu Arg Gly Asn Arg Gly Gly Tyr Cys Asn Gly
   20 25 30
   Lys Ala Val Cys Val Cys Arg Asn
   35 40
<210> 91
   <211> 46
   <212> PRT
   <213> unknown
<220>
   <223> Thionin (crambin) plant
<400> 91
   Thr Thr Cys Cys Pro Ser Ile Val Ala Arg Ser Asn Phe Asn Val Cys
   1               5                   10                  15
   Arg Ile Pro Gly Thr Pro Glu Ala Ile Cys Ala Thr Tyr Thr Gly Cys
               20                  25                  30
   Ile Ile Ile Pro Gly Ala Thr Cys Pro Gly Asp Tyr Ala Asn
           35                  40                  45
<210> 92
   <211> 50
   <212> PRT
   <213> unknown
<220>
   <223> defensin from radish
<400> 92
   Gln Lys Leu Cys Gln Arg Pro Ser Gly Thr Trp Ser Gly Val Cys Gly
   1               5                   10                  15
   Asn Asn Asn Ala Cys Lys Asn Gln Cys Ile Arg Leu Glu Lys Ala Arg
               20                  25                  30
   His Gly Ser Cys Asn Tyr Val Phe Pro Ala His Cys Ile Cys Tyr Phe
           35                  40                  45
   Pro Cys
       50
<210> 93
   <211> 44
   <212> PRT
   <213> Drosophila melanogaster
<400> 93
   Asp Cys Leu Ser Gly Arg Tyr Lys Gly Pro Cys Ala Val Trp Asp Asn
   1               5                   10                  15
   Glu Thr Cys Arg Arg Val Cys Lys Glu Glu Gly Arg Ser Ser Gly His
               20                  25                  30
   Cys Ser Pro Ser Leu Lys Cys Trp Cys Glu Gly Cys
           35                  40
<210> 94
   <211> 25
   <212> PRT
   <213> Homo sapiens
<400> 94
   Asp Thr His Phe Pro Ile Cys Ile Phe Cys Cys Gly Cys Cys His Arg
   1               5                   10                  15
   Ser Lys Cys Gly Met Cys Cys Lys Thr
               20                  25
<210> 95
   <211> 44
   <212> PRT
   <213> unknown
<220>
   <223> Bac 5 Cow
<400> 95
   Arg Phe Arg Pro Pro Ile Arg Arg Pro Pro Ile Arg Pro Pro Phe Tyr
   1               5                   10                  15
   Pro Pro Phe Arg Pro Pro Ile Arg Pro Pro Ile Phe Pro Pro Ile Arg
               20                  25                  30
   Pro Pro Phe Arg Pro Pro Leu Gly Arg Pro Phe Pro
           35                  40
<210> 96
   <211> 39
   <212> PRT
   <213> unknown
<220>
   <223> PR-39 Pig
<400> 96
   Arg Arg Arg Pro Arg Pro Pro Tyr Leu Pro Arg Pro Arg Pro Pro Pro
   1               5                   10                  15
   Phe Phe Pro Pro Arg Leu Pro Pro Arg Ile Pro Pro Gly Phe Pro Pro
               20                  25                  30
   Arg Phe Pro Pro Arg Phe Pro
           35
<210> 97
   <211> 20
   <212> PRT
   <213> unknown
<220>
   <223> Pyrrhocoricin Insect
<400> 97
   Val Asp Lys Gly Ser Tyr Leu Pro Arg Pro Thr Pro Pro Arg Pro Ile
   1               5                   10                  15
   Tyr Asn Arg Asn
               20
<210> 98
   <211> 24
   <212> PRT
   <213> Homo sapiens
<400> 98
   Asp Ser His Ala Lys Arg His His Gly Tyr Lys Arg Lys Phe His Glu
   1               5                   10                  15
   Lys His His Ser His Arg Gly Tyr
               20
<210> 99
   <211> 39
   <212> DNA
   <213> artificial
<220>
   <223> synthetic sequence
<400> 99
   ttggaatggg gagcccgaac cgtgcaaaac gtgtaatca 39
<210> 100
   <211> 39
   <212> DNA
   <213> artificial
<220>
   <223> synthetic sequence
<400> 100
   gtttggagga gccggtacgg aaggtggtga ttacacgtt 39
<210> 101
   <211> 39
   <212> DNA
   <213> artificial
<220>
   <223> synthetic sequence
<400> 101
   ttatgggctt cttcatcccg gcagtaatcc tgccctcca 39
<210> 102
   <211> 46
   <212> DNA
   <213> artificial
<220>
   <223> synthetic sequence
<400> 102
   gtttggagga gcccggtacg atcaggaatg cgatggaggg caggat 46
<210> 103
   <211> 47
   <212> DNA
   <213> artificial
<220>
   <223> synthetic sequence
<400> 103
   ttatgggcaa accgggctgg ctgatcaaaa ggcactggta ttcaaga 47
<210> 104
   <211> 57
   <212> DNA
   <213> artificial
<220>
   <223> synthetic sequence
<400> 104
   gtttggagga gcctgccagt ctcttcagcg gacgacggat cagtttcttg aatacca 57
<210> 105
   <211> 55
   <212> DNA
   <213> artificial
<220>
   <223> synthetic sequence
<400> 105
   ggaatgggga gcatctggct gaccgcactg aaattcctcg gcaaacacgc cgcaa 55
<210> 106
   <211> 57
   <212> DNA
   <213> artificial
<220>
   <223> synthetic sequence
<400> 106
   gtttggagga gcccagtttg gataattgct gttttgccag tttctttgcg gcgtgtt 57
<210> 107
   <211> 48
   <212> DNA
   <213> artificial
<220>
   <223> synthetic sequence
<400> 107
   ttggaatggg gagcaaaggc cgtggcaagc agggaggcaa agtacgtg 48
<210> 108
   <211> 48
   <212> DNA
   <213> artificial
<220>
   <223> synthetic sequence
<400> 108
   gtttggagga gcctgaggaa cgggtctttg cttttgcacg tactttgc 48
<210> 109
   <211> 39
   <212> DNA
   <213> artificial
<220>
   <223> synthetic sequence
<400> 109
   ttggaatggg gagcccgaac cgtgcaaaac gtgtaatca 39
<210> 110
   <211> 42
   <212> DNA
   <213> artificial
<220>
   <223> synthetic sequence
<400> 110
   tatttttgga ggagccggta cggaaggtgg tgattacacg tt 42
<210> 111
   <211> 39
   <212> DNA
   <213> artificial
<220>
   <223> synthetic sequence
<400> 111
   ttatgggctt cttcatcccg gcagtaatcc tgccctcca 39
<210> 112
   <211> 53
   <212> DNA
   <213> artificial
<220>
   <223> synthetic sequence
<400> 112
   tatttttgga tctgccgccc ggtacgatca ggaatgcgat ggagggcagg att 53
<210> 113
   <211> 47
   <212> DNA
   <213> artificial
<220>
   <223> synthetic sequence
<400> 113
   ttatgggcaa accgggctgg ctgatcaaaa ggcactggta ttcaaga 47
<210> 114
   <211> 64
   <212> DNA
   <213> artificial
<220>
   <223> synthetic sequence
<400> 114
   tatttttgga tctgccgcct gccagtctct tcagcggacg acggatcagt ttcttgaata 60
   ccag 64
<210> 115
   <211> 55
   <212> DNA
   <213> artificial
<220>
   <223> synthetic sequence
<400> 115
   ggaatgggga gcatctggct gaccgcactg aaattcctcg gcaaacacgc cgcaa 55
<210> 116
   <211> 60
   <212> DNA
   <213> artificial
<220>
   <223> synthetic sequence
<400> 116
   tatttttgga ggagcccagt ttggataatt gctgttttgc cagtttcttt gcggcgtgtt 60
<210> 117
   <211> 48
   <212> DNA
   <213> artificial
<220>
   <223> synthetic sequence
<400> 117
   ttggaatggg gagcaaaggc cgtggcaagc agggaggcaa agtacgtg 48
<210> 118
   <211> 51
   <212> DNA
   <213> artificial
<220>
   <223> synthetic sequence
<400> 118
   tatttttgga ggagcctgag gaacgggtct ttgcttttgc acgtactttg c 51
<210> 119
   <211> 60
   <212> DNA
   <213> artificial
<220>
   <223> synthetic sequence
<400> 119
   ggaatgggga gctcctccaa acgcaagaaa cgtaagaaac gcaaaaaaaa tagcgagaat 60
<210> 120
   <211> 24
   <212> DNA
   <213> artificial
<220>
   <223> synthetic sequence
<400> 120
   aactattccg agtgctttct ttgt 24
<210> 121
   <211> 57
   <212> DNA
   <213> artificial
<220>
   <223> synthetic sequence
<400> 121
   atgggatcca aacgcaagaa acgtaagaaa cgcaaaggct cctccccgaa ccgtgca 57
<210> 122
   <211> 57
   <212> DNA
   <213> artificial
<220>
   <223> synthetic sequence
<400> 122
   atgggatcca aacgcaagaa acgtaagaaa cgcaaagcct cctccttctt cgtagca 57
<210> 123
   <211> 33
   <212> DNA
   <213> artificial
<220>
   <223> synthetic sequence
<400> 123
   ggaatgggga gctcctccaa aaatagcgag aag 33
<210> 124
   <211> 24
   <212> DNA
   <213> artificial
<220>
   <223> synthetic sequence
<400> 124
   aactattccg tgtgctttct ttgt 24
<210> 125
   <211> 48
   <212> DNA
   <213> artificial
<220>
   <223> synthetic sequence
<400> 125
   atgggatcct tcttcgtagc accgggctcc tccaaaaata gcgagaag 48
<210> 126
   <211> 24
   <212> DNA
   <213> artificial
<220>
   <223> synthetic sequence
<400> 126
   aactattccg tgtgctttct ttgt 24
<210> 127
   <211> 298
   <212> PRT
   <213> artificial
<220>
   <223> synthetic sequence
<400> 127
   Met Lys Arg Lys Lys Arg Lys Lys Arg Lys Arg Gly Leu Arg Arg Leu
   1               5                   10                  15
   Gly Arg Lys Ile Ala His Gly Val Lys Lys Tyr Gly Pro Thr Val Leu
               20                  25                  30
   Arg Ile Ile Arg Ile Ala Gly Lys Val Leu Arg Lys Gly Asp Arg Gly
           35                  40                  45
   Asp Glu Val Cys Gln Leu Gln Thr Leu Leu Asn Leu Cys Gly Tyr Asp
       50                  55                  60
   Val Gly Lys Pro Asp Gly Ile Phe Gly Asn Asn Thr Phe Asn Gln Val
   65                  70                  75                  80
   Val Lys Phe Gln Lys Asp Asn Cys Leu Asp Ser Asp Gly Ile Val Gly
                   85                  90                  95
   Lys Asn Thr Trp Ala Glu Leu Phe Ser Lys Tyr Ser Pro Pro Ile Pro
               100                 105                 110
   Tyr Lys Thr Ile Pro Met Pro Thr Ala Asn Lys Ser Arg Ala Ala Ala
           115                 120                 125
   Thr Pro Val Met Asn Ala Val Glu Asn Ala Thr Gly Val Arg Ser Gln
       130                 135                 140
   Leu Leu Leu Thr Phe Ala Ser Ile Glu Ser Ala Phe Asp Tyr Glu Ile
   145                 150                 155                 160
   Lys Ala Lys Thr Ser Ser Ala Thr Gly Trp Phe Gln Phe Leu Thr Gly
                   165                 170                 175
   Thr Trp Lys Thr Met Ile Glu Asn Tyr Gly Met Lys Tyr Gly Val Leu
               180                 185                 190
   Thr Asp Pro Thr Gly Ala Leu Arg Lys Asp Pro Arg Ile Ser Ala Leu
           195                 200                 205
   Met Gly Ala Glu Leu Ile Lys Glu Asn Met Asn Ile Leu Arg Pro Val
       210                 215                 220
   Leu Lys Arg Glu Pro Thr Asp Thr Asp Leu Tyr Leu Ala His Phe Phe
   225                 230                 235                 240
   Gly Pro Gly Ala Ala Arg Arg Phe Leu Thr Thr Gly Gln Asn Glu Leu
                   245                 250                 255
   Ala Ala Thr His Phe Pro Lys Glu Ala Gln Ala Asn Pro Ser Ile Phe
               260                 265                 270
   Tyr Asn Lys Asp Gly Ser Pro Lys Thr Ile Gln Glu Val Tyr Asn Leu
           275                 280                 285
   Met Asp Gly Lys Val Ala Ala His Arg Lys
       290                 295
<210> 128
   <211> 622
   <212> PRT
   <213> artificial
<220>
   <223> synthetic sequence
<400> 128
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Lys Arg
               20                  25                  30
   Lys Lys Arg Lys Lys Arg Lys Asn Phe Arg Thr Lys Asn Gly Tyr Arg
           35                  40                  45
   Asp Leu Gln Ala Leu Val Lys Glu Leu Gly Leu Tyr Thr Gly Gln Ile
       50                  55                  60
   Asp Gly Val Trp Gly Lys Gly Thr Ser Ser Ser Thr Glu Thr Leu Leu
   65                  70                  75                  80
   Arg Gly Tyr Ala Glu Val Val Gly Lys Asn Thr Gly Gly Ile Gly Leu
                   85                  90                  95
   Pro Thr Thr Ser Asp Ala Ser Gly Tyr Asn Val Ile Thr Ala Leu Gln
               100                 105                 110
   Arg Asn Leu Ala Phe Leu Gly Leu Tyr Ser Leu Thr Val Asp Gly Ile
           115                 120                 125
   Trp Gly Asn Gly Thr Leu Ser Gly Leu Asp Lys Ala Phe Glu Val Tyr
       130                 135                 140
   Lys Glu Arg Tyr Arg Thr Pro Thr Tyr Asp Ile Ala Trp Ser Gly Lys
   145                 150                 155                 160
   Val Ser Pro Ala Phe Thr Ala Lys Val Lys Asp Trp Cys Gly Val His
                   165                 170                 175
   Val Pro Asn His Arg Ala Pro His Trp Leu Met Ala Cys Met Ala Phe
               180                 185                 190
   Glu Thr Gly Gln Thr Phe Ser Pro Ser Ile Lys Asn Ala Ala Gly Ser
           195                 200                 205
   Glu Ala Tyr Gly Leu Ile Gln Phe Met Ser Pro Ala Ala Asn Asp Leu
       210                 215                 220
   Asn Val Pro Leu Ser Val Ile Arg Ser Met Asp Gln Leu Thr Gln Leu
   225                 230                 235                 240
   Asp Leu Val Phe Lys Tyr Phe Glu Met Trp Met Lys Arg Gly Lys Arg
                   245                 250                 255
   Tyr Thr Gln Leu Glu Asp Phe Tyr Leu Thr Ile Phe His Pro Ala Ser
               260                 265                 270
   Val Gly Lys Lys Ala Asp Glu Val Leu Phe Leu Gln Gly Ser Lys Ala
           275                 280                 285
   Tyr Leu Gln Asn Lys Gly Phe Asp Val Asp Lys Asp Gly Lys Ile Thr
       290                 295                 300
   Leu Gly Glu Ile Ser Ser Thr Leu Tyr Thr Thr Tyr Tyr Lys Gly Leu
   305                 310                 315                 320
   Leu Pro Glu Asn Arg His Val Ile Ser Tyr Met Asn Phe Arg Thr Lys
                   325                 330                 335
   Asn Gly Tyr Arg Asp Leu Gln Ala Leu Val Lys Glu Leu Gly Leu Tyr
               340                 345                 350
   Thr Gly Gln Ile Asp Gly Val Trp Gly Lys Gly Thr Ser Ser Ser Thr
           355                 360                 365
   Glu Thr Leu Leu Arg Gly Tyr Ala Glu Val Val Gly Lys Asn Thr Gly
       370                 375                 380
   Gly Ile Gly Leu Pro Thr Thr Ser Asp Ala Ser Gly Tyr Asn Val Ile
   385                 390                 395                 400
   Thr Ala Leu Gln Arg Asn Leu Ala Phe Leu Gly Leu Tyr Ser Leu Thr
                   405                 410                 415
   Val Asp Gly Ile Trp Gly Asn Gly Thr Leu Ser Gly Leu Asp Lys Ala
               420                 425                 430
   Phe Glu Val Tyr Lys Glu Arg Tyr Arg Thr Pro Thr Tyr Asp Ile Ala
           435                 440                 445
   Trp Ser Gly Lys Val Ser Pro Ala Phe Thr Ala Lys Val Lys Asp Trp
       450                 455                 460
   Cys Gly Val His Val Pro Asn His Arg Ala Pro His Trp Leu Met Ala
   465                 470                 475                 480
   Cys Met Ala Phe Glu Thr Gly Gln Thr Phe Ser Pro Ser Ile Lys Asn
   485 490 495
   Ala Ala Gly Ser Glu Ala Tyr Gly Leu Ile Gln Phe Met Ser Pro Ala
   500 505 510
   Ala Asn Asp Leu Asn Val Pro Leu Ser Val Ile Arg Ser Met Asp Gln
   515 520 525
   Leu Thr Gln Leu Asp Leu Val Phe Lys Tyr Phe Glu Met Trp Met Lys
   530 535 540
   Arg Gly Lys Arg Tyr Thr Gln Leu Glu Asp Phe Tyr Leu Thr Ile Phe
   545 550 555 560
   His Pro Ala Ser Val Gly Lys Lys Ala Asp Glu Val Leu Phe Leu Gln
   565 570 575
   Gly Ser Lys Ala Tyr Leu Gln Asn Lys Gly Phe Asp Val Asp Lys Asp
   580 585 590
   Gly Lys Ile Thr Leu Gly Glu Ile Ser Ser Thr Leu Tyr Thr Thr Tyr
   595 600 605
   Tyr Lys Gly Leu Leu Pro Glu Asn Arg His Val Ile Ser Tyr
   610 615 620
<210> 129
   <211> 318
   <212> PRT
   <213> artificial
<220>
   <223> synthetic sequence
<400> 129
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Arg Gly
               20                  25                  30
   Leu Arg Arg Leu Gly Arg Lys Ile Ala His Gly Val Lys Lys Tyr Gly
           35                  40                  45
   Pro Thr Val Leu Arg Ile Ile Arg Ile Ala Gly Lys Val Leu Arg Lys
       50                  55                  60
   Gly Asp Arg Gly Asp Glu Val Cys Gln Leu Gln Thr Leu Leu Asn Leu
   65                  70                  75                  80
   Cys Gly Tyr Asp Val Gly Lys Pro Asp Gly Ile Phe Gly Asn Asn Thr
                   85                  90                  95
   Phe Asn Gln Val Val Lys Phe Gln Lys Asp Asn Cys Leu Asp Ser Asp
               100                 105                 110
   Gly Ile Val Gly Lys Asn Thr Trp Ala Glu Leu Phe Ser Lys Tyr Ser
           115                 120                 125
   Pro Pro Ile Pro Tyr Lys Thr Ile Pro Met Pro Thr Ala Asn Lys Ser
       130                 135                 140
   Arg Ala Ala Ala Thr Pro Val Met Asn Ala Val Glu Asn Ala Thr Gly
   145                 150                 155                 160
   Val Arg Ser Gln Leu Leu Leu Thr Phe Ala Ser Ile Glu Ser Ala Phe
                   165                 170                 175
   Asp Tyr Glu Ile Lys Ala Lys Thr Ser Ser Ala Thr Gly Trp Phe Gln
               180                 185                 190
   Phe Leu Thr Gly Thr Trp Lys Thr Met Ile Glu Asn Tyr Gly Met Lys
           195                 200                 205
   Tyr Gly Val Leu Thr Asp Pro Thr Gly Ala Leu Arg Lys Asp Pro Arg
       210                 215                 220
   Ile Ser Ala Leu Met Gly Ala Glu Leu Ile Lys Glu Asn Met Asn Ile
   225                 230                 235                 240
   Leu Arg Pro Val Leu Lys Arg Glu Pro Thr Asp Thr Asp Leu Tyr Leu
                   245                 250                 255
   Ala His Phe Phe Gly Pro Gly Ala Ala Arg Arg Phe Leu Thr Thr Gly
               260                 265                 270
   Gln Asn Glu Leu Ala Ala Thr His Phe Pro Lys Glu Ala Gln Ala Asn
           275                 280                 285
   Pro Ser Ile Phe Tyr Asn Lys Asp Gly Ser Pro Lys Thr Ile Gln Glu
       290                 295                 300
   Val Tyr Asn Leu Met Asp Gly Lys Val Ala Ala His Arg Lys
   305                 310                 315
<210> 130
   <211> 274
   <212> PRT
   <213> artificial
<220>
   <223> synthetic sequence
<400> 130
<210> 131
   <211> 313
   <212> PRT
   <213> artificial
<220>
   <223> synthetic sequence
<400> 131
   Met Gly Trp Leu Lys Lys Ile Gly Lys Lys Ile Glu Arg Val Gly Gln
   1 5 10 15
   His Thr Arg Asp Ala Thr Ile Gln Gly Leu Gly Ile Ala Gln Gln Ala
   20 25 30
   Ala Asn Val Ala Ala Thr Ala Arg Arg Gly Leu Arg Arg Leu Gly Arg
   35 40 45
   Lys Ile Ala His Gly Val Lys Lys Tyr Gly Pro Thr Val Leu Arg Ile
   50 55 60
   Ile Arg Ile Ala Gly Ala His Glu His Ser Ala Gln Trp Leu Asn Asn
   65 70 75 80
   Tyr Lys Lys Gly Tyr Gly Tyr Gly Pro Tyr Pro Leu Gly Ile Asn Gly
   85 90 95
   Gly Met His Tyr Gly Val Asp Phe Phe Met Asn Ile Gly Thr Pro Val
   100 105 110
   Lys Ala Ile Ser Ser Gly Lys Ile Val Glu Ala Gly Trp Ser Asn Tyr
   115 120 125
   Gly Gly Gly Asn Gln Ile Gly Leu Ile Glu Asn Asp Gly Val His Arg
   130 135 140
   Gln Trp Tyr Met His Leu Ser Lys Tyr Asn Val Lys Val Gly Asp Tyr
   145 150 155 160
   Val Lys Ala Gly Gln Ile Ile Gly Trp Ser Gly Ser Thr Gly Tyr Ser
   165 170 175
   Thr Ala Pro His Leu His Phe Gln Arg Met Val Asn Ser Phe Ser Asn
   180 185 190
   Ser Thr Ala Gln Asp Pro Met Pro Phe Leu Lys Ser Ala Gly Tyr Gly
   195 200 205
   Lys Ala Gly Gly Thr Val Thr Pro Thr Pro Asn Thr Gly Trp Lys Thr
   210 215 220
   Asn Lys Tyr Gly Thr Leu Tyr Lys Ser Glu Ser Ala Ser Phe Thr Pro
   225 230 235 240
   Asn Thr Asp Ile Ile Thr Arg Thr Thr Gly Pro Phe Arg Ser Met Pro
   245 250 255
   Gln Ser Gly Val Leu Lys Ala Gly Gln Thr Ile His Tyr Asp Glu Val
   260 265 270
   Met Lys Gln Asp Gly His Val Trp Val Gly Tyr Thr Gly Asn Ser Gly
   275 280 285
   Gln Arg Ile Tyr Leu Pro Val Arg Thr Trp Asn Lys Ser Thr Asn Thr
   290 295 300
   Leu Gly Val Leu Trp Gly Thr Ile Lys
   305 310
<210> 132
   <211> 313
   <212> PRT
   <213> artificial
<220>
   <223> synthetic sequence
<400> 132
   Met Ala His Glu His Ser Ala Gln Trp Leu Asn Asn Tyr Lys Lys Gly
   1 5 10 15
   Tyr Gly Tyr Gly Pro Tyr Pro Leu Gly Ile Asn Gly Gly Met His Tyr
   20 25 30
   Gly Val Asp Phe Phe Met Asn Ile Gly Thr Pro Val Lys Ala Ile Ser
           35                  40                  45
   Ser Gly Lys Ile Val Glu Ala Gly Trp Ser Asn Tyr Gly Gly Gly Asn
       50                  55                  60
   Gln Ile Gly Leu Ile Glu Asn Asp Gly Val His Arg Gln Trp Tyr Met
   65                  70                  75                  80
   His Leu Ser Lys Tyr Asn Val Lys Val Gly Asp Tyr Val Lys Ala Gly
                   85                  90                  95
   Gln Ile Ile Gly Trp Ser Gly Ser Thr Gly Tyr Ser Thr Ala Pro His
               100                 105                 110
   Leu His Phe Gln Arg Met Val Asn Ser Phe Ser Asn Ser Thr Ala Gln
           115                 120                 125
   Asp Pro Met Pro Phe Leu Lys Ser Ala Gly Tyr Gly Lys Ala Gly Gly
       130                 135                 140
   Thr Val Thr Pro Thr Pro Asn Thr Gly Trp Lys Thr Asn Lys Tyr Gly
   145                 150                 155                 160
   Thr Leu Tyr Lys Ser Glu Ser Ala Ser Phe Thr Pro Asn Thr Asp Ile
                   165                 170                 175
   Ile Thr Arg Thr Thr Gly Pro Phe Arg Ser Met Pro Gln Ser Gly Val
               180                 185                 190
   Leu Lys Ala Gly Gln Thr Ile His Tyr Asp Glu Val Met Lys Gln Asp
           195                 200                 205
   Gly His Val Trp Val Gly Tyr Thr Gly Asn Ser Gly Gln Arg Ile Tyr
       210                 215                 220
   Leu Pro Val Arg Thr Trp Asn Lys Ser Thr Asn Thr Leu Gly Val Leu
   225                 230                 235                 240
   Trp Gly Thr Ile Lys Gly Trp Leu Lys Lys Ile Gly Lys Lys Ile Glu
                   245                 250                 255
   Arg Val Gly Gln His Thr Arg Asp Ala Thr Ile Gln Gly Leu Gly Ile
               260                 265                 270
   Ala Gln Gln Ala Ala Asn Val Ala Ala Thr Ala Arg Arg Gly Leu Arg
           275                 280                 285
   Arg Leu Gly Arg Lys Ile Ala His Gly Val Lys Lys Tyr Gly Pro Thr
       290                 295                 300
   Val Leu Arg Ile Ile Arg Ile Ala Gly
   305                 310
<210> 133
   <211> 263
   <212> PRT
   <213> artificial
<220>
   <223> synthetic sequence
<400> 133
   Met Gly Ile Gly Ala Val Leu Lys Val Leu Thr Thr Gly Leu Pro Ala
   1               5                   10                  15
   Leu Ile Ser Trp Ile Lys Arg Lys Arg Gln Gln Arg Gly Leu Arg Arg
               20                  25                  30
   Leu Gly Arg Lys Ile Ala His Gly Val Lys Lys Tyr Gly Pro Thr Val
           35                  40                  45
   Leu Arg Ile Ile Arg Ile Ala Gly Ala Ile Ser Lys Lys Lys Val Gly
       50                  55                  60
   Gly Val Gly Gly Val Ile Ala Ala Ile Ile Ala Ala Val Phe Ala Val
   65                  70                  75                  80
   Glu Gly Gly Tyr Val Asn Asp Pro Lys Asp Pro Gly Gly Glu Thr Asn
                   85                  90                  95
   His Gly Val Thr Ile Gln Val Ala Gln Lys His Lys Gln Glu Leu Glu
               100                 105                 110
   Ser Met Tyr Asn Trp Asp Gly Ser Met Lys Asn Leu Thr Gln Glu Met
           115                 120                 125
   Ala Ser Ser Ile Tyr Tyr Asn Asp Tyr Ile Leu Lys Pro Gly Phe Val
       130                 135                 140
   Lys Phe Ala Asp Val Ser Pro Ala Val Thr Glu Lys Leu Val Asp Ala
   145                 150                 155                 160
   Gly Val Asn Thr Gly Pro Ala Arg Pro Ser Arg Trp Leu Gln Glu Ser
                   165                 170                 175
   Leu Asn Ala Phe Ser Arg Asn Gly Lys Asp Tyr Pro Lys Ile Gln Val
   180 185 190
   Asp Gly Lys Val Gly Ser Gly Thr Leu Ser Ala Tyr Lys Ser Leu Gln
   195 200 205
   Asn Lys Arg Gly Lys Val Glu Ala Cys Lys Leu Ile Leu Lys Ser Leu
   210 215 220
   Asp Gly Lys Gln Leu Asn Tyr Tyr Leu Ser Leu Asn Met Pro Glu Tyr
   225 230 235 240
   Thr Thr Gly Trp Ile Ala Asn Arg Ile Gly Asn Val Pro Leu Glu Arg
   245 250 255
   Cys Asn Glu Asp Ile Val Asn
   260
<210> 134
   <211> 400
   <212> PRT
   <213> artificial
<220>
   <223> synthetic sequence
<400> 134
<210> 135
   <211> 385
   <212> PRT
   <213> artificial
<220>
   <223> synthetic sequence
<400> 135
   Met Gly Ile Gly Lys Phe Leu His Ser Ala Lys Lys Phe Gly Lys Ala
   1               5                   10                  15
   Phe Val Gly Glu Ile Met Asn Ser Thr Arg Ser Ser Arg Ala Gly Leu
               20                  25                  30
   Gln Phe Pro Val Gly Arg Val His Arg Leu Leu Arg Lys Val Lys Tyr
           35                  40                  45
   Thr Val Glu Asn Lys Ile Ile Ala Gly Leu Pro Lys Gly Lys Leu Lys
       50                  55                  60
   Gly Ala Asn Phe Val Ile Ala His Glu Thr Ala Asn Ser Lys Ser Thr
   65                  70                  75                  80
   Ile Asp Asn Glu Val Ser Tyr Met Thr Arg Asn Trp Lys Asn Ala Phe
                   85                  90                  95
   Val Thr His Phe Val Gly Gly Gly Gly Arg Val Val Gln Val Ala Asn
               100                 105                 110
   Val Asn Tyr Val Ser Trp Gly Ala Gly Gln Tyr Ala Asn Ser Tyr Ser
           115                 120                 125
   Tyr Ala Gln Val Glu Leu Cys Arg Thr Ser Asn Ala Thr Thr Phe Lys
       130                 135                 140
   Lys Asp Tyr Glu Val Tyr Cys Gln Leu Leu Val Asp Leu Ala Lys Lys
   145                 150                 155                 160
   Ala Gly Ile Pro Ile Thr Leu Asp Ser Gly Ser Lys Thr Ser Asp Lys
                   165                 170                 175
   Gly Ile Lys Ser His Lys Trp Val Ala Asp Lys Leu Gly Gly Thr Thr
               180                 185                 190
   His Gln Asp Pro Tyr Ala Tyr Leu Ser Ser Trp Gly Ile Ser Lys Ala
           195                 200                 205
   Gln Phe Ala Ser Asp Leu Ala Lys Val Ser Gly Gly Gly Asn Thr Gly
       210                 215                 220
   Thr Ala Pro Ala Lys Pro Ser Thr Pro Ala Pro Lys Pro Ser Thr Pro
   225                 230                 235                 240
   Ser Thr Asn Leu Asp Lys Leu Gly Leu Val Asp Tyr Met Asn Ala Lys
                   245                 250                 255
   Lys Met Asp Ser Ser Tyr Ser Asn Arg Asp Lys Leu Ala Lys Gln Tyr
               260                 265                 270
   Gly Ile Ala Asn Tyr Ser Gly Thr Ala Ser Gln Asn Thr Thr Leu Leu
           275                 280                 285
   Ser Lys Ile Lys Gly Gly Ala Pro Lys Pro Ser Thr Pro Ala Pro Lys
       290                 295                 300
   Pro Ser Thr Ser Thr Ala Lys Lys Ile Tyr Phe Pro Pro Asn Lys Gly
   305                 310                 315                 320
   Asn Trp Ser Val Tyr Pro Thr Asn Lys Ala Pro Val Lys Ala Asn Ala
                   325                 330                 335
   Ile Gly Ala Ile Asn Pro Thr Lys Phe Gly Gly Leu Thr Tyr Thr Ile
               340                 345                 350
   Gln Lys Asp Arg Gly Asn Gly Val Tyr Glu Ile Gln Thr Asp Gln Phe
           355                 360                 365
   Gly Arg Val Gln Val Tyr Gly Ala Pro Ser Thr Gly Ala Val Ile Lys
       370                 375                 380
   Lys
   385
<210> 136
   <211> 243
   <212> PRT
   <213> artificial
<220>
   <223> synthetic sequence
<400> 136
<210> 137
   <211> 232
   <212> PRT
   <213> artificial
<220>
   <223> synthetic sequence
<400> 137
   Met Gly Ile Gly Ala Val Leu Lys Val Leu Thr Thr Gly Leu Pro Ala
   1               5                   10                  15
   Leu Ile Ser Trp Ile Lys Arg Lys Arg Gln Gln Arg Gly Leu Arg Arg
               20                  25                  30
   Leu Gly Arg Lys Ile Ala His Gly Val Lys Lys Tyr Gly Pro Thr Val
           35                  40                  45
   Leu Arg Ile Ile Arg Ile Ala Gly Asn Pro Ile Ile Asp Gly Ile Ile
       50                  55                  60
   Ala Leu Glu Gly Gly Tyr Val Phe Asn Pro Lys Asp Lys Gly Gly Ala
   65                  70                  75                  80
   Thr His Trp Gly Ile Thr Glu Ala Thr Ala Arg Ala His Gly Tyr Ala
                   85                  90                  95
   Gly Asp Met Arg Asp Leu Thr His Ala Glu Ala Tyr Ala Ile Leu Glu
               100                 105                 110
   Glu Asp Tyr Trp Ile Lys Pro Gly Phe Asp Val Ile Ser Thr Leu Ser
           115                 120                 125
   Trp Pro Val Ser Phe Glu Leu Cys Asp Ala Ala Val Asn Ile Gly Ala
       130                 135                 140
   Tyr His Pro Ser Ala Trp Leu Gln Arg Trp Leu Asn Val Phe Asn His
   145                 150                 155                 160
   Glu Gly Lys Arg Tyr Pro Asp Ile His Val Asp Gly Asn Ile Gly Pro
                   165                 170                 175
   Arg Thr Leu Ala Ala Leu Glu His Tyr Leu Ala Trp Arg Gly Gln Glu
               180                 185                 190
   Gly Glu Ala Val Leu Val Lys Ala Leu Asn Cys Ser Gln Gly Thr Tyr
           195                 200                 205
   Tyr Leu Asn Val Ala Glu Lys Asn His Asn Asn Glu Gln Phe Ile Tyr
       210                 215                 220
   Gly Trp Ile Lys Asn Arg Val Thr
   225                 230
<210> 138
   <211> 376
   <212> PRT
   <213> artificial
<220>
   <223> synthetic sequence
<400> 138
   Met Ile Leu Pro Trp Lys Trp Pro Trp Trp Pro Trp Arg Arg Gly Leu
   1               5                   10                  15
   Asn Thr Leu Lys Lys Val Phe Gln Gly Leu His Glu Ala Ile Lys Leu
               20                  25                  30
   Ile Asn Asn His Val Gln Val Lys Lys Asn Asp Leu Phe Val Asp Val
           35                  40                  45
   Ser Ser His Asn Gly Tyr Asp Ile Thr Gly Ile Leu Glu Gln Met Gly
       50                  55                  60
   Thr Thr Asn Thr Ile Ile Lys Ile Ser Glu Ser Thr Thr Tyr Leu Asn
   65                  70                  75                  80
   Pro Cys Leu Ser Ala Gln Val Glu Gln Ser Asn Pro Ile Gly Phe Tyr
                   85                  90                  95
   His Phe Ala Arg Phe Gly Gly Asp Val Ala Glu Ala Glu Arg Glu Ala
               100                 105                 110
   Gln Phe Phe Leu Asp Asn Val Pro Met Gln Val Lys Tyr Leu Val Leu
           115                 120                 125
   Asp Tyr Glu Asp Asp Pro Ser Gly Asp Ala Gln Ala Asn Thr Asn Ala
       130                 135                 140
   Cys Leu Arg Phe Met Gln Met Ile Ala Asp Ala Gly Tyr Lys Pro Ile
   145                 150                 155                 160
   Tyr Tyr Ser Tyr Lys Pro Phe Thr His Asp Asn Val Asp Tyr Gln Gln
                   165                 170                 175
   Ile Leu Ala Gln Phe Pro Asn Ser Leu Trp Ile Ala Gly Tyr Gly Leu
   180 185 190
   Asn Asp Gly Thr Ala Asn Phe Glu Tyr Phe Pro Ser Met Asp Gly Ile
   195 200 205
   Arg Trp Trp Gln Tyr Ser Ser Asn Pro Phe Asp Lys Asn Ile Val Leu
   210 215 220
   Leu Asp Asp Glu Glu Asp Asp Lys Pro Lys Thr Ala Gly Thr Trp Lys
   225 230 235 240
   Gln Asp Ser Lys Gly Trp Trp Phe Arg Arg Asn Asn Gly Ser Phe Pro
   245 250 255
   Tyr Asn Lys Trp Glu Lys Ile Gly Gly Val Trp Tyr Tyr Phe Asp Ser
   260 265 270
   Lys Gly Tyr Cys Leu Thr Ser Glu Trp Leu Lys Asp Asn Glu Lys Trp
   275 280 285
   Tyr Tyr Leu Lys Asp Asn Gly Ala Met Ala Thr Gly Trp Val Leu Val
   290 295 300
   Gly Ser Glu Trp Tyr Tyr Met Asp Asp Ser Gly Ala Met Val Thr Gly
   305 310 315 320
   Trp Val Lys Tyr Lys Asn Asn Trp Tyr Tyr Met Thr Asn Glu Arg Gly
   325 330 335
   Asn Met Val Ser Asn Glu Phe Ile Lys Ser Gly Lys Gly Trp Tyr Phe
   340 345 350
   Met Asn Thr Asn Gly Glu Leu Ala Asp Asn Pro Ser Phe Thr Lys Glu
   355 360 365
   Pro Asp Gly Leu Ile Thr Val Ala
   370 375
<210> 139
   <211> 549
   <212> PRT
   <213> artificial
<220>
   <223> synthetic sequence
<400> 139
   Met Ala Lys Thr Gln Ala Glu Ile Asn Lys Arg Leu Asp Ala Tyr Ala
   1               5                   10                  15
   Lys Gly Thr Val Asp Ser Pro Tyr Arg Val Lys Lys Ala Thr Ser Tyr
               20                  25                  30
   Asp Pro Ser Phe Gly Val Met Glu Ala Gly Ala Ile Asp Ala Asp Gly
           35                  40                  45
   Tyr Tyr His Ala Gln Cys Gln Asp Leu Ile Thr Asp Tyr Val Leu Trp
       50                  55                  60
   Leu Thr Asp Asn Lys Val Arg Thr Trp Gly Asn Ala Lys Asp Gln Ile
   65                  70                  75                  80
   Lys Gln Ser Tyr Gly Thr Gly Phe Lys Ile His Glu Asn Lys Pro Ser
                   85                  90                  95
   Thr Val Pro Lys Lys Gly Trp Ile Ala Val Phe Thr Ser Gly Ser Tyr
               100                 105                 110
   Glu Gln Trp Gly His Ile Gly Ile Val Tyr Asp Gly Gly Asn Thr Ser
           115                 120                 125
   Thr Phe Thr Ile Leu Glu Gln Asn Trp Asn Gly Tyr Ala Asn Lys Lys
       130                 135                 140
   Pro Thr Lys Arg Val Asp Asn Tyr Tyr Gly Leu Thr His Phe Ile Glu
   145                 150                 155                 160
   Ile Pro Val Lys Ala Gly Thr Thr Val Lys Lys Glu Thr Ala Lys Lys
                   165                 170                 175
   Ser Ala Ser Lys Thr Pro Ala Pro Lys Lys Lys Ala Thr Leu Lys Val
               180                 185                 190
   Ser Lys Asn His Ile Asn Tyr Thr Met Asp Lys Arg Gly Lys Lys Pro
           195                 200                 205
   Glu Gly Met Val Ile His Asn Asp Ala Gly Arg Ser Ser Gly Gln Gln
       210                 215                 220
   Tyr Glu Asn Ser Leu Ala Asn Ala Gly Tyr Ala Arg Tyr Ala Asn Gly
   225                 230                 235                 240
   Ile Ala His Tyr Tyr Gly Ser Glu Gly Tyr Val Trp Glu Ala Ile Asp
                   245                 250                 255
   Ala Lys Asn Gln Ile Ala Trp His Thr Gly Asp Gly Thr Gly Ala Asn
               260                 265                 270
   Ser Gly Asn Phe Arg Phe Ala Gly Ile Glu Val Cys Gln Ser Met Ser
           275                 280                 285
   Ala Ser Asp Ala Gln Phe Leu Lys Asn Glu Gln Ala Val Phe Gln Phe
       290                 295                 300
   Thr Ala Glu Lys Phe Lys Glu Trp Gly Leu Thr Pro Asn Arg Lys Thr
   305                 310                 315                 320
   Val Arg Leu His Met Glu Phe Val Pro Thr Ala Cys Pro His Arg Ser
                   325                 330                 335
   Met Val Leu His Thr Gly Phe Asn Pro Val Thr Gln Gly Arg Pro Ser
               340                 345                 350
   Gln Ala Ile Met Asn Lys Leu Lys Asp Tyr Phe Ile Lys Gln Ile Lys
           355                 360                 365
   Asn Tyr Met Asp Lys Gly Thr Ser Ser Ser Thr Val Val Lys Asp Gly
       370                 375                 380
   Lys Thr Ser Ser Ala Ser Thr Pro Ala Thr Arg Pro Val Thr Gly Ser
   385                 390                 395                 400
   Trp Lys Lys Asn Gln Tyr Gly Thr Trp Tyr Lys Pro Glu Asn Ala Thr
                   405                 410                 415
   Phe Val Asn Gly Asn Gln Pro Ile Val Thr Arg Ile Gly Ser Pro Phe
               420                 425                 430
   Leu Asn Ala Pro Val Gly Gly Asn Leu Pro Ala Gly Ala Thr Ile Val
           435                 440                 445
   Tyr Asp Glu Val Cys Ile Gln Ala Gly His Ile Trp Ile Gly Tyr Asn
       450                 455                 460
   Ala Tyr Asn Gly Asn Arg Val Tyr Cys Pro Val Arg Thr Cys Gln Gly
   465                 470                 475                 480
   Val Pro Pro Asn Gln Ile Pro Gly Val Ala Trp Gly Val Phe Lys Lys
                   485                 490                 495
   Arg Lys Lys Arg Lys Lys Arg Lys Lys Arg Lys Lys Arg Lys Lys Arg
               500                 505                 510
   Lys Gly Gly Leu Lys Lys Leu Gly Lys Lys Leu Glu Gly Ala Gly Lys
   515 520 525
   Arg Val Phe Asn Ala Ala Glu Lys Ala Leu Pro Val Val Ala Gly Ala
   530 535 540
   Lys Ala Leu Arg Lys
   545
<210> 140
   <211> 298
   <212> PRT
   <213> artificial
<220>
   <223> synthetic peptide
<400> 140
   Met Lys Val Leu Arg Lys Gly Asp Arg Gly Asp Glu Val Cys Gln Leu
   1 5 10 15
   Gln Thr Leu Leu Asn Leu Cys Gly Tyr Asp Val Gly Lys Pro Asp Gly
   20 25 30
   Ile Phe Gly Asn Asn Thr Phe Asn Gln Val Val Lys Phe Gln Lys Asp
   35 40 45
   Asn Cys Leu Asp Ser Asp Gly Ile Val Gly Lys Asn Thr Trp Ala Glu
   50 55 60
   Leu Phe Ser Lys Tyr Ser Pro Pro Ile Pro Tyr Lys Thr Ile Pro Met
   65 70 75 80
   Pro Thr Ala Asn Lys Ser Arg Ala Ala Ala Thr Pro Val Met Asn Ala
   85 90 95
   Val Glu Asn Ala Thr Gly Val Arg Ser Gln Leu Leu Leu Thr Phe Ala
   100 105 110
   Ser Ile Glu Ser Ala Phe Asp Tyr Glu Ile Lys Ala Lys Thr Ser Ser
   115 120 125
   Ala Thr Gly Trp Phe Gln Phe Leu Thr Gly Thr Trp Lys Thr Met Ile
   130 135 140
   Glu Asn Tyr Gly Met Lys Tyr Gly Val Leu Thr Asp Pro Thr Gly Ala
   145 150 155 160
   Leu Arg Lys Asp Pro Arg Ile Ser Ala Leu Met Gly Ala Glu Leu Ile
   165 170 175
   Lys Glu Asn Met Asn Ile Leu Arg Pro Val Leu Lys Arg Glu Pro Thr
   180 185 190
   Asp Thr Asp Leu Tyr Leu Ala His Phe Phe Gly Pro Gly Ala Ala Arg
   195 200 205
   Arg Phe Leu Thr Thr Gly Gln Asn Glu Leu Ala Ala Thr His Phe Pro
   210 215 220
   Lys Glu Ala Gln Ala Asn Pro Ser Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys Arg Gly Leu Arg Arg Leu Gly Arg Lys Ile Ala His
   260 265 270
   Gly Val Lys Lys Tyr Gly Pro Thr Val Leu Arg Ile Ile Arg Ile Ala
   275 280 285
   Gly Lys Arg Lys Lys Arg Lys Lys Arg Lys
   290 295
<210> 141
   <211> 329
   <212> PRT
   <213> artificial
<220>
   <223> synthetic sequence
<400> 141
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Lys Arg Lys Lys Arg Lys Lys Arg Lys Arg Gly Leu Arg
   290 295 300
   Arg Leu Gly Arg Lys Ile Ala His Gly Val Lys Lys Tyr Gly Pro Thr
   305 310 315 320
   Val Leu Arg Ile Ile Arg Ile Ala Gly
   325
<210> 142
   <211> 243
   <212> PRT
   <213> artificial
<220>
   <223> synthetic sequence
<400> 142
<210> 143
   <211> 232
   <212> PRT
   <213> artificial
<220>
   <223> synthetic peptide
<400> 143
   Met Asn Pro Ile Ile Asp Gly Ile Ile Ala Leu Glu Gly Gly Tyr Val
   1               5                   10                  15
   Phe Asn Pro Lys Asp Lys Gly Gly Ala Thr His Trp Gly Ile Thr Glu
               20                  25                  30
   Ala Thr Ala Arg Ala His Gly Tyr Ala Gly Asp Met Arg Asp Leu Thr
           35                  40                  45
   His Ala Glu Ala Tyr Ala Ile Leu Glu Glu Asp Tyr Trp Ile Lys Pro
       50                  55                  60
   Gly Phe Asp Val Ile Ser Thr Leu Ser Trp Pro Val Ser Phe Glu Leu
   65                  70                  75                  80
   Cys Asp Ala Ala Val Asn Ile Gly Ala Tyr His Pro Ser Ala Trp Leu
                   85                  90                  95
   Gln Arg Trp Leu Asn Val Phe Asn His Glu Gly Lys Arg Tyr Pro Asp
               100                 105                 110
   Ile His Val Asp Gly Asn Ile Gly Pro Arg Thr Leu Ala Ala Leu Glu
           115                 120                 125
   His Tyr Leu Ala Trp Arg Gly Gln Glu Gly Glu Ala Val Leu Val Lys
       130                 135                 140
   Ala Leu Asn Cys Ser Gln Gly Thr Tyr Tyr Leu Asn Val Ala Glu Lys
   145                 150                 155                 160
   Asn His Asn Asn Glu Gln Phe Ile Tyr Gly Trp Ile Lys Asn Arg Val
                   165                 170                 175
   Thr Arg Gly Leu Arg Arg Leu Gly Arg Lys Ile Ala His Gly Val Lys
               180                 185                 190
   Lys Tyr Gly Pro Thr Val Leu Arg Ile Ile Arg Ile Ala Gly Gly Ile
           195                 200                 205
   Gly Ala Val Leu Lys Val Leu Thr Thr Gly Leu Pro Ala Leu Ile Ser
       210                 215                 220
   Trp Ile Lys Arg Lys Arg Gln Gln
   225                 230
<210> 144
   <211> 313
   <212> PRT
   <213> artificial
<220>
   <223> synthetic sequence
<400> 144
   Met Ala His Glu His Ser Ala Gln Trp Leu Asn Asn Tyr Lys Lys Gly
   1               5                   10                  15
   Tyr Gly Tyr Gly Pro Tyr Pro Leu Gly Ile Asn Gly Gly Met His Tyr
               20                  25                  30
   Gly Val Asp Phe Phe Met Asn Ile Gly Thr Pro Val Lys Ala Ile Ser
           35                  40                  45
   Ser Gly Lys Ile Val Glu Ala Gly Trp Ser Asn Tyr Gly Gly Gly Asn
       50                  55                  60
   Gln Ile Gly Leu Ile Glu Asn Asp Gly Val His Arg Gln Trp Tyr Met
   65                  70                  75                  80
   His Leu Ser Lys Tyr Asn Val Lys Val Gly Asp Tyr Val Lys Ala Gly
                   85                  90                  95
   Gln Ile Ile Gly Trp Ser Gly Ser Thr Gly Tyr Ser Thr Ala Pro His
               100                 105                 110
   Leu His Phe Gln Arg Met Val Asn Ser Phe Ser Asn Ser Thr Ala Gln
           115                 120                 125
   Asp Pro Met Pro Phe Leu Lys Ser Ala Gly Tyr Gly Lys Ala Gly Gly
       130                 135                 140
   Thr Val Thr Pro Thr Pro Asn Thr Gly Trp Lys Thr Asn Lys Tyr Gly
   145                 150                 155                 160
   Thr Leu Tyr Lys Ser Glu Ser Ala Ser Phe Thr Pro Asn Thr Asp Ile
                   165                 170                 175
   Ile Thr Arg Thr Thr Gly Pro Phe Arg Ser Met Pro Gln Ser Gly Val
               180                 185                 190
   Leu Lys Ala Gly Gln Thr Ile His Tyr Asp Glu Val Met Lys Gln Asp
           195                 200                 205
   Gly His Val Trp Val Gly Tyr Thr Gly Asn Ser Gly Gln Arg Ile Tyr
       210                 215                 220
   Leu Pro Val Arg Thr Trp Asn Lys Ser Thr Asn Thr Leu Gly Val Leu
   225                 230                 235                 240
   Trp Gly Thr Ile Lys Arg Gly Leu Arg Arg Leu Gly Arg Lys Ile Ala
                   245                 250                 255
   His Gly Val Lys Lys Tyr Gly Pro Thr Val Leu Arg Ile Ile Arg Ile
               260                 265                 270
   Ala Gly Gly Trp Leu Lys Lys Ile Gly Lys Lys Ile Glu Arg Val Gly
           275                 280                 285
   Gln His Thr Arg Asp Ala Thr Ile Gln Gly Leu Gly Ile Ala Gln Gln
       290                 295                 300
   Ala Ala Asn Val Ala Ala Thr Ala Arg
   305                 310
<210> 145
   <211> 376
   <212> PRT
   <213> artificial
<220>
   <223> synthetic sequence
<400> 145
   Met Val Lys Lys Asn Asp Leu Phe Val Asp Val Ser Ser His Asn Gly
   1               5                   10                  15
   Tyr Asp Ile Thr Gly Ile Leu Glu Gln Met Gly Thr Thr Asn Thr Ile
               20                  25                  30
   Ile Lys Ile Ser Glu Ser Thr Thr Tyr Leu Asn Pro Cys Leu Ser Ala
           35                  40                  45
   Gln Val Glu Gln Ser Asn Pro Ile Gly Phe Tyr His Phe Ala Arg Phe
       50                  55                  60
   Gly Gly Asp Val Ala Glu Ala Glu Arg Glu Ala Gln Phe Phe Leu Asp
   65                  70                  75                  80
   Asn Val Pro Met Gln Val Lys Tyr Leu Val Leu Asp Tyr Glu Asp Asp
                   85                  90                  95
   Pro Ser Gly Asp Ala Gln Ala Asn Thr Asn Ala Cys Leu Arg Phe Met
               100                 105                 110
   Gln Met Ile Ala Asp Ala Gly Tyr Lys Pro Ile Tyr Tyr Ser Tyr Lys
           115                 120                 125
   Pro Phe Thr His Asp Asn Val Asp Tyr Gln Gln Ile Leu Ala Gln Phe
       130                 135                 140
   Pro Asn Ser Leu Trp Ile Ala Gly Tyr Gly Leu Asn Asp Gly Thr Ala
   145                 150                 155                 160
   Asn Phe Glu Tyr Phe Pro Ser Met Asp Gly Ile Arg Trp Trp Gln Tyr
                   165                 170                 175
   Ser Ser Asn Pro Phe Asp Lys Asn Ile Val Leu Leu Asp Asp Glu Glu
               180                 185                 190
   Asp Asp Lys Pro Lys Thr Ala Gly Thr Trp Lys Gln Asp Ser Lys Gly
           195                 200                 205
   Trp Trp Phe Arg Arg Asn Asn Gly Ser Phe Pro Tyr Asn Lys Trp Glu
       210                 215                 220
   Lys Ile Gly Gly Val Trp Tyr Tyr Phe Asp Ser Lys Gly Tyr Cys Leu
   225                 230                 235                 240
   Thr Ser Glu Trp Leu Lys Asp Asn Glu Lys Trp Tyr Tyr Leu Lys Asp
                   245                 250                 255
   Asn Gly Ala Met Ala Thr Gly Trp Val Leu Val Gly Ser Glu Trp Tyr
               260                 265                 270
   Tyr Met Asp Asp Ser Gly Ala Met Val Thr Gly Trp Val Lys Tyr Lys
   275 280 285
   Asn Asn Trp Tyr Tyr Met Thr Asn Glu Arg Gly Asn Met Val Ser Asn
   290 295 300
   Glu Phe Ile Lys Ser Gly Lys Gly Trp Tyr Phe Met Asn Thr Asn Gly
   305 310 315 320
   Glu Leu Ala Asp Asn Pro Ser Phe Thr Lys Glu Pro Asp Gly Leu Ile
   325 330 335
   Thr Val Ala Gly Leu Asn Thr Leu Lys Lys Val Phe Gln Gly Leu His
   340 345 350
   Glu Ala Ile Lys Leu Ile Asn Asn His Val Gln Ile Leu Pro Trp Lys
   355 360 365
   Trp Pro Trp Trp Pro Trp Arg Arg
   370 375
<210> 146
   <211> 385
   <212> PRT
   <213> artificial
<220>
   <223> synthetic sequence
<400> 146
   Met Val Lys Tyr Thr Val Glu Asn Lys Ile Ile Ala Gly Leu Pro Lys
   1 5 10 15
   Gly Lys Leu Lys Gly Ala Asn Phe Val Ile Ala His Glu Thr Ala Asn
   20 25 30
   Ser Lys Ser Thr Ile Asp Asn Glu Val Ser Tyr Met Thr Arg Asn Trp
   35 40 45
   Lys Asn Ala Phe Val Thr His Phe Val Gly Gly Gly Gly Arg Val Val
   50 55 60
   Gln Val Ala Asn Val Asn Tyr Val Ser Trp Gly Ala Gly Gln Tyr Ala
   65 70 75 80
   Asn Ser Tyr Ser Tyr Ala Gln Val Glu Leu Cys Arg Thr Ser Asn Ala
   85 90 95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys Thr Arg Ser Ser Arg Ala Gly Leu Gln Phe Pro
               340                 345                 350
   Val Gly Arg Val His Arg Leu Leu Arg Lys Gly Ile Gly Lys Phe Leu
   355 360 365
   His Ser Ala Lys Lys Phe Gly Lys Ala Phe Val Gly Glu Ile Met Asn
   370 375 380
   Ser
   385
<210> 147
   <211> 549
   <212> PRT
   <213> artificial
<220>
   <223> synthetic sequence
<400> 147
   Met Lys Arg Lys Lys Arg Lys Lys Arg Lys Lys Arg Lys Lys Arg Lys
   1 5 10 15
   Lys Arg Lys Gly Gly Leu Lys Lys Leu Gly Lys Lys Leu Glu Gly Ala
   20 25 30
   Gly Lys Arg Val Phe Asn Ala Ala Glu Lys Ala Leu Pro Val Val Ala
   35 40 45
   Gly Ala Lys Ala Leu Arg Lys Ala Lys Thr Gln Ala Glu Ile Asn Lys
   50 55 60
   Arg Leu Asp Ala Tyr Ala Lys Gly Thr Val Asp Ser Pro Tyr Arg Val
   65 70 75 80
   Lys Lys Ala Thr Ser Tyr Asp Pro Ser Phe Gly Val Met Glu Ala Gly
   85 90 95
   Ala Ile Asp Ala Asp Gly Tyr Tyr His Ala Gln Cys Gln Asp Leu Ile
   100 105 110
   Thr Asp Tyr Val Leu Trp Leu Thr Asp Asn Lys Val Arg Thr Trp Gly
   115 120 125
   Asn Ala Lys Asp Gln Ile Lys Gln Ser Tyr Gly Thr Gly Phe Lys Ile
   130 135 140
   His Glu Asn Lys Pro Ser Thr Val Pro Lys Lys Gly Trp Ile Ala Val
   145 150 155 160
   Phe Thr Ser Gly Ser Tyr Glu Gln Trp Gly His Ile Gly Ile Val Tyr
                   165                 170                 175
   Asp Gly Gly Asn Thr Ser Thr Phe Thr Ile Leu Glu Gln Asn Trp Asn
               180                 185                 190
   Gly Tyr Ala Asn Lys Lys Pro Thr Lys Arg Val Asp Asn Tyr Tyr Gly
           195                 200                 205
   Leu Thr His Phe Ile Glu Ile Pro Val Lys Ala Gly Thr Thr Val Lys
       210                 215                 220
   Lys Glu Thr Ala Lys Lys Ser Ala Ser Lys Thr Pro Ala Pro Lys Lys
   225                 230                 235                 240
   Lys Ala Thr Leu Lys Val Ser Lys Asn His Ile Asn Tyr Thr Met Asp
                   245                 250                 255
   Lys Arg Gly Lys Lys Pro Glu Gly Met Val Ile His Asn Asp Ala Gly
               260                 265                 270
   Arg Ser Ser Gly Gln Gln Tyr Glu Asn Ser Leu Ala Asn Ala Gly Tyr
           275                 280                 285
   Ala Arg Tyr Ala Asn Gly Ile Ala His Tyr Tyr Gly Ser Glu Gly Tyr
       290                 295                 300
   Val Trp Glu Ala Ile Asp Ala Lys Asn Gln Ile Ala Trp His Thr Gly
   305                 310                 315                 320
   Asp Gly Thr Gly Ala Asn Ser Gly Asn Phe Arg Phe Ala Gly Ile Glu
                   325                 330                 335
   Val Cys Gln Ser Met Ser Ala Ser Asp Ala Gln Phe Leu Lys Asn Glu
               340                 345                 350
   Gln Ala Val Phe Gln Phe Thr Ala Glu Lys Phe Lys Glu Trp Gly Leu
           355                 360                 365
   Thr Pro Asn Arg Lys Thr Val Arg Leu His Met Glu Phe Val Pro Thr
       370                 375                 380
   Ala Cys Pro His Arg Ser Met Val Leu His Thr Gly Phe Asn Pro Val
   385                 390                 395                 400
   Thr Gln Gly Arg Pro Ser Gln Ala Ile Met Asn Lys Leu Lys Asp Tyr
                   405                 410                 415
   Phe Ile Lys Gln Ile Lys Asn Tyr Met Asp Lys Gly Thr Ser Ser Ser
   420 425 430
   Thr Val Val Lys Asp Gly Lys Thr Ser Ser Ala Ser Thr Pro Ala Thr
   435 440 445
   Arg Pro Val Thr Gly Ser Trp Lys Lys Asn Gln Tyr Gly Thr Trp Tyr
   450 455 460
   Lys Pro Glu Asn Ala Thr Phe Val Asn Gly Asn Gln Pro Ile Val Thr
   465 470 475 480
   Arg Ile Gly Ser Pro Phe Leu Asn Ala Pro Val Gly Gly Asn Leu Pro
   485 490 495
   Ala Gly Ala Thr Ile Val Tyr Asp Glu Val Cys Ile Gln Ala Gly His
   500 505 510
   Ile Trp Ile Gly Tyr Asn Ala Tyr Asn Gly Asn Arg Val Tyr Cys Pro
   515 520 525
   Val Arg Thr Cys Gln Gly Val Pro Pro Asn Gln Ile Pro Gly Val Ala
   530 535 540
   Trp Gly Val Phe Lys
   545
<210> 148
   <211> 269
   <212> PRT
   <213> artificial
<220>
   <223> synthetic sequence
<400> 148
   Met Lys Val Leu Arg Lys Gly Asp Arg Gly Asp Glu Val Cys Gln Leu
   1 5 10 15
   Gln Thr Leu Leu Asn Leu Cys Gly Tyr Asp Val Gly Lys Pro Asp Gly
   20 25 30
   Ile Phe Gly Asn Asn Thr Phe Asn Gln Val Val Lys Phe Gln Lys Asp
   35 40 45
   Asn Cys Leu Asp Ser Asp Gly Ile Val Gly Lys Asn Thr Trp Ala Glu
   50 55 60
   Leu Phe Ser Lys Tyr Ser Pro Pro Ile Pro Tyr Lys Thr Ile Pro Met
   65 70 75 80
   Pro Thr Ala Asn Lys Ser Arg Ala Ala Ala Thr Pro Val Met Asn Ala
   85 90 95
   Val Glu Asn Ala Thr Gly Val Arg Ser Gln Leu Leu Leu Thr Phe Ala
   100 105 110
   Ser Ile Glu Ser Ala Phe Asp Tyr Glu Ile Lys Ala Lys Thr Ser Ser
   115 120 125
   Ala Thr Gly Trp Phe Gln Phe Leu Thr Gly Thr Trp Lys Thr Met Ile
   130 135 140
   Glu Asn Tyr Gly Met Lys Tyr Gly Val Leu Thr Asp Pro Thr Gly Ala
   145 150 155 160
   Leu Arg Lys Asp Pro Arg Ile Ser Ala Leu Met Gly Ala Glu Leu Ile
   165 170 175
   Lys Glu Asn Met Asn Ile Leu Arg Pro Val Leu Lys Arg Glu Pro Thr
   180 185 190
   Asp Thr Asp Leu Tyr Leu Ala His Phe Phe Gly Pro Gly Ala Ala Arg
   195 200 205
   Arg Phe Leu Thr Thr Gly Gln Asn Glu Leu Ala Ala Thr His Phe Pro
   210 215 220
   Lys Glu Ala Gln Ala Asn Pro Ser Ile Phe Tyr Asn Lys Asp Gly Ser
   225 230 235 240
   Pro Lys Thr Ile Gln Glu Val Tyr Asn Leu Met Asp Gly Lys Val Ala
   245 250 255
   Ala His Arg Lys Lys Arg Lys Lys Arg Lys Lys Arg Lys
   260 265
<210> 149
   <211> 289
   <212> PRT
   <213> artificial
<220>
   <223> synthetic sequence
<400> 149
   Met Lys Val Leu Arg Lys Gly Asp Arg Gly Asp Glu Val Cys Gln Leu
   1               5                   10                  15
   Gln Thr Leu Leu Asn Leu Cys Gly Tyr Asp Val Gly Lys Pro Asp Gly
               20                  25                  30
   Ile Phe Gly Asn Asn Thr Phe Asn Gln Val Val Lys Phe Gln Lys Asp
           35                  40                  45
   Asn Cys Leu Asp Ser Asp Gly Ile Val Gly Lys Asn Thr Trp Ala Glu
       50                  55                  60
   Leu Phe Ser Lys Tyr Ser Pro Pro Ile Pro Tyr Lys Thr Ile Pro Met
   65                  70                  75                  80
   Pro Thr Ala Asn Lys Ser Arg Ala Ala Ala Thr Pro Val Met Asn Ala
                   85                  90                  95
   Val Glu Asn Ala Thr Gly Val Arg Ser Gln Leu Leu Leu Thr Phe Ala
               100                 105                 110
   Ser Ile Glu Ser Ala Phe Asp Tyr Glu Ile Lys Ala Lys Thr Ser Ser
           115                 120                 125
   Ala Thr Gly Trp Phe Gln Phe Leu Thr Gly Thr Trp Lys Thr Met Ile
       130                 135                 140
   Glu Asn Tyr Gly Met Lys Tyr Gly Val Leu Thr Asp Pro Thr Gly Ala
   145                 150                 155                 160
   Leu Arg Lys Asp Pro Arg Ile Ser Ala Leu Met Gly Ala Glu Leu Ile
                   165                 170                 175
   Lys Glu Asn Met Asn Ile Leu Arg Pro Val Leu Lys Arg Glu Pro Thr
               180                 185                 190
   Asp Thr Asp Leu Tyr Leu Ala His Phe Phe Gly Pro Gly Ala Ala Arg
           195                 200                 205
   Arg Phe Leu Thr Thr Gly Gln Asn Glu Leu Ala Ala Thr His Phe Pro
       210                 215                 220
   Lys Glu Ala Gln Ala Asn Pro Ser Ile Phe Tyr Asn Lys Asp Gly Ser
   225                 230                 235                 240
   Pro Lys Thr Ile Gln Glu Val Tyr Asn Leu Met Asp Gly Lys Val Ala
                   245                 250                 255
   Ala His Arg Lys Arg Gly Leu Arg Arg Leu Gly Arg Lys Ile Ala His
   260 265 270
   Gly Val Lys Lys Tyr Gly Pro Thr Val Leu Arg Ile Ile Arg Ile Ala
   275 280 285
   Gly
<210> 150
   <211> 320
   <212> PRT
   <213> artificial
<220>
   <223> synthetic sequence
<400> 150
   Met Asn Phe Arg Thr Lys Asn Gly Tyr Arg Asp Leu Gln Ala Leu Val
   1 5 10 15
   Lys Glu Leu Gly Leu Tyr Thr Gly Gln Ile Asp Gly Val Trp Gly Lys
   20 25 30
   Gly Thr Ser Ser Ser Thr Glu Thr Leu Leu Arg Gly Tyr Ala Glu Val
   35 40 45
   Val Gly Lys Asn Thr Gly Gly Ile Gly Leu Pro Thr Thr Ser Asp Ala
   50 55 60
   Ser Gly Tyr Asn Val Ile Thr Ala Leu Gln Arg Asn Leu Ala Phe Leu
   65 70 75 80
   Gly Leu Tyr Ser Leu Thr Val Asp Gly Ile Trp Gly Asn Gly Thr Leu
   85 90 95
   Ser Gly Asp Lys Ala Phe Glu Val Tyr Lys Glu Arg Tyr Arg Thr Pro
   100 105 110
   Thr Tyr Asp Ile Ala Trp Ser Gly Lys Val Ser Pro Ala Phe Thr Ala
   115 120 125
   Lys Val Lys Asp Trp Cys Gly Val His Val Pro Asn His Arg Ala Pro
   130 135 140
   His Trp Leu Met Ala Cys Met Ala Phe Glu Thr Gly Gln Thr Phe Ser
   145 150 155 160
   Pro Ser Ile Lys Asn Ala Ala Gly Ser Glu Ala Tyr Gly Leu Ile Gln
   165 170 175
   Phe Met Ser Pro Ala Ala Asn Asp Leu Asn Val Pro Leu Ser Val Ile
   180 185 190
   Arg Ser Met Asp Gln Leu Thr Gln Leu Asp Leu Val Phe Lys Tyr Phe
   195 200 205
   Glu Met Trp Met Lys Arg Gly Lys Arg Tyr Thr Gln Leu Glu Asp Phe
   210 215 220
   Tyr Leu Thr Ile Phe His Pro Ala Ser Val Gly Lys Lys Ala Asp Glu
   225 230 235 240
   Val Leu Phe Leu Gln Gly Ser Lys Ala Tyr Leu Gln Asn Lys Gly Phe
   245 250 255
   Asp Val Asp Lys Asp Gly Lys Ile Thr Leu Gly Glu Ile Ser Ser Thr
   260 265 270
   Leu Tyr Thr Thr Tyr Tyr Lys Gly Leu Leu Pro Glu Asn Arg His Val
   275 280 285
   Ile Ser Tyr Arg Gly Leu Arg Arg Leu Gly Arg Lys Ile Ala His Gly
   290 295 300
   Val Lys Lys Tyr Gly Pro Thr Val Leu Arg Ile Ile Arg Ile Ala Gly
   305 310 315 320
<210> 151
   <211> 300
   <212> PRT
   <213> artificial
<220>
   <223> synthetic sequence
<400> 151
   Met Asn Phe Arg Thr Lys Asn Gly Tyr Arg Asp Leu Gln Ala Leu Val
   1               5                   10                  15
   Lys Glu Leu Gly Leu Tyr Thr Gly Gln Ile Asp Gly Val Trp Gly Lys
               20                  25                  30
   Gly Thr Ser Ser Ser Thr Glu Thr Leu Leu Arg Gly Tyr Ala Glu Val
           35                  40                  45
   Val Gly Lys Asn Thr Gly Gly Ile Gly Leu Pro Thr Thr Ser Asp Ala
       50                  55                  60
   Ser Gly Tyr Asn Val Ile Thr Ala Leu Gln Arg Asn Leu Ala Phe Leu
   65                  70                  75                  80
   Gly Leu Tyr Ser Leu Thr Val Asp Gly Ile Trp Gly Asn Gly Thr Leu
                   85                  90                  95
   Ser Gly Asp Lys Ala Phe Glu Val Tyr Lys Glu Arg Tyr Arg Thr Pro
               100                 105                 110
   Thr Tyr Asp Ile Ala Trp Ser Gly Lys Val Ser Pro Ala Phe Thr Ala
           115                 120                 125
   Lys Val Lys Asp Trp Cys Gly Val His Val Pro Asn His Arg Ala Pro
       130                 135                 140
   His Trp Leu Met Ala Cys Met Ala Phe Glu Thr Gly Gln Thr Phe Ser
   145                 150                 155                 160
   Pro Ser Ile Lys Asn Ala Ala Gly Ser Glu Ala Tyr Gly Leu Ile Gln
                   165                 170                 175
   Phe Met Ser Pro Ala Ala Asn Asp Leu Asn Val Pro Leu Ser Val Ile
               180                 185                 190
   Arg Ser Met Asp Gln Leu Thr Gln Leu Asp Leu Val Phe Lys Tyr Phe
           195                 200                 205
   Glu Met Trp Met Lys Arg Gly Lys Arg Tyr Thr Gln Leu Glu Asp Phe
       210                 215                 220
   Tyr Leu Thr Ile Phe His Pro Ala Ser Val Gly Lys Lys Ala Asp Glu
   225                 230                 235                 240
   Val Leu Phe Leu Gln Gly Ser Lys Ala Tyr Leu Gln Asn Lys Gly Phe
                   245                 250                 255
   Asp Val Asp Lys Asp Gly Lys Ile Thr Leu Gly Glu Ile Ser Ser Thr
               260                 265                 270
   Leu Tyr Thr Thr Tyr Tyr Lys Gly Leu Leu Pro Glu Asn Arg His Val
           275                 280                 285
   Ile Ser Tyr Lys Arg Lys Lys Arg Lys Lys Arg Lys
       290                 295                 300
<210> 152
   <211> 206
   <212> PRT
   <213> artificial
<220>
   <223> synthetic sequence
<400> 152
   Met Asn Pro Ile Ile Asp Gly Ile Ile Ala Leu Glu Gly Gly Tyr Val
   1               5                   10                  15
   Phe Asn Pro Lys Asp Lys Gly Gly Ala Thr His Trp Gly Ile Thr Glu
               20                  25                  30
   Ala Thr Ala Arg Ala His Gly Tyr Ala Gly Asp Met Arg Asp Leu Thr
           35                  40                  45
   His Ala Glu Ala Tyr Ala Ile Leu Glu Glu Asp Tyr Trp Ile Lys Pro
       50                  55                  60
   Gly Phe Asp Val Ile Ser Thr Leu Ser Trp Pro Val Ser Phe Glu Leu
   65                  70                  75                  80
   Cys Asp Ala Ala Val Asn Ile Gly Ala Tyr His Pro Ser Ala Trp Leu
                   85                  90                  95
   Gln Arg Trp Leu Asn Val Phe Asn His Glu Gly Lys Arg Tyr Pro Asp
               100                 105                 110
   Ile His Val Asp Gly Asn Ile Gly Pro Arg Thr Leu Ala Ala Leu Glu
           115                 120                 125
   His Tyr Leu Ala Trp Arg Gly Gln Glu Gly Glu Ala Val Leu Val Lys
       130                 135                 140
   Ala Leu Asn Cys Ser Gln Gly Thr Tyr Tyr Leu Asn Val Ala Glu Lys
   145                 150                 155                 160
   Asn His Asn Asn Glu Gln Phe Ile Tyr Gly Trp Ile Lys Asn Arg Val
                   165                 170                 175
   Thr Arg Gly Leu Arg Arg Leu Gly Arg Lys Ile Ala His Gly Val Lys
               180                 185                 190
   Lys Tyr Gly Pro Thr Val Leu Arg Ile Ile Arg Ile Ala Gly
           195                 200                 205
<210> 153
   <211> 214
   <212> PRT
   <213> artificial
<220>
   <223> synthetic sequence
<400> 153
   Met Asn Pro Ile Ile Asp Gly Ile Ile Ala Leu Glu Gly Gly Tyr Val
   1               5                   10                  15
   Phe Asn Pro Lys Asp Lys Gly Gly Ala Thr His Trp Gly Ile Thr Glu
               20                  25                  30
   Ala Thr Ala Arg Ala His Gly Tyr Ala Gly Asp Met Arg Asp Leu Thr
           35                  40                  45
   His Ala Glu Ala Tyr Ala Ile Leu Glu Glu Asp Tyr Trp Ile Lys Pro
       50                  55                  60
   Gly Phe Asp Val Ile Ser Thr Leu Ser Trp Pro Val Ser Phe Glu Leu
   65                  70                  75                  80
   Cys Asp Ala Ala Val Asn Ile Gly Ala Tyr His Pro Ser Ala Trp Leu
                   85                  90                  95
   Gln Arg Trp Leu Asn Val Phe Asn His Glu Gly Lys Arg Tyr Pro Asp
               100                 105                 110
   Ile His Val Asp Gly Asn Ile Gly Pro Arg Thr Leu Ala Ala Leu Glu
           115                 120                 125
   His Tyr Leu Ala Trp Arg Gly Gln Glu Gly Glu Ala Val Leu Val Lys
       130                 135                 140
   Ala Leu Asn Cys Ser Gln Gly Thr Tyr Tyr Leu Asn Val Ala Glu Lys
   145                 150                 155                 160
   Asn His Asn Asn Glu Gln Phe Ile Tyr Gly Trp Ile Lys Asn Arg Val
                   165                 170                 175
   Thr Leu Leu Gly Asp Phe Phe Arg Lys Ser Lys Glu Lys Ile Gly Lys
               180                 185                 190
   Glu Phe Lys Arg Ile Val Gln Arg Ile Lys Asp Phe Leu Arg Asn Leu
           195                 200                 205
   Val Pro Arg Thr Glu Ser
       210
<210> 154
   <211> 203
   <212> PRT
   <213> artificial
<220>
   <223> synthetic sequence
<400> 154
   Met Asn Pro Ile Ile Asp Gly Ile Ile Ala Leu Glu Gly Gly Tyr Val
   1               5                   10                  15
   Phe Asn Pro Lys Asp Lys Gly Gly Ala Thr His Trp Gly Ile Thr Glu
               20                  25                  30
   Ala Thr Ala Arg Ala His Gly Tyr Ala Gly Asp Met Arg Asp Leu Thr
           35                  40                  45
   His Ala Glu Ala Tyr Ala Ile Leu Glu Glu Asp Tyr Trp Ile Lys Pro
       50                  55                  60
   Gly Phe Asp Val Ile Ser Thr Leu Ser Trp Pro Val Ser Phe Glu Leu
   65                  70                  75                  80
   Cys Asp Ala Ala Val Asn Ile Gly Ala Tyr His Pro Ser Ala Trp Leu
                   85                  90                  95
   Gln Arg Trp Leu Asn Val Phe Asn His Glu Gly Lys Arg Tyr Pro Asp
               100                 105                 110
   Ile His Val Asp Gly Asn Ile Gly Pro Arg Thr Leu Ala Ala Leu Glu
           115                 120                 125
   His Tyr Leu Ala Trp Arg Gly Gln Glu Gly Glu Ala Val Leu Val Lys
       130                 135                 140
   Ala Leu Asn Cys Ser Gln Gly Thr Tyr Tyr Leu Asn Val Ala Glu Lys
   145                 150                 155                 160
   Asn His Asn Asn Glu Gln Phe Ile Tyr Gly Trp Ile Lys Asn Arg Val
                   165                 170                 175
   Thr Gly Ile Gly Ala Val Leu Lys Val Leu Thr Thr Gly Leu Pro Ala
               180                 185                 190
   Leu Ile Ser Trp Ile Lys Arg Lys Arg Gln Gln
           195                 200
<210> 155
   <211> 284
   <212> PRT
   <213> artificial
<220>
   <223> synthetic sequence
<400> 155
   Met Ala His Glu His Ser Ala Gln Trp Leu Asn Asn Tyr Lys Lys Gly
   1               5                   10                  15
   Tyr Gly Tyr Gly Pro Tyr Pro Leu Gly Ile Asn Gly Gly Met His Tyr
               20                  25                  30
   Gly Val Asp Phe Phe Met Asn Ile Gly Thr Pro Val Lys Ala Ile Ser
           35                  40                  45
   Ser Gly Lys Ile Val Glu Ala Gly Trp Ser Asn Tyr Gly Gly Gly Asn
       50                  55                  60
   Gln Ile Gly Leu Ile Glu Asn Asp Gly Val His Arg Gln Trp Tyr Met
   65                  70                  75                  80
   His Leu Ser Lys Tyr Asn Val Lys Val Gly Asp Tyr Val Lys Ala Gly
                   85                  90                  95
   Gln Ile Ile Gly Trp Ser Gly Ser Thr Gly Tyr Ser Thr Ala Pro His
               100                 105                 110
   Leu His Phe Gln Arg Met Val Asn Ser Phe Ser Asn Ser Thr Ala Gln
           115                 120                 125
   Asp Pro Met Pro Phe Leu Lys Ser Ala Gly Tyr Gly Lys Ala Gly Gly
       130                 135                 140
   Thr Val Thr Pro Thr Pro Asn Thr Gly Trp Lys Thr Asn Lys Tyr Gly
   145                 150                 155                 160
   Thr Leu Tyr Lys Ser Glu Ser Ala Ser Phe Thr Pro Asn Thr Asp Ile
                   165                 170                 175
   Ile Thr Arg Thr Thr Gly Pro Phe Arg Ser Met Pro Gln Ser Gly Val
               180                 185                 190
   Leu Lys Ala Gly Gln Thr Ile His Tyr Asp Glu Val Met Lys Gln Asp
           195                 200                 205
   Gly His Val Trp Val Gly Tyr Thr Gly Asn Ser Gly Gln Arg Ile Tyr
   210 215 220
   Leu Pro Val Arg Thr Trp Asn Lys Ser Thr Asn Thr Leu Gly Val Leu
   225 230 235 240
   Trp Gly Thr Ile Lys Gly Trp Leu Lys Lys Ile Gly Lys Lys Ile Glu
   245 250 255
   Arg Val Gly Gln His Thr Arg Asp Ala Thr Ile Gln Gly Leu Gly Ile
   260 265 270
   Ala Gln Gln Ala Ala Asn Val Ala Ala Thr Ala Arg
   275 280
<210> 156
   <211> 274
   <212> PRT
   <213> artificial
<220>
   <223> synthetic sequence
<400> 156
   Met Ala His Glu His Ser Ala Gln Trp Leu Asn Asn Tyr Lys Lys Gly
   1               5                   10                  15
   Tyr Gly Tyr Gly Pro Tyr Pro Leu Gly Ile Asn Gly Gly Met His Tyr
               20                  25                  30
   Gly Val Asp Phe Phe Met Asn Ile Gly Thr Pro Val Lys Ala Ile Ser
           35                  40                  45
   Ser Gly Lys Ile Val Glu Ala Gly Trp Ser Asn Tyr Gly Gly Gly Asn
       50                  55                  60
   Gln Ile Gly Leu Ile Glu Asn Asp Gly Val His Arg Gln Trp Tyr Met
   65                  70                  75                  80
   His Leu Ser Lys Tyr Asn Val Lys Val Gly Asp Tyr Val Lys Ala Gly
                   85                  90                  95
   Gln Ile Ile Gly Trp Ser Gly Ser Thr Gly Tyr Ser Thr Ala Pro His
               100                 105                 110
   Leu His Phe Gln Arg Met Val Asn Ser Phe Ser Asn Ser Thr Ala Gln
           115                 120                 125
   Asp Pro Met Pro Phe Leu Lys Ser Ala Gly Tyr Gly Lys Ala Gly Gly
   130 135 140
   Thr Val Thr Pro Thr Pro Asn Thr Gly Trp Lys Thr Asn Lys Tyr Gly
   145 150 155 160
   Thr Leu Tyr Lys Ser Glu Ser Ala Ser Phe Thr Pro Asn Thr Asp Ile
   165 170 175
   Ile Thr Arg Thr Thr Gly Pro Phe Arg Ser Met Pro Gln Ser Gly Val
   180 185 190
   Leu Lys Ala Gly Gln Thr Ile His Tyr Asp Glu Val Met Lys Gln Asp
   195 200 205
   Gly His Val Trp Val Gly Tyr Thr Gly Asn Ser Gly Gln Arg Ile Tyr
   210 215 220
   Leu Pro Val Arg Thr Trp Asn Lys Ser Thr Asn Thr Leu Gly Val Leu
   225 230 235 240
   Trp Gly Thr Ile Lys Arg Gly Leu Arg Arg Leu Gly Arg Lys Ile Ala
   245 250 255
   His Gly Val Lys Lys Tyr Gly Pro Thr Val Leu Arg Ile Ile Arg Ile
   260 265 270
   Ala Gly
<210> 157
   <211> 284
   <212> PRT
   <213> artificial
<220>
   <223> synthetic sequence
<400> 157
   Met Gly Trp Leu Lys Lys Ile Gly Lys Lys Ile Glu Arg Val Gly Gln
   1               5                   10                  15
   His Thr Arg Asp Ala Thr Ile Gln Gly Leu Gly Ile Ala Gln Gln Ala
               20                  25                  30
   Ala Asn Val Ala Ala Thr Ala Arg Ala His Glu His Ser Ala Gln Trp
           35                  40                  45
   Leu Asn Asn Tyr Lys Lys Gly Tyr Gly Tyr Gly Pro Tyr Pro Leu Gly
       50                  55                  60
   Ile Asn Gly Gly Met His Tyr Gly Val Asp Phe Phe Met Asn Ile Gly
   65                  70                  75                  80
   Thr Pro Val Lys Ala Ile Ser Ser Gly Lys Ile Val Glu Ala Gly Trp
                   85                  90                  95
   Ser Asn Tyr Gly Gly Gly Asn Gln Ile Gly Leu Ile Glu Asn Asp Gly
               100                 105                 110
   Val His Arg Gln Trp Tyr Met His Leu Ser Lys Tyr Asn Val Lys Val
           115                 120                 125
   Gly Asp Tyr Val Lys Ala Gly Gln Ile Ile Gly Trp Ser Gly Ser Thr
       130                 135                 140
   Gly Tyr Ser Thr Ala Pro His Leu His Phe Gln Arg Met Val Asn Ser
   145                 150                 155                 160
   Phe Ser Asn Ser Thr Ala Gln Asp Pro Met Pro Phe Leu Lys Ser Ala
                   165                 170                 175
   Gly Tyr Gly Lys Ala Gly Gly Thr Val Thr Pro Thr Pro Asn Thr Gly
               180                 185                 190
   Trp Lys Thr Asn Lys Tyr Gly Thr Leu Tyr Lys Ser Glu Ser Ala Ser
           195                 200                 205
   Phe Thr Pro Asn Thr Asp Ile Ile Thr Arg Thr Thr Gly Pro Phe Arg
       210                 215                 220
   Ser Met Pro Gln Ser Gly Val Leu Lys Ala Gly Gln Thr Ile His Tyr
   225                 230                 235                 240
   Asp Glu Val Met Lys Gln Asp Gly His Val Trp Val Gly Tyr Thr Gly
                   245                 250                 255
   Asn Ser Gly Gln Arg Ile Tyr Leu Pro Val Arg Thr Trp Asn Lys Ser
               260                 265                 270
   Thr Asn Thr Leu Gly Val Leu Trp Gly Thr Ile Lys
           275                 280
<210> 158
   <211> 274
   <212> PRT
   <213> artificial
<220>
   <223> synthetic sequence
<400> 158
   Met Arg Gly Leu Arg Arg Leu Gly Arg Lys Ile Ala His Gly Val Lys
   1               5                   10                  15
   Lys Tyr Gly Pro Thr Val Leu Arg Ile Ile Arg Ile Ala Gly Ala His
               20                  25                  30
   Glu His Ser Ala Gln Trp Leu Asn Asn Tyr Lys Lys Gly Tyr Gly Tyr
           35                  40                  45
   Gly Pro Tyr Pro Leu Gly Ile Asn Gly Gly Met His Tyr Gly Val Asp
       50                  55                  60
   Phe Phe Met Asn Ile Gly Thr Pro Val Lys Ala Ile Ser Ser Gly Lys
   65                  70                  75                  80
   Ile Val Glu Ala Gly Trp Ser Asn Tyr Gly Gly Gly Asn Gln Ile Gly
                   85                  90                  95
   Leu Ile Glu Asn Asp Gly Val His Arg Gln Trp Tyr Met His Leu Ser
               100                 105                 110
   Lys Tyr Asn Val Lys Val Gly Asp Tyr Val Lys Ala Gly Gln Ile Ile
           115                 120                 125
   Gly Trp Ser Gly Ser Thr Gly Tyr Ser Thr Ala Pro His Leu His Phe
       130                 135                 140
   Gln Arg Met Val Asn Ser Phe Ser Asn Ser Thr Ala Gln Asp Pro Met
   145                 150                 155                 160
   Pro Phe Leu Lys Ser Ala Gly Tyr Gly Lys Ala Gly Gly Thr Val Thr
                   165                 170                 175
   Pro Thr Pro Asn Thr Gly Trp Lys Thr Asn Lys Tyr Gly Thr Leu Tyr
               180                 185                 190
   Lys Ser Glu Ser Ala Ser Phe Thr Pro Asn Thr Asp Ile Ile Thr Arg
           195                 200                 205
   Thr Thr Gly Pro Phe Arg Ser Met Pro Gln Ser Gly Val Leu Lys Ala
       210                 215                 220
   Gly Gln Thr Ile His Tyr Asp Glu Val Met Lys Gln Asp Gly His Val
   225                 230                 235                 240
   Trp Val Gly Tyr Thr Gly Asn Ser Gly Gln Arg Ile Tyr Leu Pro Val
   245 250 255
   Arg Thr Trp Asn Lys Ser Thr Asn Thr Leu Gly Val Leu Trp Gly Thr
   260 265 270
   Ile Lys
<210> 159
   <211> 352
   <212> PRT
   <213> artificial
<220>
   <223> synthetic sequence
<400> 159
   Met Val Lys Lys Asn Asp Leu Phe Val Asp Val Ser Ser His Asn Gly
   1 5 10 15
   Tyr Asp Ile Thr Gly Ile Leu Glu Gln Met Gly Thr Thr Asn Thr Ile
   20 25 30
   Ile Lys Ile Ser Glu Ser Thr Thr Tyr Leu Asn Pro Cys Leu Ser Ala
   35 40 45
   Gln Val Glu Gln Ser Asn Pro Ile Gly Phe Tyr His Phe Ala Arg Phe
   50 55 60
   Gly Gly Asp Val Ala Glu Ala Glu Arg Glu Ala Gln Phe Phe Leu Asp
   65 70 75 80
   Asn Val Pro Met Gln Val Lys Tyr Leu Val Leu Asp Tyr Glu Asp Asp
   85 90 95
   Pro Ser Gly Asp Ala Gln Ala Asn Thr Asn Ala Cys Leu Arg Phe Met
   100 105 110
   Gln Met Ile Ala Asp Ala Gly Tyr Lys Pro Ile Tyr Tyr Ser Tyr Lys
   115 120 125
   Pro Phe Thr His Asp Asn Val Asp Tyr Gln Gln Ile Leu Ala Gln Phe
   130 135 140
   Pro Asn Ser Leu Trp Ile Ala Gly Tyr Gly Leu Asn Asp Gly Thr Ala
   145 150 155 160
   Asn Phe Glu Tyr Phe Pro Ser Met Asp Gly Ile Arg Trp Trp Gln Tyr
                   165                 170                 175
   Ser Ser Asn Pro Phe Asp Lys Asn Ile Val Leu Leu Asp Asp Glu Glu
               180                 185                 190
   Asp Asp Lys Pro Lys Thr Ala Gly Thr Trp Lys Gln Asp Ser Lys Gly
           195                 200                 205
   Trp Trp Phe Arg Arg Asn Asn Gly Ser Phe Pro Tyr Asn Lys Trp Glu
       210                 215                 220
   Lys Ile Gly Gly Val Trp Tyr Tyr Phe Asp Ser Lys Gly Tyr Cys Leu
   225                 230                 235                 240
   Thr Ser Glu Trp Leu Lys Asp Asn Glu Lys Trp Tyr Tyr Leu Lys Asp
                   245                 250                 255
   Asn Gly Ala Met Ala Thr Gly Trp Val Leu Val Gly Ser Glu Trp Tyr
               260                 265                 270
   Tyr Met Asp Asp Ser Gly Ala Met Val Thr Gly Trp Val Lys Tyr Lys
           275                 280                 285
   Asn Asn Trp Tyr Tyr Met Thr Asn Glu Arg Gly Asn Met Val Ser Asn
       290                 295                 300
   Glu Phe Ile Lys Ser Gly Lys Gly Trp Tyr Phe Met Asn Thr Asn Gly
   305                 310                 315                 320
   Glu Leu Ala Asp Asn Pro Ser Phe Thr Lys Glu Pro Asp Gly Leu Ile
                   325                 330                 335
   Thr Val Ala Ile Leu Pro Trp Lys Trp Pro Trp Trp Pro Trp Arg Arg
               340                 345                 350
<210> 160
   <211> 363
   <212> PRT
   <213> artificial
<220>
   <223> synthetic sequence
<400> 160
   Met Val Lys Lys Asn Asp Leu Phe Val Asp Val Ser Ser His Asn Gly
   1               5                   10                  15
   Tyr Asp Ile Thr Gly Ile Leu Glu Gln Met Gly Thr Thr Asn Thr Ile
               20                  25                  30
   Ile Lys Ile Ser Glu Ser Thr Thr Tyr Leu Asn Pro Cys Leu Ser Ala
           35                  40                  45
   Gln Val Glu Gln Ser Asn Pro Ile Gly Phe Tyr His Phe Ala Arg Phe
       50                  55                  60
   Gly Gly Asp Val Ala Glu Ala Glu Arg Glu Ala Gln Phe Phe Leu Asp
   65                  70                  75                  80
   Asn Val Pro Met Gln Val Lys Tyr Leu Val Leu Asp Tyr Glu Asp Asp
                   85                  90                  95
   Pro Ser Gly Asp Ala Gln Ala Asn Thr Asn Ala Cys Leu Arg Phe Met
               100                 105                 110
   Gln Met Ile Ala Asp Ala Gly Tyr Lys Pro Ile Tyr Tyr Ser Tyr Lys
           115                 120                 125
   Pro Phe Thr His Asp Asn Val Asp Tyr Gln Gln Ile Leu Ala Gln Phe
       130                 135                 140
   Pro Asn Ser Leu Trp Ile Ala Gly Tyr Gly Leu Asn Asp Gly Thr Ala
   145                 150                 155                 160
   Asn Phe Glu Tyr Phe Pro Ser Met Asp Gly Ile Arg Trp Trp Gln Tyr
                   165                 170                 175
   Ser Ser Asn Pro Phe Asp Lys Asn Ile Val Leu Leu Asp Asp Glu Glu
               180                 185                 190
   Asp Asp Lys Pro Lys Thr Ala Gly Thr Trp Lys Gln Asp Ser Lys Gly
           195                 200                 205
   Trp Trp Phe Arg Arg Asn Asn Gly Ser Phe Pro Tyr Asn Lys Trp Glu
       210                 215                 220
   Lys Ile Gly Gly Val Trp Tyr Tyr Phe Asp Ser Lys Gly Tyr Cys Leu
   225                 230                 235                 240
   Thr Ser Glu Trp Leu Lys Asp Asn Glu Lys Trp Tyr Tyr Leu Lys Asp
                   245                 250                 255
   Asn Gly Ala Met Ala Thr Gly Trp Val Leu Val Gly Ser Glu Trp Tyr
               260                 265                 270
   Tyr Met Asp Asp Ser Gly Ala Met Val Thr Gly Trp Val Lys Tyr Lys
   275 280 285
   Asn Asn Trp Tyr Tyr Met Thr Asn Glu Arg Gly Asn Met Val Ser Asn
   290 295 300
   Glu Phe Ile Lys Ser Gly Lys Gly Trp Tyr Phe Met Asn Thr Asn Gly
   305 310 315 320
   Glu Leu Ala Asp Asn Pro Ser Phe Thr Lys Glu Pro Asp Gly Leu Ile
   325 330 335
   Thr Val Ala Gly Leu Asn Thr Leu Lys Lys Val Phe Gln Gly Leu His
   340 345 350
   Glu Ala Ile Lys Leu Ile Asn Asn His Val Gln
   355 360
<210> 161
   <211> 364
   <212> PRT
   <213> artificial
<220>
   <223> synthetic sequence
<400> 161
   Met Val Lys Tyr Thr Val Glu Asn Lys Ile Ile Ala Gly Leu Pro Lys
   1 5 10 15
   Gly Lys Leu Lys Gly Ala Asn Phe Val Ile Ala His Glu Thr Ala Asn
   20 25 30
   Ser Lys Ser Thr Ile Asp Asn Glu Val Ser Tyr Met Thr Arg Asn Trp
   35 40 45
   Lys Asn Ala Phe Val Thr His Phe Val Gly Gly Gly Gly Arg Val Val
   50 55 60
   Gln Val Ala Asn Val Asn Tyr Val Ser Trp Gly Ala Gly Gln Tyr Ala
   65 70 75 80
   Asn Ser Tyr Ser Tyr Ala Gln Val Glu Leu Cys Arg Thr Ser Asn Ala
   85 90 95
   Thr Thr Phe Lys Lys Asp Tyr Glu Val Tyr Cys Gln Leu Leu Val Asp
   100 105 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys Gly Ile Gly Lys Phe Leu His Ser Ala Lys Lys
               340                 345                 350
   Phe Gly Lys Ala Phe Val Gly Glu Ile Met Asn Ser
           355                 360
<210> 162
   <211> 362
   <212> PRT
   <213> artificial
<220>
   <223> synthetic sequence
<400> 162
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
           35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Arg Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys Thr Arg Ser Ser Arg Ala Gly Leu Gln Phe Pro
               340                 345                 350
   Val Gly Arg Val His Arg Leu Leu Arg Lys
           355                 360
<210> 163
   <211> 513
   <212> PRT
   <213> artificial
<220>
   <223> synthetic sequence
<400> 163
   Met Lys Arg Lys Lys Arg Lys Lys Arg Lys Lys Arg Lys Lys Arg Lys
   1               5                   10                  15
   Lys Arg Lys Ala Lys Thr Gln Ala Glu Ile Asn Lys Arg Leu Asp Ala
               20                  25                  30
   Tyr Ala Lys Gly Thr Val Asp Ser Pro Tyr Arg Val Lys Lys Ala Thr
           35                  40                  45
   Ser Tyr Asp Pro Ser Phe Gly Val Met Glu Ala Gly Ala Ile Asp Ala
       50                  55                  60
   Asp Gly Tyr Tyr His Ala Gln Cys Gln Asp Leu Ile Thr Asp Tyr Val
   65                  70                  75                  80
   Leu Trp Leu Thr Asp Asn Lys Val Arg Thr Trp Gly Asn Ala Lys Asp
                   85                  90                  95
   Gln Ile Lys Gln Ser Tyr Gly Thr Gly Phe Lys Ile His Glu Asn Lys
               100                 105                 110
   Pro Ser Thr Val Pro Lys Lys Gly Trp Ile Ala Val Phe Thr Ser Gly
           115                 120                 125
   Ser Tyr Glu Gln Trp Gly His Ile Gly Ile Val Tyr Asp Gly Gly Asn
       130                 135                 140
   Thr Ser Thr Phe Thr Ile Leu Glu Gln Asn Trp Asn Gly Tyr Ala Asn
   145                 150                 155                 160
   Lys Lys Pro Thr Lys Arg Val Asp Asn Tyr Tyr Gly Leu Thr His Phe
                   165                 170                 175
   Ile Glu Ile Pro Val Lys Ala Gly Thr Thr Val Lys Lys Glu Thr Ala
               180                 185                 190
   Lys Lys Ser Ala Ser Lys Thr Pro Ala Pro Lys Lys Lys Ala Thr Leu
           195                 200                 205
   Lys Val Ser Lys Asn His Ile Asn Tyr Thr Met Asp Lys Arg Gly Lys
       210                 215                 220
   Lys Pro Glu Gly Met Val Ile His Asn Asp Ala Gly Arg Ser Ser Gly
   225                 230                 235                 240
   Gln Gln Tyr Glu Asn Ser Leu Ala Asn Ala Gly Tyr Ala Arg Tyr Ala
                   245                 250                 255
   Asn Gly Ile Ala His Tyr Tyr Gly Ser Glu Gly Tyr Val Trp Glu Ala
               260                 265                 270
   Ile Asp Ala Lys Asn Gln Ile Ala Trp His Thr Gly Asp Gly Thr Gly
           275                 280                 285
   Ala Asn Ser Gly Asn Phe Arg Phe Ala Gly Ile Glu Val Cys Gln Ser
       290                 295                 300
   Met Ser Ala Ser Asp Ala Gln Phe Leu Lys Asn Glu Gln Ala Val Phe
   305                 310                 315                 320
   Gln Phe Thr Ala Glu Lys Phe Lys Glu Trp Gly Leu Thr Pro Asn Arg
                   325                 330                 335
   Lys Thr Val Arg Leu His Met Glu Phe Val Pro Thr Ala Cys Pro His
               340                 345                 350
   Arg Ser Met Val Leu His Thr Gly Phe Asn Pro Val Thr Gln Gly Arg
           355                 360                 365
   Pro Ser Gln Ala Ile Met Asn Lys Leu Lys Asp Tyr Phe Ile Lys Gln
       370                 375                 380
   Ile Lys Asn Tyr Met Asp Lys Gly Thr Ser Ser Ser Thr Val Val Lys
   385                 390                 395                 400
   Asp Gly Lys Thr Ser Ser Ala Ser Thr Pro Ala Thr Arg Pro Val Thr
                   405                 410                 415
   Gly Ser Trp Lys Lys Asn Gln Tyr Gly Thr Trp Tyr Lys Pro Glu Asn
               420                 425                 430
   Ala Thr Phe Val Asn Gly Asn Gln Pro Ile Val Thr Arg Ile Gly Ser
           435                 440                 445
   Pro Phe Leu Asn Ala Pro Val Gly Gly Asn Leu Pro Ala Gly Ala Thr
       450                 455                 460
   Ile Val Tyr Asp Glu Val Cys Ile Gln Ala Gly His Ile Trp Ile Gly
   465                 470                 475                 480
   Tyr Asn Ala Tyr Asn Gly Asn Arg Val Tyr Cys Pro Val Arg Thr Cys
                   485                 490                 495
   Gln Gly Val Pro Pro Asn Gln Ile Pro Gly Val Ala Trp Gly Val Phe
               500                 505                 510
   Lys
<210> 164
   <211> 531
   <212> PRT
   <213> artificial
<220>
   <223> synthetic sequence
<400> 164
   Met Gly Gly Leu Lys Lys Leu Gly Lys Lys Leu Glu Gly Ala Gly Lys
   1               5                   10                  15
   Arg Val Phe Asn Ala Ala Glu Lys Ala Leu Pro Val Val Ala Gly Ala
               20                  25                  30
   Lys Ala Leu Arg Lys Ala Lys Thr Gln Ala Glu Ile Asn Lys Arg Leu
           35                  40                  45
   Asp Ala Tyr Ala Lys Gly Thr Val Asp Ser Pro Tyr Arg Val Lys Lys
       50                  55                  60
   Ala Thr Ser Tyr Asp Pro Ser Phe Gly Val Met Glu Ala Gly Ala Ile
   65                  70                  75                  80
   Asp Ala Asp Gly Tyr Tyr His Ala Gln Cys Gln Asp Leu Ile Thr Asp
                   85                  90                  95
   Tyr Val Leu Trp Leu Thr Asp Asn Lys Val Arg Thr Trp Gly Asn Ala
               100                 105                 110
   Lys Asp Gln Ile Lys Gln Ser Tyr Gly Thr Gly Phe Lys Ile His Glu
           115                 120                 125
   Asn Lys Pro Ser Thr Val Pro Lys Lys Gly Trp Ile Ala Val Phe Thr
       130                 135                 140
   Ser Gly Ser Tyr Glu Gln Trp Gly His Ile Gly Ile Val Tyr Asp Gly
   145                 150                 155                 160
   Gly Asn Thr Ser Thr Phe Thr Ile Leu Glu Gln Asn Trp Asn Gly Tyr
                   165                 170                 175
   Ala Asn Lys Lys Pro Thr Lys Arg Val Asp Asn Tyr Tyr Gly Leu Thr
               180                 185                 190
   His Phe Ile Glu Ile Pro Val Lys Ala Gly Thr Thr Val Lys Lys Glu
           195                 200                 205
   Thr Ala Lys Lys Ser Ala Ser Lys Thr Pro Ala Pro Lys Lys Lys Ala
       210                 215                 220
   Thr Leu Lys Val Ser Lys Asn His Ile Asn Tyr Thr Met Asp Lys Arg
   225                 230                 235                 240
   Gly Lys Lys Pro Glu Gly Met Val Ile His Asn Asp Ala Gly Arg Ser
                   245                 250                 255
   Ser Gly Gln Gln Tyr Glu Asn Ser Leu Ala Asn Ala Gly Tyr Ala Arg
               260                 265                 270
   Tyr Ala Asn Gly Ile Ala His Tyr Tyr Gly Ser Glu Gly Tyr Val Trp
           275                 280                 285
   Glu Ala Ile Asp Ala Lys Asn Gln Ile Ala Trp His Thr Gly Asp Gly
       290                 295                 300
   Thr Gly Ala Asn Ser Gly Asn Phe Arg Phe Ala Gly Ile Glu Val Cys
   305                 310                 315                 320
   Gln Ser Met Ser Ala Ser Asp Ala Gln Phe Leu Lys Asn Glu Gln Ala
                   325                 330                 335
   Val Phe Gln Phe Thr Ala Glu Lys Phe Lys Glu Trp Gly Leu Thr Pro
               340                 345                 350
   Asn Arg Lys Thr Val Arg Leu His Met Glu Phe Val Pro Thr Ala Cys
           355                 360                 365
   Pro His Arg Ser Met Val Leu His Thr Gly Phe Asn Pro Val Thr Gln
       370                 375                 380
   Gly Arg Pro Ser Gln Ala Ile Met Asn Lys Leu Lys Asp Tyr Phe Ile
   385                 390                 395                 400
   Lys Gln Ile Lys Asn Tyr Met Asp Lys Gly Thr Ser Ser Ser Thr Val
                   405                 410                 415
   Val Lys Asp Gly Lys Thr Ser Ser Ala Ser Thr Pro Ala Thr Arg Pro
               420                 425                 430
   Val Thr Gly Ser Trp Lys Lys Asn Gln Tyr Gly Thr Trp Tyr Lys Pro
           435                 440                 445
   Glu Asn Ala Thr Phe Val Asn Gly Asn Gln Pro Ile Val Thr Arg Ile
       450                 455                 460
   Gly Ser Pro Phe Leu Asn Ala Pro Val Gly Gly Asn Leu Pro Ala Gly
   465                 470                 475                 480
   Ala Thr Ile Val Tyr Asp Glu Val Cys Ile Gln Ala Gly His Ile Trp
                   485                 490                 495
   Ile Gly Tyr Asn Ala Tyr Asn Gly Asn Arg Val Tyr Cys Pro Val Arg
               500                 505                 510
   Thr Cys Gln Gly Val Pro Pro Asn Gln Ile Pro Gly Val Ala Trp Gly
           515                 520                 525
   Val Phe Lys
       530

## Description

The present invention relates to a fusion protein composed of an enzyme having the activity of degrading the cell wall of Gram-negative bacteria and/or Gram-positive bacteria and at least two peptide stretches fused to the enzyme at the N- or C-terminus, wherein the peptide stretches have a length of 5 to 50 amino acid residues and wherein the peptide stretches are a synthetic polycationic peptide and an naturally occurring antimicrobial peptide, like sushi peptide and defensin, and wherein the enzyme exhibits an amino acid sequence selected from the group consisting of SEQ ID NO: 1 to 18, wherein at least 70% of the amino acid residues of the polycationic peptide are positively charged amino acid residues, in particular lysine and/or arginine residues, and wherein the antimicrobial peptide exhibits an amino acid sequence selected from the group consisting of SEQ ID NOs: 44 to 63 and 78 to 98. Moreover, the present invention relates to nucleic acid molecules encoding said fusion protein, vectors comprising said nucleic acid molecules and host cells comprising either said nucleic acid molecules or said vectors. In addition, the present invention relates to said fusion protein for use as a medicament, diagnostic means, cosmetic substance, a disinfectant or a food additive. The present invention also relates to the removal, reduction and/or prevention of Gram-positive and/or Gram-negative bacterial contamination of foodstuff, of food processing equipment, of food processing plants, of surfaces coming into contact with foodstuff, of medical devices, of surfaces in hospitals and surgeries.

Gram-negative bacteria possess an outer membrane, with its characteristic asymmetric bilayer as a hallmark. The outer membrane bilayer consists of an inner monolayer containing phospholipids (primarily phosphatidyl ethanolamine) and an outer monolayer that is mainly composed of a single glycolipid, lipopolysaccharide (LPS). There is an immense diversity of LPS structures in the bacterial kingdom and the LPS structure may be modified in response to prevailing environmental conditions. The stability of the LPS layer and interaction between different LPS molecules is mainly achieved by the electrostatic interaction of divalent ions (Mg²⁺, Ca²⁺) with the anionic components of the LPS molecule (phosphate groups in the lipid A and the inner core and carboxyl groups of KDO). Furthermore, the dense and ordered packing of the hydrophobic moiety of lipid A, favored by the absence of unsaturated fatty acids, forms a rigid structure with high viscosity. This makes it less permeable for lipophilic molecules and confers additional stability to the outer membrane (OM).

In contrast to Gram-negative bacteria, Gram-positive bacteria do not possess an outer membrane. The cytoplasmic membrane is surrounded by an up to 25 nm thick layer of peptidoglycan (which is only up to 5 nm for Gram-negative bacteria) which forms the cell wall. Main purpose of the cell wall of Gram-positives is to maintain bacterial shape and to counteract the internal bacterial cell pressure. Peptidoglycan or murein is a polymer consisting of sugars and amino acids. The sugar component consists of alternating residues of β-(1,4) linked N-acetylglucosamine and N-acetylmuramic acid residues compose the sugar components. A peptide chain of three to five amino acids is attached to the N-acetylmuramic acid. The peptide chain can be cross-linked to the peptide chain of another strand forming a 3D mesh-like layer. The peptide chain may contain D- and L- amino acid residues and the composition may vary for different bacteria.

Various types of agents having bactericidal or bacteriostatic activity are known, e.g. antibiotics, endolysins, antimicrobial peptides and defensins. Increasingly microbial resistance to antibiotics, however, is creating difficulties in treating more and more infections caused by bacteria. Particular difficulties arise with infections caused by Gram-negative bacteria like *Pseudomonas aeruginosa* and Enterobacteriaceae and with infections caused by Gram-positive bacteria like *Staphylococcus aureus, Enterococci, Streptococci, Listeria monocytogenes* and *Clostridium difficile,* especially with e.g. Methicillin-resistant *Staphylococcus aureus* and Vancomycin-resistant *Enterococci.*

Endolysins are peptidoglycan hydrolases encoded by bacteriophages (or bacterial viruses). They are synthesized during late gene expression in the lytic cycle of phage multiplication and mediate the release of progeny virions from infected bacterial cells through degradation of the bacterial peptidoglycan. They are either ß(1,4)-glycosylases, transglycosylases, amidases or endopeptidases. Antimicrobial application of endolysins was already suggested in 1991 by Gasson (GB2243611). Although the killing capacity of endolysins has been known for a long time, the use of these enzymes as antibacterials was ignored due to the success and dominance of antibiotics. Only after the appearance of multiple antibiotic resistant bacteria this concept of combating human pathogens with endolysins received interest. A compelling need to develop totally new classes of antibacterial agents emerged and endolysins used as 'enzybiotics' - a hybrid term of 'enzymes' and 'antibiotics' - seem to meet this need. In 2001, Fischetti and coworkers demonstrated for the first time the therapeutic potential of bacteriophage Cl endolysin towards group A streptococci (Nelson et al., 2001). Since then many publications have established endolysins as an attractive and complementary alternative to control bacterial infections, particularly by Gram-positive bacteria. Subsequently different endolysins against other Gram-positive pathogens such as *Streptococcus pneumoniae* (Loeffler et al., 2001), *Bacillus anthracis* (Schuch et al., 2002), *S. agalactiae* (Cheng et al., 2005) and *Staphylococcus aureus* (Rashel et al, 2007) have proven their efficacy as enzybiotics. Nowadays, the most important challenge of endolysin therapy lies in the insensitivity of Gram-negative bacteria towards the exogenous action of endolysins, since the outer membrane shields the access of endolysins from the peptidoglycan. This currently prevents the expansion of the range of effective endolysins to important Gram-negative pathogens. However, it is also known that endolysins can, under some conditions (e.g. high ionic strength), create stable protoplast, where the internal bacterial cell pressure is not sufficient to lead to a cell burst. Under these conditions the bacterial cell wall can regenerate and the bacteria will survive. Up to now endolysins are thus not qualified to substitute antibiotics.

Antimicrobial peptides (AMPs) represent a wide range of short, cationic or amphiphatic, gene encoded peptide antimicrobials that can be found in virtually every organism. Different AMPs display different properties, and many peptides in this class are being intensively researched not only as antimicrobials, but also as templates for cell penetrating peptides. Despite sharing a few common features (e.g. cationicity, amphiphaticity and short size), AMP sequences vary greatly, and at least four structural groups (α-helical, β-sheet, extended and looped) have been proposed to accommodate the diversity of the observed AMP conformations. Likewise, several modes of action as antimicrobials have been proposed, and it was shown e.g. that the primary target of many of these peptides is the cell membrane whereas for other peptides the primary target is cytoplasmic invasion and disruption of core metabolic functions. AMPs may become concentrated enough to exhibit cooperative activity despite the absence of specific target binding; for example, by forming a pore in the membrane, as is the case for most AMPs. However, this phenomenon has only been observed in model phospholipid bilayers, and in some cases, AMP concentrations in the membrane that were as high as one peptide molecule per six phospholipid molecules were required for these events to occur. These concentrations are close to, if not at, full membrane saturation. As the minimum inhibitory concentration (MIC) for AMPs are typically in the low micromolar range, scepticism has understandably arisen regarding the relevance of these thresholds and their importance *in vivo* (Melo et al., Nature reviews, Microbiology, 2009, 245).

Defensins are a large family of small, cationic or amphiphatic, cysteine- and arginine-rich antimicrobial peptides, found in both vertebrates and invertebrates. Defensins are divided into five groups according to the spacing pattern of cysteines: plant, invertebrate, α-, β-, and θ-defensins. The latter three are mostly found in mammals. α -defensins are proteins found in neutrophils and intestinal epithelia. β-defensins are the most widely distributed and are secreted by leukocytes and epithelial cells of many kinds. θ-defensins have been rarely found so far e.g. in leukocytes of rhesus macaques. Defensins are active against bacteria, fungi and many enveloped and nonenveloped viruses. However, the concentrations needed for efficient killing of bacteria are mostly high, i.e. in the µ-molar range. Activity of many peptides may be limited in presence of physiological salt conditions, divalent cations and serum. Depending on the content of hydrophobic amino acid residues defensins also show haemolytic activity. WO 2010/023207 discloses endolysin variants comprising an endolysin to which a peptide stretch with membrane or LPS disrupting activity is fused, such as cationic or polycationic peptides.

Van der Linden et al. (Biotechnology Letters, 2009, 31(8): 1265-1267) reports synergistic effects of ovine-derived cathelicidins and other antimicrobials against *Escherichia coli* and *Staphylococcus aureus* bacteria. Egg white lysozyme showed synergy against *E. coli* with SMAP29, polymyxin B and lactoferrin. Some other combinations were either additive or non-synergistic.

The molecular evolution of lytic enzymes of *Streptococcus pneumoniae* and its bacteriophages is addressed in Garcia et al. (Proc. Natl. Acad. Sci. USA, , 1988, Vol. 85. 914-918). The authors also report the nucleotide sequence of the cp-gene of the pneumococcal bacteriophage Cp-1.

WO 2010/011960 discloses an antimicrobial endolysin - Lysostaphin triple fusion protein, comprising (1) an endolysin CHAP endopeptidase domain, (2) an endolysin amidase domain, and (3) a Lysostaphin glycyl-glycine endopeptidase domain. The domains are derived from two proteins that show antimicrobial synergy when used in combination. The protein has specificity and exolytic activity for the peptidoglycan cell wall of untreated, live *Staphylococcus aureus.*

Thus, there is a need for new antimicrobial agents.

This object is solved by the subject matter defined in the claims.

The following figures serve to illustrate the invention.

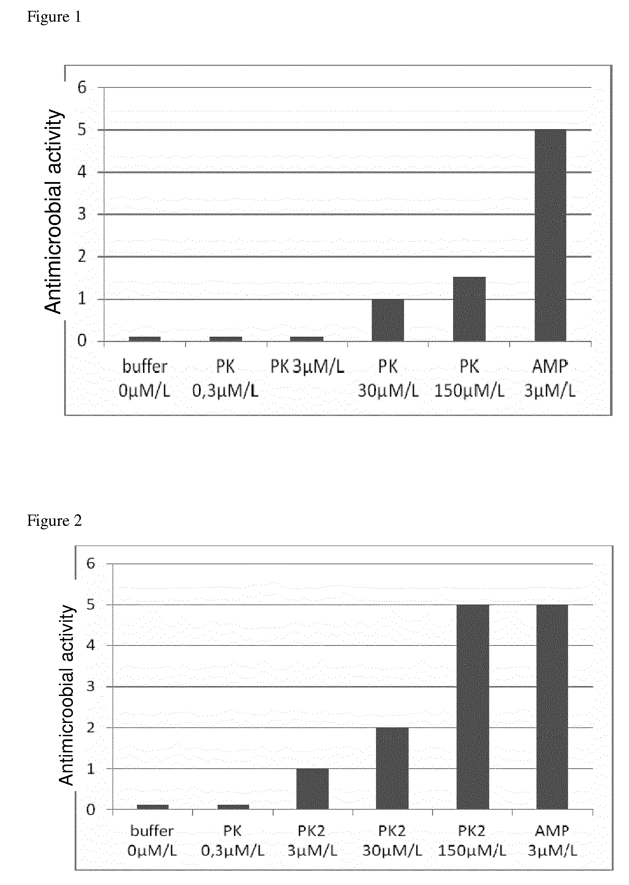

Figure 1 shows a bar chart representing the antibacterial activities of a polycationic peptide named PK (SEQ ID NO: 23) at different concentrations compared to buffer (10 mM HEPES, 0.5 mM EDTA; pH 7.4) and an antimicrobial peptide (AMP = SMAP-29; SEQ ID NO: 45). The antibacterial activities are given as relative inactivation in logarithmic units on the y-axis.
Figure 2 shows a bar chart representing the antibacterial activities shows the antibacterial activities of a polycationic peptide named PK2 (SEQ ID NO: 34) at different concentrations compared to buffer (10 mM HEPES, 0.5 mM EDTA; pH 7.4) and an antimicrobial peptide (AMP = SMAP-29, SEQ ID NO: 45). The antibacterial activities are given as relative inactivation in logarithmic units on the y-axis.

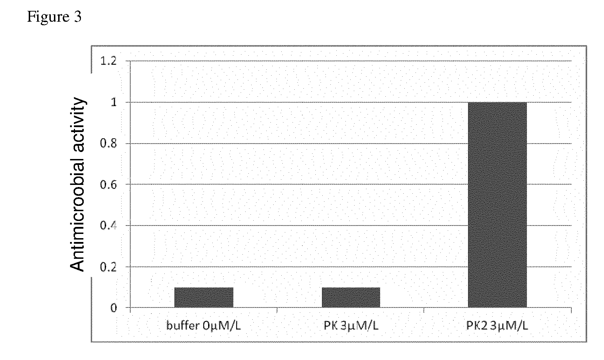

Figure 3 shows a bar chart representing the antibacterial activities of a polycationic peptide named PK (SEQ ID NO: 23) and a polycationic peptide named PK2 (SEQ ID NO: 34) at a concentration of 3 µM/L compared to buffer (10 mM HEPES, 0.5 mM EDTA; pH 7.4). The antibacterial activities are given as relative inactivation in logarithmic units on the y-axis.

The term "protein" as used herein refers to a linear polymer of amino acid residues linked by peptide bonds in a specific sequence. The amino acid residues of a protein may be modified by e.g. covalent attachments of various groups such as carbohydrates and phosphate. Other substances may be more loosely associated with the protein, such as heme or lipid, giving rise to the conjugated proteins which are also comprised by the term "protein" as used herein. The various ways in which the protein fold have been elucidated, in particular with regard to the presence of alpha helices and beta-pleated sheets. The term "protein" as used herein refers to all four classes of proteins being all-alpha, all-beta, alpha/beta and alpha plus beta. Moreover, the term "protein" refers to a complex, wherein the complex refers to a homomer.

The term "fusion protein" as used herein refers to an expression product resulting from the fusion of three nucleic acid sequences. Such a protein may be produced, e.g. in recombinant DNA expression systems. Moreover, the term "fusion protein" as used herein refers to a fusion of a first amino acid sequence selected from the group consisting of SEQ ID NO: 1 to 18 with a second and a third amino acid sequence. The second and third amino acid sequences are a polycationic peptide and an antimicrobial peptide, wherein at least 70% of the amino acid residues of the polycationic peptide are positively charged amino acid residues, in particular lysine and/or arginine residues, and wherein the antimicrobial peptide exhibits an amino acid sequence selected from the group consisting of SEQ ID NOs: 44 to 63 and 78 to 98. Moreover, the fusion protein of the present invention also refers to an expression product resulting from the fusion of at least three nucleic acid sequences. The fusion protein also refers to a fusion of a first amino acid sequence e.g. an endolysin, with more than two additional amino acid sequences encoding peptide stretches. The amino acid sequences encoding peptide stretches of the fusion protein may be distinct from each other or the same.

The term "peptide stretch" as used herein refers to a polycationic peptide and/or an antimicrobial peptide. However, a peptide stretch in the meaning of the present invention does not refer to tags like His-tags, preferably His₅-tags, His₆-tags, His₇-tags, His₈-tags, His₉-tags, His₁₀-tags, His₁₁-tags, His₁₂-tags, His₁₆-tags and His₂₀-tags, Strep-tags, Avi-tags, Myc-tags, Gst-tags, JS-tags, cystein-tags, FLAG-tags or other tags known in the art, thioredoxin or maltose binding proteins (MBP). The term "tag" in contrast to the term "peptide stretch" as used herein refers to a peptide which can be useful to facilitate expression and/or affinity purification of a polypeptide, to immobilize a polypeptide to a surface or to serve as a marker or a label moiety for detection of a polypeptide e.g. by antibody binding in different ELISA assay formats as long as the function making the tag useful for one of the above listed facilitation is not caused by the positively charge of said peptide. However, the His₆-tag may, depending on the respective pH, also be positively charged, but is used as affinity purification tool as it binds to immobilized divalent cations and is not used as a peptide stretch according to the present invention.

The term "first peptide stretch" as used herein refers to a peptide stretch which is linked to the enzyme on the N- or C-terminus of said enzyme.

The term "second peptide stretch" as used herein refers to a peptide stretch which is linked to the first peptide stretch on the N- or C-terminus of said first peptide stretch.

The term "peptide" as used herein refers to short polypeptides consisting of from about 2 to about 100 amino acid residues, more preferably from about 4 to about 50 amino acid residues, more preferably from about 5 to about 30 amino acid residues, wherein the amino group of one amino acid residue is linked to the carboxyl group of another amino acid residue by a peptide bond. A peptide may have a specific function. A peptide can be a naturally occurring peptide or a synthetically designed and produced peptide. The peptide can be, for example, derived or removed from a native protein by enzymatic or chemical cleavage, or can be prepared using conventional peptide synthesis techniques (e.g. solid phase synthesis) or molecular biology techniques (see Sambrook, J. et al., Molecular Cloning: A Laboratory Manual, Cold Spring Harbor Press, Cold Spring Harbor, N.Y. (1989)). Preferred naturally occurring peptides are e.g. antimicrobial peptides, defensins, and sushi peptides. A peptide in the meaning of the present invention does not refer to His-tags, Strep-tags, thioredoxin or maltose binding proteins (MBP) or the like, which are used to purify or locate proteins.

The term "endolysin" as used herein refers to an enzyme which has a peptidoglycan degrading effect and is naturally encoded by bacteriophages or bacterial viruses and which is suitable to degrade bacterial cell walls. "Endolysins" comprise at least one "enzymatically active domain" (EAD) having at least one of the following activities: endopeptidase, N-acetyl-muramoyl-L-alanine-amidase (amidase), N-acetyl-muramidase, N-acetyl-glucosaminidase or transglycosylases. In addition, the endolysins may contain also regions which are enzymatically inactive and bind to the cell wall of the host bacteria, wherein said regions are CBDs (cell wall binding domains). The endolysin may contain two or more CBDs. Generally, the cell wall binding domain is able to bind different components on the surface of bacteria. Preferably, the cell wall binding domain is a peptidoglycan binding domain and binds to the bacteria's peptidoglycan structure. The different domains of an endolysin can be connected by a domain linker.

The term "domain linker" as used herein refers to an amino acid sequence functioning to connect single protein domains with one another. As a rule domain linkers form no or only few regular secondary structures like α-helices or ß-sheets and can occupy different conformations with the respective structural context. Methods to detect domain linker and properties of linker sequences are well known in the art as e.g. described in Bae et al., 2005, Bioinformatics, 21, 2264-2270 or George & Heringa, 2003, Protein Engineering, 15, 871-879.

The term "deletion" as used herein refers to the removal of 1, 2, 3, 4, 5 or more amino acid residues from the respective starting sequence.

The term "insertion" or "addition" as used herein refers to the insertion or addition of 1, 2, 3, 4, 5 or more amino acid residues to the respective starting sequence.

The term "substitution" as used herein refers to the exchange of an amino acid residue located at a certain position for a different one.

The term "cell wall" as used herein refers to all components that form the outer cell enclosure of the Gram-positive and Gram-negative bacteria and thus guarantee their integrity. In particular, the term "cell wall" as used herein refers to peptidoglycan, the outer membrane of the Gram-negative bacteria with the lipopolysaccharide, the bacterial cell membrane, but also to additional layers deposited on the peptidoglycan as e.g. capsules, outer protein layers or slimes.

The term "EAD" as used herein refers to the enzymatically active domain of an endolysin. The EAD is responsible for hydrolysing bacterial peptidoglycans. It exhibits at least one enzymatic activity of an endolysin. The EAD can also be composed of more than one enzymatically active module. The term "EAD" is used herein synonymously with the term "catalytic domain".

As used herein, the term "cationic peptide" refers to a synthetic peptide having positively charged amino acid residues. Preferably a cationic peptide has a pKa-value of 9.0 or greater. Typically, at least four of the amino acid residues of the cationic peptide can be positively charged, for example, lysine or arginine. "Positively charged" refers to the side chains of the amino acid residues which have a net positive charge at about physiological conditions. The term "cationic peptide" as used herein refers also to polycationic peptides.

The term "polycationic peptide" as used herein refers to a synthetically produced peptide composed of mostly positively charged amino acid residues, in particular lysine and/or arginine residues. A peptide is composed of mostly positively charged amino acid residues of at least about 70, 75, 80, 85, 90, 95 or about 100 % of the amino acid residues are positively charged amino acid residues, in particular lysine and/or arginine residues. The amino acid residues being not positively charged amino acid residues can be neutrally charged amino acid residues and/or negatively charged amino acid residues and/or hydrophobic amino acid residues. Preferably the amino acid residues being not positively charged amino acid residues are neutrally charged amino acid residues, in particular serine and/or glycine.

The term, "antimicrobial peptide" (AMP) as used herein refers to any naturally occurring peptide that has microbicidal and/or microbistatic activity on for example bacteria, viruses, fungi, yeasts, mycoplasma and protozoa. Thus, the term "antimicrobial peptide" as used herein refers in particular to any peptide having anti-bacterial, anti-fungal, anti-mycotic, antiparasitic, anti-protozoal, anti-viral, anti-infectious, anti-infective and/or germicidal, algicidal, amoebicidal, microbicidal, bactericidal, fungicidal, parasiticidal, protozoacidal, protozoicidal properties.

The term "sushi peptide" as used herein refers to complement control proteins (CCP) having short consensus repeats. The sushi module of sushi peptides functions as a protein-protein interaction domain in many different proteins. Peptides containing a Sushi domain have been shown to have antimicrobial activities. Preferably, sushi peptides are naturally occurring peptides.

The term "hydrophobic group" as used herein refers to chemical groups such as amino acid side chains which are substantially water insoluble, but soluble in an oil phase, with the solubility in the oil phase being higher than that in water or in an aqueous phase. In water, amino acid residues having a hydrophobic side chain interact with one another to generate a nonaqueous environment. Examples of amino acid residues with hydrophobic side chains are valine, isoleucine, leucine, methionine, phenylalanine, tryptophan, cysteine, alanine, tyrosine, histidine, threonine, serine, proline and glycine residues.

The present invention relates to new antibacterial agents against Gram-positive and/or Gram-negative bacteria, in particular to fusion proteins composed of an enzyme exhibiting an amino acid sequence selected from the group consisting of SEQ ID NO: 1 to 18 and having the activity of degrading the cell wall of Gram-positive and/or Gram-negative bacteria and two or more peptide stretches as defined in the claims. These peptide stretches may be the same or distinct. These peptide stretches are linked to the N- or C-terminus of the enzyme of the fusion protein according to the present invention. The skilled person understands that the fusion proteins according to the present invention comprises 2, 3, 4, 5 or more peptide stretches.

In a preferred embodiment the fusion proteins according to the present invention are composed of an enzyme exhibiting an amino acid sequence selected from the group consisting of SEQ ID NO: 1 to 18 and having the activity of degrading the cell wall of Gram-positive and/or Gram-negative bacteria and two peptide stretches as defined in claim 1 fused to the enzyme on the N- or C-terminus.

The inventors found out that the fusion proteins according to the present invention show different spectra in view of the species to be effected.

Preferred fusion proteins according to the present invention are depicted in SEQ ID NO: 74, 75, 127, 128, 139 to 141 and 147 and may comprise one or more additional amino acid residues on the N-terminus. Preferably the additional amino acid residue is methionine.

In a preferred embodiment, the enzyme according to the present invention has cell wall degrading activity against Gram-positive bacteria of bacterial groups, families, genera or species comprising strains pathogenic for humans or animals like *Listeria monocytogenes, Staphylococcus aureus, Enterococcus faecalis, Enterococcus faecium, Streptococcus pneumoniae, Streptococcus pyogenes, Streptococcus mutans, Streptococcus equi, Clostridium difficile, Clostridium botulinum, Clostridium tetani, Clostridium perfringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

The endolysin part is selected from the group consisting of Cpl-1 according to SEQ ID NO: 1, Ply511 according to SEQ ID NO: 2, LysK according to SEQ ID NO: 3, Lysostaphin according to SEQ ID NO: 4, PA6-gp20 according to SEQ ID NO: 5, phiKZgp144 according to SEQ ID NO: 6, ELgp188 according to SEQ ID NO: 7, *Salmonella* endolysin according to SEQ ID NO:8, Enterobacteria phage T4 endolysin according to SEQ ID NO: 9, *Acinetobacter* baumanii endolysin according to SEQ ID NO: 10, *E.coli* Phage K1F endolysin according to SEQ ID NO: 11, OBPgpLYS according to SEQ ID NO: 12, PSP3 *Salmonella* endolysin (PSP3gp10) according to SEQ ID NO:13, *E.coli* Phage P2 endolysin (P2gp09) according to SEQ ID NO: 14, *Salmonella typhimurium* phage muramidase STM0016 according to SEQ ID NO:15, *E. coli* Phage N4 muramidase N4-gp61 according to SEQ ID NO: 16 and N4-gp61 trunc. according to SEQ ID NO: 17, KZ144 according to SEQ ID NO: 18.

The endolysins of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, pegylation, chemical changes of the amino-, SH- or carboxyl-groups. Said endolysins of the fusion protein according to the present invention exhibit the lytic activity of the respective wild-type endolysin. However, said activity can be the same, higher or lower as the activity of the respective wild-type endolysin. Said activity can be about 10, 20, 30, 40, 50, 60, 70, 80, 90, 100, 110, 120, 130, 140, 150, 160, 170, 180, 190 or about 200 % of the activity of the respective wild-type endolysin or even more. The activity can be measured by assays well known in the art by a person skilled in the art as e.g. the plate lysis assay or the liquid lysis assay which are e.g. described in Briers et al., J. Biochem. Biophys Methods 70: 531-533, (2007*)* or Donovan DM, Lardeo M, Foster-Frey J. FEMS Microbiol Lett. 2006 Dec;265(1*)* or similar publications.

The peptide stretches of the fusion protein according to the present invention may be linked to the enzyme by additional amino acid residues e.g. due to cloning reasons. The peptide stretches of the fusion protein according to the present invention may be linked to each other by additional amino acid residues. Preferably, said additional amino acid residues may be not recognized and/or cleaved by proteases. Preferably said peptide stretches may be linked to each other and/or to the enzyme by at least 1, 2, 3, 4, 5, 6, 7, 8, 9 or 10 additional amino acid residues. In a preferred embodiment the second peptide stretch is linked to the first peptide stretch which is fused to the N- or C-terminus of the enzyme by the additional amino acid residues glycine, serine and serine (Gly-Ser-Ser), glycine, alanine, glycine and alanine (Gly-Ala-Gly-Ala), glycine, alanine, glycine, alanine, glycine, alanine, glycine and alanine (Gly-Ala-Gly-Ala-Gly-Ala-Gly-Ala) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala). Moreover, the second peptide stretch fused on the N-terminus of the first peptide stretch of the fusion protein according to the invention further comprises additional amino acids on its N-terminus. Preferably the peptide stretch comprises the amino acid methionine (Met), or methionine, glycine and serine (Met-Gly-Ser). In another preferred embodiment the first peptide stretch is linked to the N-terminus of the enzyme by additional amino acid residues, in particular glycine and serine (Gly-Ser) and the second peptide stretch is linked to the N-terminus of the first peptide stretch by additional amino acid residues, in particular glycine and serine (Gly-Ser) or glycine, serine and serine (Gly-Ser-Ser). In another preferred embodiment the second peptide stretch is linked to the N- or C-terminus of the first peptide stretch by additional amino acid residues glycine, serine and serine (Gly-Ser-Ser), glycine, alanine, glycine and alanine (Gly-Ala-Gly-Ala), glycine, alanine, glycine, alanine, glycine, alanine, glycine and alanine (Gly-Ala-Gly-Ala-Gly-Ala-Gly-Ala) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala). In another preferred embodiment the first peptide stretch is linked to the C-terminus of the enzyme by additional amino acid residues, in particular glycine and serine (Gly-Ser) and the second peptide stretch is linked to the C-terminus of the first peptide stretch by additional amino acid residues, in particular glycine and serine (Gly-Ser).

The peptide stretches of the fusion protein according to the present invention are preferably covalently bound to the enzyme. Said peptide stretches consist of at least 5, more preferably at least of 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 amino acid residues. Especially preferred are peptide stretches having a length of 5 to 30 amino acid residues. More preferred are peptide stretches comprising 6 to 42 amino acid residues, 6 to 39 amino acid residues, 6 to 38 amino acid residues, 6 to 31 amino acid residues, 6 to 25 amino acid residues, 6 to 24 amino acid residues, 6 to 22 amino acid residues, 6 to 21 amino acid residues, 6 to 20 amino acid residues, 6 to 19 amino acid residues, 6 to 16 amino acid residues, 6 to 14 amino acid residues, 6 to 12 amino acid residues, 6 to 10 amino acid residues or 6 to 9 amino acid residues.

The peptide stretches are no tag such as a His-tag, Strep-tag, Avi-tag, Myc-tag, Gst-tag, JS-tag, cystein-tag, FLAG-tag or other tags known in the art and no thioredoxin or maltose binding proteins (MBP). However, the fusion protein according to the present invention may comprise in addition such tag or tags.

More preferably the peptide stretches have the function to lead the fusion protein through the outer membrane but may have activity or may have no or only low activity when administered without being fused to the enzyme. The function to lead the fusion protein through the outer membrane of Gram-negative bacteria is caused by the potential of the outer membrane or LPS disrupting or permeabilising or destabilizing activity of said peptide stretches. Such outer membrane or LPS disrupting or permeabilising or destabilizing activity of the peptide stretches may be determined in a method as follows: The bacteria cells to be treated are cultured in liquid medium or on agar plates. Then the bacteria cell concentration in the liquid medium is determined photometrically at OD600nm or the colonies on the agar plates are counted, respectively. Now, the bacteria cells in liquid medium or on the plates are treated with a fusion protein according to the invention. After incubation the bacteria cell concentration in the liquid medium is determined photometrically at OD600nm or the colonies on the agar plates are counted again. If the fusion protein exhibits such outer membrane or LPS disrupting or permeabilising or destabilizing activity, the bacteria cells are lysed due to the treatment with the fusion protein and thus, the bacteria cell concentration in the liquid medium or the number of the bacteria colonies on the agar plate is reduced. Thus, the reduction in bacteria cell concentration or in the number of bacteria colonies after treatment with fusion protein is indicative for an outer membrane or LPS disrupting or permeabilising or destabilizing activity of the fusion protein.

The peptide stretches of the fusion protein according to the present invention are selected from the group of polycationic peptides and antimicrobial peptides. The two or more peptide stretches at the N- or C-terminus of the fusion protein according to the invention are any combination of a polycationic peptide and an antimicrobial peptide as defined in claim 1.

Especially preferred are polycationic peptide stretches comprising at least one motive according to SEQ ID NO: 19 (KRKKRK). In particular cationic peptide stretches comprising at least 2, 3, 4, 5, 6, 7, 8, 9, 10, 11, 12, 13, 14, 15, 16 or 17 motives according to SEQ ID NO: 19 (KRKKRK) are preferred. More preferred are cationic peptide stretches comprising at least one KRK motive (lys-arg-lys), preferable at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32 or 33 KRK motives.

In another preferred embodiment of the present invention the cationic peptide stretch comprises beside the positively charged amino acid residues, in particular lysine and/or arginine residues, neutrally charged amino acid residues, in particular glycine and/or serine residues. The cationic peptide stretches consist of 70 % to 100 %, or 80 % to 95 %, or 85 % to 90 % positively charged amino acid residues, in particular lysine, arginine and/or histidine residues, more preferably lysine and/or arginine residues and of 0 % to 30 %, or 5 % to 20 %, or 10 % to 20 % neutrally charged amino acid residues, in particular glycine and/or serine residues. Preferred are polypeptide stretches consisting of 4 % to 8 % serine residues, of 33 % to 36 % arginine residues and of 56 % to 63 % lysine residues. Especially preferred are polypeptide stretches comprising at least one motive according to SEQ ID NO: 20 (KRXKR), wherein X is any other amino acid than lysine, arginine and histidine. Especially preferred are polypeptide stretches comprising at least one motive according to SEQ ID NO: 21 (KRSKR). More preferred are cationic stretches comprising at least about 2, 3, 4, 5, 6, 7, 8, 9, 10, 11, 12, 13, 14, 15, 16, 17, 18, 19 or about 20 motives according to SEQ ID NO: 20 (KRXKR) or SEQ ID NO: 21 (KRSKR).

Also preferred are polypeptide stretches consisting of about 9 to about 16 % glycine residues, of about 4 to about 11 % serine residues, of about 26 to about 32 % arginine residues and of about 47 to about 55 % lysine residues. Especially preferred are polypeptide stretches comprising at least one motive according to SEQ ID NO: 22 (KRGSG). More preferred are cationic stretches comprising at least about 2, 3, 4, 5, 6, 7, 8, 9, 10, 11, 12, 13, 14, 15, 16, 17, 18, 19 or about 20 motives according to SEQ ID NO: 22 (KRGSG).

In another preferred embodiment of the present invention the cationic peptide stretch comprises beside the positively charged amino acid residues, in particular lysine and/or arginine residues, hydrophobic amino acid residues, in particular valine, isoleucine, leucine, methionine, phenylalanine, tryptophan, cysteine, alanine, tyrosine, histidine, threonine, serine, proline and glycine residues, more preferably alanine, valine, leucine, isoleucine, phenylalanine, and/or tryptophan residues. The cationic peptide stretches consist of 70 % to 100 %, or 80 % to 95 %, or 85 % to 90 % positively charged amino acid residues, in particular lysine and/or arginine residues and of about 0 % to about 30 %, or about 5 % to about 20 %, or about 10 % to about 20 % hydrophobic amino acid residues, valine, isoleucine, leucine, methionine, phenylalanine, tryptophan, cysteine, alanine, tyrosine, histidine, threonine, serine, proline and glycine residues, more preferably alanine, valine, leucine, isoleucine, phenylalanine, and/or tryptophan residues.

Examples for cationic and polycationic peptide stretches are listed in the following table:

**Table 1:**

| **peptide stretch** | **length** | **SEQ ID NO:** |
|---|---|---|
| KRKKRK | 6 | SEQ ID NO:19 |
| KRKKRKKRK | 9 | SEQ ID NO:23 |
| RRRRRRRRR | 9 | SEQ ID NO:24 |
| KKKKKKKK | 8 | SEQ ID NO:25 |
| KRKKRKKRKK | 10 | SEQ ID NO:26 |
| KRKKRKKRKKRK | 12 | SEQ ID NO:27 |
| KRKKRKKRKKRKKR | 14 | SEQ ID NO:28 |
| KKKKKKKKKKKKKKKK | 16 | SEQ ID NO:29 |
| KRKKRKKRKKRKKRKKRK | 18 | SEQ ID NO:30 |
| KRKKRKKRKKRKKRKKRKK | 19 | SEQ ID NO:31 |
| RRRRRRRRRRRRRRRRRRR | 19 | SEQ ID NO:32 |
| KKKKKKKKKKKKKKKKKKK | 19 | SEQ ID NO:33 |
| KRKKRKKRKRSKRKKRKKRK | 20 | SEQ ID NO:34 |
| KRKKRKKRKRSKRKKRKKRKK | 21 | SEQ ID NO:35 |
| KRKKRKKRKKRKKRKKRKKRK | 21 | SEQ ID NO:36 |
| KRKKRKKRKRGSGKRKKRKKRK | 22 | SEQ ID NO:37 |
| KRKKRKKRKRGSGSGKRKKRKKRK | 24 | SEQ ID NO:38 |
| KRKKRKKRKKRKKRKKRKKRKKRKK | 25 | SEQ ID NO:39 |
| KRKKRKKRKRSKRKKRKKRKRSKRKKRKKRK | 31 | SEQ ID NO:40 |
| KRKKRKKRKRGSGSGKRKKRKKRKGSGSGKRKKRKKRK | 38 | SEQ ID NO:41 |
| KRKKRKKRKKRKKRKKRKKRKKRKKRKKRKKRKKRKKRK | 39 | SEQ ID NO:42 |
| KRKKRKKRKRSKRKKRKKRKRSKRKKRKKRKRSKRKKRKKRK | 42 | SEQ ID NO:43 |

Antimicrobial peptides according to the present invention are listed in the following table.

**Table 2:**

| **Peptide** | **Sequence** | **SEQ ID NO** |
|---|---|---|
| LL-37 | LLGDFFRKSKEKIGKEFKRIVQRIKDFLRNLVPRTES | SEQ ID NO:44 |
| SMAP-29 | RGLRRLGRKIAHGVKKYGPTVLRIIRIAG | SEO ID NO:45 |
| Indolicidin | ILPWKWPWWPWRR | SEQ ID NO:46 |
| Protegrin | RGGRLCYCRRRFCVCVGR | SEQ ID NO:47 |
| Cecropin P1 | SWLSKTAKKLENSAKKRISEGIAIAIQGGPR | SEQ ID NO:48 |
| Magainin | GIGKFLHSAKKFGKAFVGEIMNS | SEQ ID NO:49 |
| Pleurocidin | GWGSFFKKAAHVGKHVGKAALTHYL | SEQ ID NO:50 |
| Cecropin A (A.aeavpti) | GGLKKLGKKLEGAGKRVFNAAEKALPVVAGAKALRK | SEQ ID NO:51 |
| Cecropin A (D. melanogaster) | | SEQ ID NO:52 |
| Buforin II | TRSSRAGLQFPVGRVHRLLRK | SEQ ID NO:53 |
| Sarcotoxin IA | | SEQ ID NO:54 |
| Apidaecin | ANRPVYIPPPRPPHPRL | SEQ ID NO:55 |
| Ascaphine 5 | GIKDWIKGAAKKLIKTVASHIANQ | SEQ ID NO:56 |
| Nigrocine 2 | GLLSKVLGVGKKVLCGVSGLVC | SEQ ID NO:57 |
| Pseudin 1 | GLNTLKKVFQGLHEAIKLINNHVQ | SEQ ID NO:58 |
| Ranalexin | FLGGLIVPAMICAVTKKC | SEQ ID NO:59 |
| Melittin | GIGAVLKVLTTGLPALISWIKRKRQQ | SEQ ID NO:60 |
| Lycotoxin 1 | IWLTALKFLGKHAAKKLAKQQLSKL | SEQ ID NO:61 |
| Parasin 1 | KGRGKQGGKVRAKAKTRSS | SEQ ID NO:62 |
| Buforin I | | SEQ ID NO:78 |
| Dermaseptin 1 | ALWKTMLKKLGTMALHAGKAALGAAADTISQGTQ | SEQ ID NO:79 |
| Bactenecin 1 | RLCRIVVIRVCR | SEQ ID NO:80 |
| Thanatin | GSKKPVPIIYCNRRTGKCQRM | SEQ ID NO:81 |
| Brevinin 1T | VNPIILGVLPKVCLITKKC | SEQ ID NO:82 |
| Ranateurin 1 | SMLSVLKNLGKVGLGFVACKINIKQC | SEQ ID NO:83 |
| Esculentin 1 | | SEQ ID NO:84 |
| Tachyplesin | RWCFRVCYRGICYRKCR | SEQ ID NO:85 |
| Androctonin | RSVCRQIKICRRRGGCYYKCTNRPY | SEQ ID NO:86 |
| alpha-defensin | DCYCRIPACIAGERRYGTCIYQGRLWAFCC | SEQ ID NO:87 |
| beta-defensin | NPVSCVRNKGICVPIRCPGSMKQIGTCVGRAVKCCRKK | SEQ ID NO:88 |
| theta-defensin | GFCRCLCRRGVCRCICTR | SEQ ID NO:89 |
| defensin (sapecin A) | | SEQ ID NO:90 |
| Thionin (crambin) | | SEQ ID NO:91 |
| defensin from radish | | SEQ ID NO:92 |
| Drosomycin | | SEQ ID NO:93 |
| Hepcidin | DTHFPICIFCCGCCHRSKCGMCCKT | SEQ ID NO:94 |
| Bac 5 | | SEQ ID NO:95 |
| PR-39 | | SEQ ID NO:96 |
| Pyrrhocoricin | VDKGSYLPRPTPPRPIYNRN | SEQ ID NO:97 |
| Histatin 5 | DSHAKRHHGYKRKFHEKHHSHRGY | SEQ ID NO:98 |

In a further aspect of the present invention at least one of the fused peptide stretches is the sushi 1 peptide according to SEQ ID NO: 63.

In another preferred embodiment of the present invention the peptide stretches of the fusion protein according to the present invention comprise modifications and/or alterations of the amino acid sequences. Such alterations and/or modifications may comprise mutations such as deletions, insertions and additions, substitutions or combinations thereof and/or chemical changes of the amino acid residues, e.g. biotinylation, acetylation, pegylation, chemical changes of the amino-, SH- or carboxyl-groups.

According to the invention the two peptide stretches of the fusion protein on the N- or C-terminus according to the present invention consist of a polycationic peptide combined with an antimicrobial peptide. The antimicrobial peptide may be Parasin 1 according to SEQ ID NO: 62, Lycotoxin 1 according to SEQ ID NO: 61, SMAP-29 according to SEQ ID NO: 45, LL37 according to SEQ ID NO: 44, Melittin according to SEQ ID NO: 60, Sarcotoxin IA according to SEQ ID NO: 54, Pseudin1 according to SEQ ID NO: 58, Indolicidin according to SEQ ID NO: 46, Buforin II according to SEQ ID NO: 53, Magainin according to SEQ ID NO: 49 or Cecropin A (A. aegyptii) according to SEQ ID NO: 51. In a preferred embodiment the polycationic peptide may be a peptide according to SEQ ID NO: 23 or a peptide according to SEQ ID NO: 34. In another preferred embodiment the antimicrobial peptide may be Parasin 1 according to SEQ ID NO: 62 and the polycationic peptide may be a peptide according to SEQ ID NO: 23. In another preferred embodiment the antimicrobial peptide may be Lycotoxin 1 according to SEQ ID NO: 61 and the polycationic peptide may be a peptide according to SEQ ID NO: 23. In another preferred embodiment the antimicrobial peptide may be SMAP-29 according to SEQ ID NO: 45 and the polycationic peptide may be a peptide according to SEQ ID NO: 23. In another preferred embodiment the antimicrobial peptide may be Cecropin A (A. aegyptii) according to SEQ ID NO: 51 and the polycationic peptide may be a peptide according to SEQ ID NO: 34. The antimicrobial and polycationic peptide of the fusion protein according to the present invention are arranged successively at the N- or C-terminus. The order of the antimicrobial and polycationic peptide on the N- or C-terminus does not matter, e.g. starting at the N-terminus of the fusion protein the antimicrobial peptide is followed by the polycationic peptide which is followed by the enzyme, or the polycationic peptide is followed by the antimicrobial peptide which is followed by the enzyme. Alternatively, the enzyme is followed by polycationic peptide which is followed by the antimicrobial peptide or the enzyme is followed by the antimicrobial peptide which is followed by the polycationic peptide, so that both peptides are arranged at the C-terminus of the enzyme.

In another preferred embodiment the antimicrobial peptide Parasin 1 according to SEQ ID NO: 62 may be combined with the polycationic peptide according to SEQ ID NO: 23. In another preferred embodiment the antimicrobial peptide Lycotoxin 1 according to SEQ ID NO: 61 may be combined with the polycationic peptide according to SEQ ID NO: 23.. In another preferred embodiment the antimicrobial peptide SMAP-29 according to SEQ ID NO: 45 may be combined with the polycationic peptide according to SEQ ID NO: 23. In another preferred embodiment the antimicrobial peptide Cecropin A (A. aegyptii) according to SEQ ID NO: 51 may be combined with the polycationic peptide according to SEQ ID NO: 34.

In a preferred embodiment of the present invention the fusion protein consists of two or more peptide stretches according to SEQ ID NO: 19 to 63 and 78 to 98 and an enzyme according to SEQ ID NO: 1 to 18. In a preferred embodiment of the present invention the fusion protein comprises two or more peptide stretches selected from the group of peptide stretches according to SEQ ID NO: 19 to 63 and 78 to 98 and an enzyme selected from the group of enzymes according to SEQ ID NO: 1 to 18. In another preferred embodiment of the present invention the fusion protein comprises two or more peptide stretches selected from the group of peptides stretches according to SEQ ID NO: 19 to 63 and 78 to 98 and an enzyme selected from the group of enzymes according to SEQ ID NO: 1 to 18, wherein the peptide stretches are fused to the N- or C-terminus of the enzyme.

Specific examples of fusion proteins according to the present invention are listed in the following table:

**Table 3:**

| Fusion protein | Second peptide stretch (N-terminal unless otherwise indicated) | First peptide stretch (N-terminal unless otherwise indicated) | Enzyme part |
|---|---|---|---|
| SEQ ID NO: 74 | Parasin 1 (SEQ ID NO: 62) | polycationic peptide (SEQ ID NO: 23) | OBPgpLys (SEQ ID NO: 12) |
| SEQ ID NO: 75 | Lycotoxin 1 (SEQ ID NO: 61) | polycationic peptide (SEQ ID NO: 23) | OBPgpLys (SEQ ID NO: 12) |
| SEQ ID NO: 127 | Polycationic peptide (SEQ ID NO: 23) | SMAP-29 (SEQ ID NO: 45) | KZ144 (SEQ ID NO: 18) |
| SEQ ID NO: 128 | SMAP-29 (SEQ ID NO: 45) | Polycationic peptide (SEQ ID NO: 23) | EL188 (SEQ ID NO: 7) |
| SEQ ID NO: 139 | (C-terminal of the second peptide stretch) Cecropin A (A. aegyptii) (SEQ ID NO: 51) | (C-terminal of the enzyme) PK2 (SEQ ID NO: 34) | LysK (SEQ ID NO: 3) |
| SEO ID NO: 140 | (C-terminal of the second peptide stretch) Polycationic peptide (SEQ ID NO: 23) | (C-terminal of the enzyme) SMAP-29 (SEQ ID NO: 45) | KZ144 (SEQ ID NO: 18) |
| SEQ ID NO: 141 | (C-terminal of the second peptide stretch) SMAP-29 (SEQ ID NO: 45) | (C-terminal of the enzyme) Polycationic peptide (SEQ ID NO: 23) | EL188 (SEQ ID NO: 7) |
| SEQ ID NO: 147 | PK2 (SEQ ID NO: 34) | Cecropin A (A. aegyptii) (SEQ ID NO: 51) | LysK (SEQ ID NO: 3) |

The fusion protein according to the present invention, and thus in particular the especially preferred fusion proteins according to SEQ ID NO: 74, 75, 127, 128, 139-141 or 147, may additional comprise a methionine on the N-terminus.

The fusion protein according to the present invention, and thus in particular the especially preferred fusion proteins according to SEQ ID NO: 74, 75, 127, 128, 139-141 or 147, may additional comprise a tag e.g. for purification. Preferred is a His₆-tag, preferably at the C-terminus and/or the N-terminus of the fusion protein. Said tag can be linked to the fusion protein by additional amino acid residues e.g. due to cloning reasons. Preferably said tag can be linked to the fusion protein by at least 1, 2, 3, 4, 5, 6, 7, 8, 9 or 10 additional amino acid residues. Preferably said additional amino acid residues may not be recognized and/or cleaved by proteases. In a preferred embodiment the fusion protein comprises a His₆-tag at its C-terminus linked to the fusion protein by the additional amino acid residues lysine and glycine (Lys-Gly) or leucine and glutamic acid (Leu-Glu). Preferably, said additional amino acid residues may be not recognized or cleaved by proteases. In another preferred embodiment the fusion protein comprises a His₆-tag at its N-terminus linked to the fusion protein by the additional amino acid residues lysine and glycine (Lys-Gly) or leucine and glutamic acid (Leu-Glu). In another preferred embodiment the fusion protein comprises a His₆-tag at its N-and C-terminus linked to the fusion protein by the additional amino acid residues lysine and glycine (Lys-Gly) or leucine and glutamic acid (Leu-Glu).

In a more preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine (Gly-Ser), glycine, serine and serine (Gly-Ser-Ser), glycine, alanine, glycine and alanine (Gly-Ala-Gly-Ala), glycine, alanine, glycine, alanine, glycine, alanine, glycine and alanine (Gly-Ala-Gly-Ala-Gly-Ala-Gly-Ala) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala) and/or and the fusion protein comprises on the N-terminus the additional amino acid residues methionine (Met) or methionine and glycine (Met-Gly) or methionine, glycine and serine (Met-Gly-Ser). In another preferred embodiment the fusion protein comprises a His₆-tag at its C-terminus linked to the fusion protein by the additional amino acid residues leucine and glutamic acid (Leu-Glu) and the second peptide stretch of the fusion protein according to the invention is linked to the N-terminus of the first peptide stretch by the additional amino acid residues glycine and serine (Gly-Ser), glycine, serine and serine (Gly-Ser-Ser), glycine, alanine, glycine and alanine (Gly-Ala-Gly-Ala), glycine, alanine, glycine, alanine, glycine, alanine, glycine and alanine (Gly-Ala-Gly-Ala-Gly-Ala-Gly-Ala) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala) and the first peptide stretch is fused to the N-terminus of the enzyme by the additional amino acid residues glycine and serine (Gly-Ser), glycine, serine and serine (Gly-Ser-Ser), glycine, alanine, glycine and alanine (Gly-Ala-Gly-Ala), glycine, alanine, glycine, alanine, glycine, alanine, glycine and alanine (Gly-Ala-Gly-Ala-Gly-Ala-Gly-Ala) or glycine, alanine, glycine, alanine, glycine, alanine, glycine, alanine, glycine, alanine, glycine and alanine (Gly-Ala-Gly-Ala-Gly-Ala-Gly-Ala-Gly-Ala-Gly-Ala) and/or the fusion protein comprises on the N-terminus the additional amino acid residues methionine (Met) or methionine and glycine (Met-Gly) or methionine, glycine and serine (Met-Gly-Ser).

Fusion proteins are constructed by linking at least three nucleic acid sequences using standard cloning techniques as described e.g. by Sambrook et al. 2001, Molecular Cloning: A Laboratory Manual. Such a protein may be produced, e.g., in recombinant DNA expression systems. Such fusion proteins according to the present invention can be obtained by fusing the nucleic acids for endolysin and the respective peptide stretches.

The fusion proteins according to the present invention may be fused or linked to other additional proteins. Example for this other additional protein is thioredoxin.

The present invention further relates to an isolated nucleic acid molecule encoding the fusion protein according to the present invention. The present invention further relates to a vector comprising the nucleic acid molecule according to the present invention. Said vector may provide for the constitutive or inducible expression of said fusion protein according to the present invention.

The invention also relates to a method for obtaining said fusion proteins from a micro-organism, such as a genetically modified suitable host cell which expresses said fusion proteins. Said host cell may be a micro-organism such as bacteria or yeast or an animal cell as e.g. a mammalian cell, in particular a human cell. In one embodiment of the present invention the host cell is a *Pichia pastoris* cell. The host may be selected due to mere biotechnological reasons, e.g. yield, solubility, costs, etc. but may be also selected from a medical point of view, e.g. a non-pathological bacteria or yeast, human cells. One aspect of the present invention refers to a method for the production of a fusion protein comprising culturing a host cell comprising a nucleic acid sequence encoding a fusion protein according to the present invention under conditions supporting the expression of said fusion protein.

Another aspect of the present invention is related to a method for genetically transforming a suitable host cell in order to obtain the expression of the fusion proteins according to the invention wherein the host cell is genetically modified by the introduction of a genetic material encoding said fusion proteins into the host cell and obtain their translation and expression by genetic engineering methods well known by the man skilled in the art.

In a further aspect the present invention relates to a composition, preferably a pharmaceutical composition, comprising a fusion protein according to the present invention and/or a host transformed with a nucleic acid molecule or a vector comprising a nucleotide sequence encoding a fusion protein according to the present invention.

In a preferred embodiment of the present invention the composition comprises additionally agents permeabilizing the outer membrane of Gram-negative bacteria such metal chelators as e.g. EDTA, TRIS, lactic acid, lactoferrin, polymyxin, citric acid and/or other substances as described e.g. by Vaara (Agents that increase the permeability of the outer membrane. Vaara M. Microbiol. Rev. 1992 Sep; 56 (3):395-441). Also preferred are compositions comprising combinations of the above mentioned permeabilizing agents. Especially preferred is a composition comprising about 10 µM to about 100 mM EDTA, more preferably about 50 µM to about 10 mM EDTA, more preferably about 0.5 mM to about 10 mM EDTA, more preferably about 0.5 mM to about 2 mM EDTA, more preferably about 0.5 mM to 1 mM EDTA. However, also compositions comprising about 10 µM to about 0.5 mM EDTA are preferred. Also preferred is a composition comprising about 0.5 mM to about 2 mM EDTA, more preferably about 1 mM EDTA and additionally about 10 mM to about 100 mM TRIS.

The present invention also relates to a fusion protein according to the present invention and/or a host transformed with a nucleic acid comprising a nucleotide sequence encoding a fusion protein according to the present invention for use as a medicament. In a further aspect the present invention relates to the use of a fusion protein according to the present invention and/or a host transformed with a vector comprising a nucleic acid molecule comprising a nucleotide sequence encoding a modified fusion protein according to the present invention in the manufacture of a medicament for the treatment and/or prevention of a disorder, disease or condition associated with Gram-positive and/or Gram-negative bacteria. In particular the treatment and/or prevention of the disorder, disease or condition may be caused by Gram-negative bacteria of bacterial groups, families, genera or species comprising strains pathogenic for humans or animals like *Enterobacteriaceae* (*Escherichia,* especially *E. coli, Salmonella, Shigella, Citrobacter, Edwardsiella, Enterobacter, Hafnia, Klebsiella, especially K. pneumoniae, Morganella, Proteus, Providencia, Serratia, Yersinia), Pseudomonadaceae (Pseudomonas, especially P. aeruginosa, Burkholderia, Stenotrophomonas, Shewanella, Sphingomonas, Comamonas), Neisseria, Moraxella, Vibrio, Aeromonas, Brucella, Francisella, Bordetella, Legionella, Bartonella, Coxiella, Haemophilus, Pasteurella, Mannheimia, Actinobacillus, Gardnerella, Spirochaetaceae* (*Treponema and Borrelia), Leptospiraceae, Campylobacter, Helicobacter, Spirillum, Streptobacillus, Bacteroidaceae* (*Bacteroides, Fusobacterium, Prevotella, Porphyromonas*)*, Acinetobacter,* especially A. *baumanii.* In particular the treatment and/or prevention of the disorder, disease or condition may be caused by Gram-positive bacteria of bacterial groups, families, genera or species comprising strains pathogenic for humans or animals like *Listeria monocytogenes, Staphylococcus aureus, Enterococcus faecalis, Enterococcus faecium, Streptococcus pneumoniae, Streptococcus pyogenes, Streptococcus mutans, Streptococcus equi, Clostridium difficile, Clostridium botulinum, Clostridium tetani, Clostridium perfringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

The present invention further relates to a medicament comprising a fusion protein according to the present invention and/or a host transformed with a nucleic acid comprising a nucleotide sequence encoding a fusion protein according to the present invention.

In a further aspect the present invention relates to a fusion protein according to the present invention and/or an effective amount of a host transformed with a nucleic acid comprising a nucleotide sequence encoding a fusion protein according to the present invention or a composition according to the present invention for use in a method of treating a disorder, disease or condition in a subject in need of treatment and/or prevention, which method comprises administering to said subject an effective amount of a fusion protein according to the present invention and/or an effective amount of a host transformed with a nucleic acid comprising a nucleotide sequence encoding a fusion protein according to the present invention or a composition according to the present invention. The subject may be a human or an animal.

In particular said method of treatment may be for the treatment and/or prevention of a disorder, disease or condition caused by Gram-positive and/or Gram-negative bacteria, in particular of bacterial infections caused by Gram-positive and/or Gram-negative bacteria.

In particular said method of treatment may be for the treatment and/or prevention of infections of the skin, of soft tissues, the respiratory system, the lung, the digestive tract, the eye, the ear, the teeth, the nasopharynx, the mouth, the bones, the vagina, of wounds of bacteraemia and/or endocarditis caused by Gram-positive and/or Gram-negative bacteria, in particular by the Gram-positive and/or Gram-negative bacteria as listed above.

The dosage and route of administration used in the method of treatment (or prophylaxis) depends on the specific disease/site of infection to be treated. The route of administration may be for example oral, topical, nasopharyngeal, parenteral, intravenous, rectal or any other route of administration.

For application of a fusion protein according to the present invention and/or an effective amount of a host transformed with a nucleic acid comprising a nucleotide sequence encoding a fusion protein according to the present invention or a composition according to the present invention to a site of infection or site endangered to be infected a formulation may be used that protects the active compounds from environmental influences such as proteases, oxidation, immune response etc., until it reaches the site of infection. Therefore, the formulation may be capsule, dragee, pill, powder, suppository, emulsion, suspension, gel, lotion, cream, salve, injectable solution, syrup, spray, inhalant or any other medical reasonable galenic formulation. Preferably, the galenic formulation may comprise suitable carriers, stabilizers, flavourings, buffers or other suitable reagents. For example, for topical application the formulation may be a lotion, cream, gel, salve or plaster, for nasopharyngeal application the formulation may be saline solution to be applied via a spray to the nose. For oral administration in case of the treatment and/or prevention of a specific infection site e.g. in the intestine, it can be necessary to protect a fusion protein according to the present invention from the harsh digestive environment of the gastrointestinal tract until the site of infection is reached. Thus, bacteria as carrier, which survive the initial steps of digestion in the stomach and which secret later on a fusion protein according to the present invention into the intestinal environment can be used.

In a specific embodiment of the present invention a fusion protein according to the present invention and/or a host transformed with a vector comprising a nucleic acid molecule comprising a nucleotide sequence encoding a fusion protein according to the present invention is used in the manufacture of a medicament for the treatment and/or prevention of a disorder, disease or condition caused by *Pseudomonas,* particularly by *Pseudomonas aeruginosa* in particular intestinal affections, in particular in infants, infections of the meninges, e.g. meningitis haemorrhagica, infections of the middle ear, the skin (Ecthyma gangraenosum), in particular burns, the urinary tract, rhinitis, bacteremic pneumonia, in particular wherein the patient is suffering from cystic fibrosis or hematologic malignancies such as leukemia, or with neutropenia from immunosuppressive therapy, septicemia, in particular because of long-term intravenous or urinary catheterization, invasive surgical procedures and severe burns, endocarditis, in particular wherein the patient is a intravenous drug user or a patient with complications from open heart surgery, highly destructive ocular infections, in particular after the use of contaminated ophthalmologic solutions or severe facial burns, osteochondritis, in particular as a result of severe trauma or puncture wounds through contaminated clothing.

In another specific embodiment of the present invention the disorder, disease or condition is caused by *Burkholderia pseudomallei,* in particular Whitmore's Disease, chronic pneumonia, septicemia, in particular wherein the patient has a traumatized skin lesion.

In another specific embodiment of the present invention the disorder, disease or condition is caused by *Salmonella thyphimurium* and *Salmonella enteritidis,* in particular acute gastroenteritis and local purulent processes, particularly osteomyelitis, endocarditis, cholecystitis and especially caused by *Salmonella thyphimurrium* meningitis, in particular wherein the patient is less than two years old.

In another specific embodiment of the present invention the disorder, disease or condition is caused by *Salmonella typhi,* in particular typus.

In another specific embodiment of the present invention the disorder, disease or condition is caused by *Salmon ell paratyphi,* in particular paratyphus.

In another specific embodiment of the present invention the disorder, disease or condition is caused by *Acinetobacter baumannii,* in particular bronchitis, pneumonia, wound infections and septicemia, in particular as a result of intravenous catheterization.

In another specific embodiment of the present invention the disorder, disease or condition is caused by *Escherichia coli,* in particular extra intestinal infections, particularly appendicitis, purulent cholecystitis, peritonitis, purulent meningitis and infection of the urinary tract, intraintestinal *E. coli* infections, particularly epidemic enteritis, and infectious disease similar to dysentery, septicemia, enterotoxemia, mastitis and dysentery.

In another specific embodiment of the present invention the disorder, disease or condition is caused by *Klebsiella pneumoniae,* in particular pneumonia, bacteremia, meningitis and infections of the urinary tract.

In a specific embodiment of the present invention a fusion protein according to the present invention and/or a host transformed with a vector comprising a nucleic acid molecule comprising a nucleotide sequence encoding a fusion protein according to the present invention is used in the manufacture of a medicament for the treatment and/or prevention of a disorder, disease or condition caused by *Listeria monocytogenes,* in particular Granulomatosis infantiseptica (listeriosis of newborns), mononucleosis, conjunctivitis, meningitis, granulomatosis septica and the listeriosis of pregnant women.

In another specific embodiment of the present invention the disorder, disease or condition is caused by *Staphylococcus aureus,* in particular infections of the skin like pyoderma, particularly folliculitis, furuncle, carbuncle, abscesses of the sweat glands and pemphigus, and like scaled skin syndrome. The scaled skin syndrome can appear in three clinical pictures: dermatitis exfoliativa, impetigo bullosa and scarlatiniform erythroderma. Moreover the disorder, disease or condition caused by *Staphylococcus aureus* is *Staphylococcus* pneumonia, hospitalism, in particular surgical wound infections, mastitis puerperalis and enterokolitis, and food poisonings.

In another specific embodiment of the present invention the disorder, disease or condition is caused by *Streptococcus pyogenes,* in particular tonsillitis, pharyngitis, scarlet, erysipelas, rheumatic fever and acute glomerulonephritis.

In another specific embodiment of the present invention the disorder, disease or condition is caused by *Streptococcus pneumoniae,* in particular pneumonia, ulcus serpens corneae, otitis media, meningitis, peritonitis, mastoiditis and osteomyelitis.

In another specific embodiment of the present invention the disorder, disease or condition is caused by *Clostridium perfringens,* in particular gas gangrene, enteritis necroticans ulcerosa and food poisonings.

In another specific embodiment of the present invention the disorder, disease or condition is caused by *Clostridium botulinum,* in particular botulism.

In another specific embodiment of the present invention the disorder, disease or condition is caused by *Clostridium difficile,* in particular pseudomembranoes enterokolitis.

In another specific embodiment of the present invention the disorder, disease or condition is caused by *Bacillus anthracis,* in particular cutaneous anthrax, inhalation anthrax, and gastrointestinal anthrax.

In another specific embodiment of the present invention the disorder, disease or condition is caused by *Enterococcus faecalis* or *Enterococcus faecium,* like nosokomial infections and endokarditis.

In another specific embodiment of the present invention the disorder, disease or condition is caused by *Bacillus cereus,* in particular food poisonings, bronchial pneumonia, septicaemia and meningitis.

In another specific embodiment of the present invention the disorder, disease or condition is caused by *Mycobacterium avium, Mycobacterium paratubercarlosis* and *Mycobacterium tuberculosis,* in particular tuberculosis.

In another specific embodiment of the present invention the disorder, disease or condition is caused by *Mycoplasma pneumoniae,* in particular pneumonia, diseases of the upper respiratory tract and inflammations of the ear drum.

In another specific embodiment of the present invention the disorder, disease or condition is caused by *Actinomyces,* in particular actinomycosis in human, cattle, cat and dog.

In another specific embodiment of the present invention the disorder, disease or condition is caused by *Corynebacterium diphteriae,* in particular localized diphtheria of the tonsils, the nose, the nasopharynx or the middle ear, progressive diphtheria of the larynx, the trachea and the bronchi, toxic or maligne diphtheria, skin and wound diphtheria.

Preferably, a fusion protein according to the present invention is used for medical treatment, if the infection to be treated or prevented is caused by multiresistant bacterial strains, in particular by strains resistant against one or more of the following antibiotics: streptomycin, tetracycline, cephalothin, gentamicin, cefotaxime, cephalosporin, ceftazidime or imipenem. Furthermore, a fusion protein according to the present invention can be used in methods of treatment by administering it in combination with conventional antibacterial agents, such as antibiotics, lantibiotics, bacteriocins or endolysins.

The present invention also relates to a pharmaceutical pack comprising one or more compartments, wherein at least one compartment comprises one or more fusion protein according to the present invention and/or one or more hosts transformed with a nucleic acid comprising a nucleotide sequence encoding a fusion protein according to the present invention or a composition according to the present invention.

In another aspect the present invention relates to a process of preparation of a pharmaceutical composition, said process comprising admixing one or more fusion protein according to the present invention and/or one or more hosts transformed with a nucleic acid comprising a nucleotide sequence encoding a fusion protein according to the present invention with a pharmaceutically acceptable diluent, excipient or carrier.

In an even further aspect the composition according to the present invention is a cosmetic composition. Several bacterial species can cause irritations on environmentally exposed surfaces of the patient's body such as the skin. In order to prevent such irritations or in order to eliminate minor manifestations of said bacterial pathogens, special cosmetic preparations may be employed, which comprise sufficient amounts of the fusion protein according to the present invention in order to degrade already existing or freshly settling pathogenic Gram-negative and/or Gram-positive bacteria.

In a further aspect the present invention relates to the fusion protein according to the present invention for use as diagnostic means in medicinal, food or feed or environmental diagnostics, in particular as a diagnostic means for the diagnostic of bacteria infection caused in particular by Gram-positive and/or Gram-negative bacteria. In this respect the fusion protein according to the present invention may be used as a tool to specifically degrade pathogenic bacteria, in particular Gram-positive and/or Gram-negative pathogenic bacteria. The degradation of the bacterial cells by the fusion protein according to the present invention can be supported by the addition of detergents like Triton X-100 or other additives which weaken the bacterial cell envelope like polymyxin B. Specific cell degradation is needed as an initial step for subsequent specific detection of bacteria using nucleic acid based methods like PCR, nucleic acid hybridization or NASBA (Nucleic Acid Sequence Based Amplification), immunological methods like IMS, immunofluorescence or ELISA techniques, or other methods relying on the cellular content of the bacterial cells like enzymatic assays using proteins specific for distinct bacterial groups or species e.g. β-galactosidase for enterobacteria, coagulase for coagulase positive strains.

In a further aspect the present invention relates to the use of the fusion protein according to the present invention for the treatment, removal, reduction or prevention of Gram-positive and/or Gram-negative bacterial contamination of foodstuff, of food processing equipment, of food processing plants, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries.

In a further aspect the present invention relates to a method for removal, reduction or prevention of Gram-positive and/or Gram-negative bacterial contamination of foodstuff, of food processing equipment, of food processing plants, of surfaces coming into contact with foodstuff such as shelves and food deposit areas and in all other situations, where pathogenic, facultative pathogenic or other undesirable bacteria can potentially infest food material, of medical devices and of all kind of surfaces in hospitals and surgeries comprising contacting said foodstuff, food processing equipment, food processing plants, surfaces coming into contact with foodstuff, medical devices or sufaces in hospitals with a fusion protein according to the present invention.

Another aspect of the present invention refers to a method for detecting a bacterium in a sample from a subject or in a food or feed, or environmental sample comprising contacting said sample with a fusion protein according to the present invention. The bacterium which is detected by a fusion protein of the present invention may be a Gram-positive and/or Gram-negative bacterium. A sample of a subject, in particular a human or animal, may be any body fluid, like blood serum and urine, as well as organs and tissues.

In particular, a fusion protein of the present invention may be used prophylactically as sanitizing agent. Said sanitizing agent may be used before or after surgery, or for example during hemodialysis. Moreover, premature infants and immunocompromised persons, or those subjects with need for prosthetic devices may be treated with a fusion protein according to the present invention. Said treatment may be either prophylactically or during acute infection. In the same context, nosocomial infections, especially by antibiotic resistant strains like *Pseudomonas aeruginosa* (FQRP), Acinetobacter species and Enterobacteriaceae such as *E.coli, Salmonella, Shigella, Citrobacter, Edwardsiella, Enterobacter, Hafnia, Klebsiella, Morganella, Proteus, Providencia, Serratia, Yersinia* species, *Methicillin-resistant Staphylococcus aureus, Vancomycin-resistant Enterococcus faecalis, Vancomycin-resistant Enterococcurs faecium, Streptococcus pneumoniae, Propionibacterium acnes, multidrug-resistant Mycobacterium tuberculosis,* may be treated prophylactically or during acute phase with a fusion protein of the present invention. Therefore, a fusion protein according to the present invention may be used as a disinfectant also in combination with other ingredients useful in a disinfecting solution like detergents, tensids, solvents, antibiotics, lanthibiotics, or bacteriocins.

For the use of the fusion protein according to the present invention as a disinfectant e.g. in hospital, dental surgery, veterinary, kitchen or bathroom, the fusion protein can be prepared in a composition in form of e.g. a fluid, a powder, a gel, or an ingredient of a wet wipe or a disinfection sheet product. Said composition may additionally comprise suitable carrier, additives, diluting agents and/or excipients for its respective use and form, respectively, - but also agents that support the antimicrobial activity like EDTA or agents enhance the antimicrobial activity of the fusion proteins. The fusion protein may also be used with common disinfectant agents like, alcohols, aldehydes, oxidizing agents, phenolics, quaternary ammonium compounds or UV-light. For disinfecting for example surfaces, objects and/or devices the fusion protein can be applied on said surfaces, objects and/or devices. The application may occur for instance by wetting the disinfecting composition with any means such as a cloth or rag, by spraying, pouring. The fusion proteins may be used in varying concentration depending on the respective application and the "reaction time" intended to obtain full antimicrobial activity.

In a further aspect the present invention relates to the use of the fusion protein according to the present invention as a food additive.

Another aspect of the present invention is that the invention can be used like a tool box, i.e. any peptide stretch and antimicrobial peptide disclosed above may be fused to any endolysin disclosed herein. Thus, it is possible to combine the respective peptide stretch, which enables the binding of the fusion protein to the respective bacteria and the endolysin, which inhibit the growth of the respective bacteria. Consequently, it is possible to construct a suitable fusion protein for any bacteria which should be eliminated.

The following examples explain the present invention but are not considered to be limiting. Unless indicated differently, molecular biological standard methods were used, as e.g., described by Sambrock et al., 1989, Molecular Cloning: A Laboratory Manual, 2nd edition, Cold Spring Harbor Laboratory Press, Cold Spring Harbor, New York.

### Example 1: N-terminal fusion of antibacterial peptide tags to PK-modified endolysin (PK-OBPgpLys) of Pseudomonas putida phage OBP

PK-OBPgpLys, the modified endolysin variant of *P. putida* phage OBP with an N-terminal polycationic peptide according to SEQ ID NO: 23, was N-terminally fused to natural antibacterial peptide tags, amphiphatic and hydrophobic peptide stretches (Table 4) to investigate its anti Gram-negative activity.

**Table 4: List of antibacterial peptide tags which were fused to PK-OBPgpLYS**

| Tag | Description + size | Amino acid sequence | Reference |
|---|---|---|---|
| α4-helix of T4-lysozyme | Amphiphatic helix (13 aa) | PNRAKRVITTFRT (SEQ ID NO: 68) | Matthews* |
| Pentapeptide (designed) | Hydrophobic (5 aa) | FFVAP (SEQ ID NO: 66) | Briers *et al.,* 2008 |
| Walmagh1 (designed) | Hydrophobic (18 aa) | GFFIPAVILPSIAFLIVP (SEQ ID NO: 65) | Walmagh 1 |
| Walmagh2 (disigned) | Amphiphatic helix (25 aa) | GKPGWLIKKALVFKKLIRRPLKR LA (SEQ ID NO: 69) | Walmagh 2 |
| Parasin 1 | Alpha-helical peptide | KGRGKQGGKVRAKAKTRSS (SEQ ID NO: 62) | Park, Y *et al.,* 1998** |
| Lycotoxin 1 | Amphiphatic helix (25 aa) | IWLTALKFLGKHAAKKLAKQQLS KL (SEQ ID NO:61) | Yan & Adams, 1988*** |

| | | | |
|---|---|---|---|
| ***Matthews, B.W. and Remington, S.J. (1974). The three dimensional structure of the lysozyme from bacteriophage T4. Proc. Natl. Acad. Sci. USA, 71: 4178-4182 ***In* Yup Park, Chan Bae Park, Mi Sun Kim, Sun Chang Kim (1998). Parasin I, an antimicrobial peptide derived from histone H2A in the cat¢sh, Parasilurus asotus. FEBS Letters 437 258-262 *****Yan, L and Adams, M.A. (1998). Lycotoxins, Antimicrobial Peptides from Venom of the Wolf Spider, Lycosa carolinensis J. Biol. Chem, 273:2059-2066*.* | | | |

### Methodology of tag modification of PK-OBPgpLys

To obtain the α4-, Walmagh 1-, Walmagh 2-, Lycotoxin1- and Parasin1-PK-OBPgplys ORFs, the corresponding peptide stretches (created by hybridization of primer pairs, see Table 5) were fused to the ORF which encodes for PK-OBPgpLYS using an adapted version of the Ligation Independent Cloning (LIC) technique. First, an unique Ec1136II restriction site (in bold) was inserted upstream of the PK modified OBPgpLys ORF by a tail PCR, with a specific designed 5' primer encoding the restriction site (5' GGAATGGGGAGCTCCTCCAAACGCAAGAAACGTAAGAAACGCAAAAAAAATAGCGAG AAT 3'; SEQ ID NO: 119) and the standard OBPgpLys reverse primer (5' AACTATTCCGAGTGCTTTCTTTGT 3'; SEQ ID NO: 120) on purified genomic DNA of phage OBP. This extended fragment was then ligated into the pEXP5CT/TOPO® expression vector (Invitrogen, Carlsbad, CA, USA) following the TA cloning protocol of the manufacturer. Pure plasmid was cut once (Ec1136II) and the hybridized peptide cassettes were inserted into the cut plasmid without a ligation step (LIC).

To fuse the PK-peptide stretch in front of the single tagged α4- and Pentapeptide-OBPgpLys ORFs a tail PCR with an extended 5' primer encoding this PK peptide stretch (for PK- α4-OBPgpLys the 5' primer is 5' ATGGGATCCAAACGCAAGAAACGTAAGAAACGCAAAGGCTCCTCCCCGAACCGT GCA 3'; SEQ ID NO:121) and for PK-Pentapeptide-OBPgpLys the 5' primer is 5' ATGGGATCCAAACGCAAGAAACGTAAGAAACGCAAAGCCTCCTCCTTCTTCGTA GCA 3' (SEQ ID NO: 122) and the standard 3' OBPgpLys reverse primer was applied on purified pEXP5-CT/α4-OBPgpLys and pEXP5-CT/ Pentapeptide-OBPgpLys plasmid DNA, respectively. Obtained PCR fragments were then recloned in the pEXP5CT/TOPO® expression vector. Correct insertion of the fragments in the expression vector was verified by sequencing analysis before introducing the construct into a suitable Escherichia coli BL21(DE3)pLysS expression strain.

**Table 5: Used primer pairs for hybridization of antibacterial peptide tags for N-terminal fusion to ORF encoding PK-OBPgpLYS**

| **Tag** | **forward primer** | **reverse primer** |
|---|---|---|
| α4-helix of T4-lysozyme | | |
| Walmagh1 (designed) | | |
| Walmagh 2 (designed) | | |
| Lycotoxin 1 | | |
| Parasin 1 | | |

### Large scale recombinant expression of modified PK-OBPgpLYS fusion variants

Standard expression is performed in Lysogeny Broth (LB) in exponentially growing cells (OD₆₀₀ₙₘ= 0.6) induced with 1mM isopropyl-beta-D-thiogalactopyranoside. Expression parameters like temperature, time and expression strain varied on a protein specific basis in order to optimize the soluble expression levels of the modified endolysins (see Table 6).

For purification, cells from an expression culture (500-600 ml) are harvested (4500 rpm, 30 min, 4°C) and resuspended in 1/25 volumes of lysis buffer (10 mM imidazole, 20 mM NaH₂PO₄, 0.5 M NaCl, pH 7.4). This suspension is frozen/thawed three times prior to sonication (8 x 30 s, amplitude 40% on a Vibra CellTM, Sonics, Dandurry, CT, USA) and filtered through 0.45 and 0.22 µm Durapore membrane filters (Millipore, Billerica, MA, USA). Purification of the His-tagged fusion protein was performed by a one-step protocol employing Ni²⁺-affinity chromatography (HisTrap HP 1 ml column, GE Healthcare, Buckinghamshire, UK) according to the manufacturer's instructions. The Ni²⁺ affinity chromatography is performed in 4 subsequent steps, all on room temperature:
1. *Equilibration* of the *Histrap HP 1 ml* column (GE Healthcare) with 10 column volumes of Washing Buffer (60 mM imidazole, 0.5 mM NaCl and 20 mM NaH₂PO₄-NaOH on pH 7.4) at a flow rate of 0.5 ml/min.
2. *Loading* of the total lysate (with wanted endolysin) on the *Histrap HP 1 ml* column at a flow rate of 0.5 ml/min.
3. Washing of the column with 15 column volumes of Washing Buffer at a flow rate of 1 ml/min.
4. Elution of bounded endolysin from the column with 10 column volumes of Elution Buffer (500 mM imidazole, 5 mM NaCl and 20 mM NaH₂PO₄-NaOH on pH 7.4) at a flow rate of 0.5 ml/min

The wash buffer included a low imidazole concentration which varied on protein specific base to ensure higher purity of the protein (see Table 6). The total yields of recombinant proteins per liter *E.coli* expression culture is also shown in Table 6. The values were determined by spectrophotometric measurement of the protein concentration and the total volume of the purified stock solution at a wavelength of 280 nm. Purified stock solutions were at least 95 % pure as determined visually on SDS-PAGE gels.

**Table 6: Expression parameters and obtained protein yields per liter expression culture of N-terminal modified PK-OBPgpLys variants**

| **Modified endolysin** | **Temperature/ time** | **Expression strain** | **Protein yield (in mg/l)** | **Imidazole (in mM)** |
|---|---|---|---|---|
| **α4-PK-OBPgpLys** | 16°C/overnight | *E.coli* BL21 RIL | 0.56 | 60 |
| **PK-α4-OBPgpLys** | 16°C/overnight | *E.coli* BL21 RIL | 7.61 | 60 |
| **Walmagh 1-PK-OBPgpLys** | 16°C/overnight | *E.coli* BL21 RIL | 0.81 | 60 |
| **Walmagh 2-PK-OBPgpLys** | 16°C/overnight | *E.coli* BL21 RIL | 1.78 | 60 |
| **Lycotoxin1-PK-OBPgpLys** | 16°C/overnight | *E.coli* BL21 RIL | 0.23 | 60 |
| **Parasin1-PK-OBPgpLys** | 16°C/overnight | *E.coli* BL21 RIL | 8.08 | 60 |
| **PK-pentapeptide-OBPgpLys** | 16°C/overnight | *E.coli BL21 RIL* | 45.09 | 60 |

### In vitro antibacterial activity and host range of modified PK-OBPgpLys fusion variants

Exponential growing Gram-negative bacterial cells (OD600nm = 0.6) were 100-fold diluted to a final density of about 10⁶ cells/ml in 5 mM HEPES pH 7.4 and incubated for 30 minutes at room temperature without shaking with the different modified OBPgpLYS variants (in Elution Buffer in an end concentration of 1500 nM). After incubation cell suspensions and as a control untreated bacterial cells without any added modified OBPgpLYS variant were diluted three times (respectively 10⁵-10⁴-10³ cells/ml) in 1x concentrated PBS buffer and 100 µl of each dilution was plated out on LB-medium. The bacteria colonies on the agar plates were counted after an overnight incubation on 37°C. Based on the counted cell numbers the antibacterial activity as the relative inactivation in logarithmic units (= log10N₀/Nᵢ with No = number of untreated cells (of the control) and Nᵢ = number of treated cells, both counted after incubation) is calculated (Table 7). The reduction in the number of bacteria colonies after treatment with fusion protein is indicative for an antimicrobial activity of the different modified OBPgpLYS variants, because the bacterial cells are lysed due to the treatment with the fusion proteins. All samples were replicated in threefold. Averages +/- standard deviations are represented.

**Table 7: In vitro antibacterial activity of different N-terminal modified PK-OBPgpLYS fusion variants on a range of exponential growing Gram-negative species without (A) and with (B) extra addition of the outer membrane permeabilizer EDTA (0.5 mM). Initial density is 10⁶ cells/ml and incubation proceeds for 30 minutes without shaking at RT. Average log reduction values are shown together with standard deviation (stdev).**

| A | | | | | | | | |
|---|---|---|---|---|---|---|---|---|
| **Fusion protein** | *P.* ***aeruginosa* PAOlp** | | ***P. putida G1*** | | ***E. coli XL1* b***lue* | | ***S. typhimurium LT2*** | |
| | average | stdev | average | stdev | average | stdev | average | stdev |
| **α4-PK-OBPgpLys** | 0.59 | 0.02 | 0.02 | 0.13 | 0.55 | 0.06 | 0.22 | 0.03 |
| **PK-α4-OBPgpLys** | 1.10 | 0.05 | 0.01 | 0.04 | 0.54 | 0.17 | 0.23 | 0.04 |
| **Walmagh1-PK-OBPgpLYS** | 0.20 | 0.09 | 0.29 | 0.22 | 1.46 | 0.08 | 0.12 | 0.03 |
| **Walmagh2-PK-OBPgplys** | 0.57 | 0.10 | 0.00 | 0.04 | 1.68 | 0.12 | 0.17 | 0.02 |
| **Lycotoxin1-PK-OBPgpLYS** | 0.08 | 0.05 | 0.56 | 0.10 | 0.41 | 0.04 | 0.17 | 0.07 |
| **Parasin1-PK-OBPgpLys** | 0.75 | 0.03 | 0.16 | 0.04 | 0.70 | 0.09 | 0.27 | 0.11 |
| **PK-Pentapeptide-OBPgplys** | 0.95 | 0.08 | 0.80 | 0.04 | 1.69 | 0.17 | 0.22 | 0.06 |

| B | | | | | | | | |
|---|---|---|---|---|---|---|---|---|
| **Fusion protein** | ***P. aeruginosa* PAOlp** | | ***P. putida G1*** | | ***E. coli XL1 blue*** | | ***S. typhimurium LT2*** | |
| | average | stdev | average | stdev | average | stdev | average | stdev |
| EDTA | 1.82 | +/-0.17 | 0.65 | +/-0.35 | 0.85 | +/-0.16 | 0.08 | +/-0.01 |
| α4-PK-OBPgpLys + | | | | | | | | |
| EDTA | 5.23 | +/-0.03 | 2.98 | +/-0.16 | 0.58 | +/-0.13 | 0.35 | +/-0.11 |
| PK-α4-OBPgpLys + | | | | | | | | |
| EDTA | 5.07 | +/-0.21 | 2.97 | +/-0.09 | 0.86 | +/-0.12 | 0.29 | +/-0.06 |
| Walmagh1-PK-OBPgpLYS+ EDTA | 3.66 | +/-0.12 | 3.22 | +/-0.21 | 1.70 | +/-0.07 | 0.17 | +/-0.07 |
| Walmagh2-PK-OBPgplys +EDTA | 3.91 | +/-0.09 | 2.60 | +/-0.12 | 1.86 | +/-0.10 | 0.25 | +/-0.04 |
| Lycotoxin1-PK-OBPgpLYS + EDTA | 3.73 | +/-0.37 | 2.26 | +/-0.10 | 0.90 | +/-0.24 | 0.29 | +/-0.06 |
| Parasin1-PK-OBPgpLys | | | | | | | | |
| + EDTA | 4.92 | +/-0.03 | 2.32 | +/-0.08 | 1.00 | +/-0.14 | 0.28 | +/-0.05 |
| PK-Pentapept-OBPgpLys + EDTA | 4.00 | +/-0.06 | 3.24 | +/-0.26 | 2.12 | +/-0.33 | 0.42 | +/-0.05 |

### Example 2: Antimicrobial activity of fusion proteins of endolysin (PK-OBPgpLys) of Pseudomonas putida phage OBP with two peptide stretches in comparison to fusion protein of endolysin (PK-OBPgpLys) of Pseudomonas putida phage OBP with only one peptide stretch

### Methodology of production of OBPgpLys with one peptide stretch

Except for the pentapeptide, all peptide stretches were fused to the ORF which encodes for the OBPgpLYS derivative using an adapted version of the Ligation Independent Cloning (LIC) as e.g. described in Berrow *et al.* 2007. Here fore, an unique Ecl136II restriction site was inserted in front of the WT endolysin gene by a tail PCR with a specific designed 5' primer (5'-GGAATGGGGAGCTCCTCCAAAAATAGCGAGAAG-3'; SEQ ID NO: 123) and the standard OBPgpLys derivative reverse primer (5'-AACTATTCCGTGTGCTTTCTTTGT -3'; SEQ ID NO:124) on pure genomic DNA of phage OBP. This extended fragment was then ligated in the pEXP5CT/TOPO® expression vector (Invitrogen, Carlsbad, CA, USA) by following the TA cloning protocol of the manufacturer. Pure plasmid was cutted once in an Ecl136II restriction digest and hybridized peptide cassettes (created by hybridization of primer pairs, see Table 8) were inserted into the cutted plasmid without a necessary ligation step (LIC). For the N-terminal pentapeptide tag fusion a tail PCR with an extended 5' primer which encodes for this pentapeptide (5'-ATGGGATCCTTCTTCGTAGCA CCGGGCTCCTCCAAAAATAGCGAGAAG-3'; SEQ ID NO: 125) and the standard OBPgpLys derivative reverse primer (5'-AACTATTCCGTGTGCTTTCTTTGT-3'; SEQ ID NO: 126) was applied on phage OBP genomic DNA. Correct insertion of the fragments in the expression vector was verified by sequencing analysis before introducing the construct into a suitable *Escherichia coli* BL21(DE3)pLysS expression strain.

**Table 8: Used primer pairs for hybridization of antibacterial peptide tags to ORF encoding the OBPgpLys derivative**

| Tag | forward primer | reverse primer |
|---|---|---|
| α4-helix of T4-lysozyme | | |
| Walmagh1 (designed) | | |
| Walmagh 2 (designed) | | |
| Parasin 1 | | |
| Lycotoxin 1 | | |

The production of_OBPgpLys with two peptide stretches is described above in Example 1. Moreover, the purification of the fusion proteins and the antimicrobial activity assay was performed as described already in Example 1. The results for the antimicrobial activity assay are given in Table 9.

**Table 9 In vitro antibacterial activity of OBPgpLYS fusion variants with one or two peptide stretches on a range of exponential growing Gram-negative species with extra addition of the outer membrane permeabilizer EDTA (0.5 mM). Initial density is 10⁶ cells/ml and incubation proceeds for 30 minutes without shaking at RT. Average log reduction values are shown.**

| Fusion protein | *P. aeruginosa* PAO1p | *P. putida* G1 | *E. coli* Xl-1 | *Salmonella typhimurium* LT2 |
|---|---|---|---|---|
| α4-OBPgpLys | ++ | ++ | ++ | + |
| α4-PK-OBPgpLys | +++ | ++ | + | + |
| PK-α4-OBPgpLys | +++ | ++ | ++ | + |
| Pentapeptide-OBPgpLys | ++ | +++ | ++ | + |
| PK-Pentapeptide-OBPgplys | +++ | +++ | ++ | + |
| Walmagh2-OBPgpLys | ++ | ++ | ++ | + |
| Walmagh2-PK-OBPgplys | +++ | ++ | ++ | + |
| Parasin1-OBPgpLys | +++ | +++ | +++ | ++ |
| Parasin1-PK-OBPgpLys | +++ | ++ | ++ | + |
| Lycotoxin1-OBPgpLys | ++ | +++ | ++ | + |
| Lycotoxin1-PK-OBPgpLys | +++ | ++ | ++ | + |
| OBPgpLYS | + | + | + | + |

| | | | | |
|---|---|---|---|---|
| Abbreviations: +: 1 log; ++: 2-3 log; +++: 4 or more logs | | | | |

**Example 3:** Antimicrobial activity of fusion proteins of Pseudomonas aeruginosa bacteriophage endolysins KZ144 (phage phiKZ) and EL188 (phage EL) with two peptide stretches in comparison to fusion protein of endolysin of *Pseudomonas aeruginosa* phage KZ144 and EL188 with only one or without peptide stretch

The purification of the fusion proteins and the antimicrobial activity assay was performed as described in Example 1. The results for the antimicrobial activity assay are given in Table 10.

**Table 10 In vitro antibacterial activity of KZ144 and EL188 fusion variants with one or two peptide stretches on a range of exponential growing Pseudomonas bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Pseudomonas* |
|---|---|
| KZ144 (SEQ ID NO: 18) | +/- |
| KZ144-PK (SEQ ID NO: 148) | + |
| KZ144-SMAP29 (SEQ ID NO: 149) | ++ |
| KZ144-SMAP29-PK (SEQ ID NO: 140) | +++ |
| EL188 (SEQ ID NO: 7) | +/- |
| EL188-SMAP29(SEQ ID NO: 150) | + |
| EL188-PK (SEQ ID NO: 151) | + |
| EL188-PK-SMAP29 (SEQ ID NO: 141) | +++ |

| | |
|---|---|
| Abbreviations: -: no log reduction; +/-: < 1 log; +: 1 log; ++: 2-3 log; +++: 4 or more logs | |

### Example 4: Antimicrobial activity of fusion proteins of endolysin STM0016 with two peptide stretches in comparison to fusion protein of endolysin STM0016 with only one or without peptide stretch

The purification of the fusion proteins and the antimicrobial activity assay was performed as described in Example 1. The results for the antimicrobial activity assay are given in Table 11.

**Table 11 In vitro antibacterial activity of STM0016 fusion variants with one or two peptide stretches on a range of exponential growing Salmonella bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Salmonella* |
|---|---|
| STM0016 (SEQ ID NO: 15) | - |
| STM0016-SMAP29 (SEQ ID NO: 152) | + |
| STM0016-LL37 (SEQ ID NO: 153) | + |
| STM0016-Melittin (SEQ ID NO: 154) | +/- |
| STM0016-LL37-SMAP29 (SEQ ID NO: 142) | +++ |
| STM0016-SMAP29-Melittin (SEQ ID NO: 143) | ++ |

| | |
|---|---|
| Abbreviations: -: no log reduction; +/-: < 1 log; +: 1 log; ++: 2-3 log; +++: 4 or more logs | |

### Example 5: Antimicrobial activity of fusion proteins of endolysin Lysostaphin with two peptide stretches in comparison to fusion protein of endolysin Lysostaphin with only one or without peptide stretch

The purification of the fusion proteins and the antimicrobial activity assay was performed as described in Example 1. The results for the antimicrobial activity assay are given in Table 12.

**Table 12 In vitro antibacterial activity of Lysostaphin fusion variants with one or two peptide stretches on a range of exponential growing Staphylococcus bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Staphylococcus* |
|---|---|
| Lysostaphin (SEQ ID NO: 4) | + |
| Lysostaphin-Sarcotoxin IA (SEQ ID NO: 155) | ++ |
| Lysostaphin-SMAP29 (SEQ ID NO: 156) | ++ |
| Sarcotoxin IA-Lysostaphin (SEQ ID NO: 157) | ++ |
| SMAP29-Lysostaphin (SEQ ID NO: 158) | ++ |
| Lysostaphin-SMAP29-Sarcotoxin IA (SEQ ID NO: 144) | +++ |
| Sarcotoxin IA-SMAP29-Lysostaphin (SEQ ID NO: 131) | +++ |

| | |
|---|---|
| Abbreviations: -: no log reduction; +/-: < 1 log; +: 1 log; ++: 2-3 log; +++: 4 or more logs | |

### Example 6: Antimicrobial activity of fusion proteins of endolysin Cpl1 with two peptide stretches in comparison to fusion protein of endolysin Cpl1 with only one or without peptide stretch

The purification of the fusion proteins and the antimicrobial activity assay was performed as described in Example 1. The results for the antimicrobial activity assay are given in Table 13.

**Table 13 In vitro antibacterial activity of Cpl1 fusion variants with one or two peptide stretches on a range of exponential growing Streptococcus bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Streptococcus* |
|---|---|
| Cpl1 (SEQ ID NO: 1) | + |
| Cpl1-Indolicidin (SEQ ID NO: 159) | ++ |
| Cpl1-Pseudin1 (SEQ ID NO: 160) | ++ |
| Cpl1-Pseudin1-Indolicidin (SEQ ID NO: 145) | +++ |

| | |
|---|---|
| Abbreviations: -: no log reduction; +/-: < 1 log; +: 1 log; ++: 2-3 log; +++: 4 or more logs | |

### Example 7: Antimicrobial activity of fusion proteins of endolysin Ply511 with two peptide stretches in comparison to fusion protein of endolysin Ply511 with only one or without peptide stretch

The purification of the fusion proteins and the antimicrobial activity assay was performed as described in Example 1. The results for the antimicrobial activity assay are given in Table 14.

**Table 14 In vitro antibacterial activity of Ply511 fusion variants with one or two peptide stretches on a range of exponential growing Listeria bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Listeria* |
|---|---|
| Ply511 (SEQ ID NO: 2) | + |
| Ply511-Magainin (SEQ ID NO: 161) | ++ |
| Ply511-BuforinII (SEQ ID NO: 162) | ++ |
| Ply511-BuforinII-Magainin (SEQ ID NO: 146) | +++ |

| | |
|---|---|
| Abbreviations: -: no log reduction; +/-: < 1 log; +: 1 log; ++: 2-3 log; +++: 4 or more logs | |

### Example 8: Antimicrobial activity of fusion proteins of endolysin LysK with two peptide stretches in comparison to fusion protein of endolysin LysK with only one or without peptide stretch

The purification of the fusion proteins and the antimicrobial activity assay was performed as described in Example 1. The results for the antimicrobial activity assay are given in Table 15.

**Table 15 In vitro antibacterial activity of LysK fusion variants with one or two peptide stretches on a range of exponential growing Staphylococcus bacteria with extra addition of the outer membrane permeabilizer EDTA (0.5 mM). Initial density is 10⁶ cells/ml and incubation proceeds for 30 minutes without shaking at RT. Average log reduction values are shown.**

| Protein | *Staphylococcus* |
|---|---|
| LysK (SEQ ID NO: 3) | + |
| PK2-LysK (SEQ ID NO: 163) | ++ |
| Cecropin A (A. aegyptii)-LysK (SEQ ID NO: 164) | ++ |
| PK2-Cecropin A (A. aegyptii)-LysK (SEQ ID NO: 147) | +++ |

| | |
|---|---|
| Abbreviations: -: no log reduction; +/-: < 1 log; +: 1 log; ++: 2-3 log; +++: 4 or more logs | |

### Example 9: Antimicrobial activity of different peptide stretches

Exponential *P. aeruginosa* PAOlp cells (Burn wound isolate, Queen Astrid Hospital, Brussels; Pirnay JP et al. (2003), http://www.ncbi.nlm.nih.gov/pubmed/12624051?ordinalpos=3&itool=EntrezSystem2.PEntre z.Pubmed.Pubmed_ResultsPanel.Pubmed_DefaultReportPanel.Pubmed_RVDocSum J Clin Microbiol., 41(3):1192-1202) were washed in 10 mM HEPES, pH 7.4 and diluted 1:10 in 10 mM HEPES, 0.5 mM EDTA, pH 7.4. Bacteria were incubated at room temperature with the respective peptide, polycationic peptide named PK according to SEQ ID NO: 23, the polycationic peptide PK2 according to SEQ ID NO: 34 or the antimicrobial peptide named SMAP-29 according to SEQ ID NO: 45 in buffer (15 mM HEPES, 250 mM NaCl, 250 µM EDTA; pH 7.4) in the concentrations 0.3 µmol/L, 3 µmol/L, 30 µmol/L and 150 µmol/L for the polycationic peptides and 3 µmol/L for the antimicrobial peptide. After 30 minutes cell suspensions were serial diluted (1:10) and plated on LB. Additionally, a negative control was plated using buffer (15 mM HEPES, 250 mM NaCl, 250 µM EDTA; pH 7.4). The bacteria colonies on the agar plates were counted after an overnight incubation at 37°C. Based on the counted cell numbers the antibacterial activity as the relative inactivation in logarithmic units (= log10N₀/Nᵢ with No = number of untreated cells (of the control) and Nᵢ = number of treated cells, both counted after incubation) is calculated. The reduction in the number of bacteria colonies after treatment with a peptide stretch is indicative for an antimicrobial activity of the peptide stretch, because the bacterial cells are lysed due to the treatment with the peptide stretch. All samples were replicated in four fold.

Both polycationic peptides PK and PK2 showed increasing antimicrobial activity against P. *aeruginosa* dependent on the used concentration of the peptide. PK2 showed higher antimicrobial activity against *P. aeruginosa* than PK. Moreover, PK2 used in a concentration of 150 µmol/L showed same antimicrobial activity against *P. aeruginosa* as SMAP-29 used in a concentration of 3 µmol/L. For both PK and PK2 more peptide has to be used to achieve the same antimicrobial activity against *P. aeruginosa* as SMAP-29. Usage of equimolar concentrations of PK or PK2 (3 µM/L) show no or only minor antimicrobial effects compared to AMP or Artilysin.

## Claims

1. A fusion protein composed of an enzyme having the activity of degrading the cell wall of Gram-negative bacteria and/or Gram-positive bacteria and at least two peptide stretches fused to the enzyme at the N- or C-terminus, wherein the peptide stretches have a length of 5 to 50 amino acid residues and wherein the peptide stretches are a polycationic peptide and an antimicrobial peptide; and
wherein the enzyme exhibits an amino acid sequence selected from the group consisting of SEQ ID NO: 1 to 18,
wherein at least 70% of the amino acid residues of the polycationic peptide are positively charged amino acid residues, in particular lysine and/or arginine residues, and
wherein the antimicrobial peptide exhibits an amino acid sequence selected from the group consisting of SEQ ID NOs: 44 to 63 and 78 to 98.

2. The fusion protein according to claim 1, wherein the polycationic peptide or antimicrobial peptide has a length of 6 to 39 amino acid residues.

3. The fusion protein according to any of the preceding claims, wherein the Gram-negative bacteria are selected from the group consisting of Enterobacteriaceae,
in particular *Escherichia, Salmonella, Shigella, Citrobacter, Edwardsiella, Enterobacter, Hafnia*, *Klebsiella, Morganella*, *Proteus*, *Providencia*, *Serratia,* and *Yersinia,* Pseudomonadaceae, in particular *Pseudomonas, Burkholderia, Stenotrophornonas, Shewanella, Sphingomonas* and *Comamonas*, *Neisseria, Moraxella, Vibrio, Aeromonas, Brucella, Fraucisella, Bordetella, Legionella*, *Bartonella*, *Coxiella, Haemophilus*, *Pasteurella*, *Mannheimia*, *Actinobacillus*, *Gardnerella*, Spirochaetaceae, in particular *Treponema* and *Borrelia,*
Leptospiraceae, *Campylobacter*, *Helicobacter, Spirillum*, *Streptobacillus*, Bacteroidaceae, in particular *Bacteroides, Fusobacterium*, *Prevotella* and *Porphyromonas,* and *Acinetobacter,* in particular A. *baumanii*; and wherein the Gram-positive bacteria are selected from the group consisting of *Listeria monocytogenes, Staphylococcus aureus, Enterococcus faecalis*, *Enterococcus faecium, Streptococcus pneumoniae, Streptococcus pyogenes, Streptococcus mutans, Streptococcus equi, Clostridium difficile, Clostridium botulinum*, *Clostridium tetani*, *Clostridium perfringens, Bacillus anthracis*, *Bacillus cereus, Propionibacterium acnes, Mycobacterium avium*, *Mycobacterium tuberculosis*, *Corynebacterium diphteriae, Mycoplasma pneumoniae, Actinomyces.*

4. The fusion protein according to any of the preceding claims, wherein said fusion protein exhibits an amino acid sequence according to SEQ ID NO: 74, 75, 127, 128, 139-141 or 147.

5. An isolated nucleic acid molecule encoding a fusion protein according to any of claims 1 to 4.

6. A vector comprising the nucleic acid molecule according to claim 5.

7. A host cell comprising the nucleic acid molecule according to claim 5 or the vector according to claim 6, in particular wherein the cell is a bacterial cell or a yeast cell.

8. The fusion protein according to any one of claims 1 to 4 for use as a medicament, as an *in vivo* diagnostic means, or for use in the treatment and/or prevention of a disorder, disease or condition associated with Gram-positive and/or Gram-negative bacteria, in particular wherein the disorder, disease or condition is caused by Gram-negative bacteria of bacterial groups, families, genera or species comprising strains pathogenic for humans or animals, in particular Enterobacteriaceae in particular *Escherichia,* especially *E. coli, Salmonella, Shigella, Citrobacter, Edwardsiella, Enterobacter*, *Hafnia, Klebsiella,* especially *K. pneumoniae, Morganella*, *Proteus, Providencia*, *Serratia, Yersinia*, Pseudomonadaceae in particular *Pseudomonas,* especially *P. aeruginosa, Borkholderia, Stenotrophomonas, Shewanella*, *Sphingomonas*, and *Comamonas*, *Neisseria, Moraxella, Vibrio, Aeromonas, Brucella, Francisella*, *Bordetella, Legionella, Bartonella, Coxiella, Haemophilus*, *Pasteurella*, *Mannheimia*, *Actinobacillus*, *Gardnerella*, Spirochaetaceae, in particular *Treponema and Borrelia,* Leptospiraceae, *Campylobacter, Helicobacter, Spirillum, Streptobacillus,* Bacteroidaceae, in particular *Bacteroides, Fusobacterium, Prevotella, Porphyromonas,* and *Acinetobacter,* especially A. *baumanii* or by Gram-positive bacteria of bacterial groups, families, genera or species comprising strains pathogenic for humans or animals in particular *Listeria monocytogenes, Staphylococcus aureus, Enterococcus faecalis*, *Enterococcus faecium, Streptococcus pneumoniae, Streptococcus pyogenes, Streptococcus mutans, Streptococcus equi, Clostridium difficile, Clostridium botulinum, Clostridium tetani, Clostridium perfringens, Bacillus anthracis, Bacillus cereus, Propionibacterium acnes, Mycobacterium avium, Mycobacterium tuberculosis, Corynebacterium diphteriae, Mycoplasma pneumoniae, Actinomyces.*

9. Use of the fusion protein according to any one of claims 1 to 4 as an ex vivo diagnostic means, cosmetic substance, ex vivo disinfectant or food additive.

10. A pharmaceutical composition comprising a fusion protein according to claim 1.

11. A method for the production of a fusion protein according to any one of claims 1 to 4 comprising culturing a host cell according to claim 7 under conditions supporting the expression of said fusion protein.

12. A fusion protein according to claim 1 for use in a method of treating a disorder, disease or condition caused by Gram-negative and/or Gram-positive bacteria in a subject, the method comprising administering to said subject said fusion protein according to claim 1.

13. The fusion protein for use according to claim 12, wherein the disorder, disease or condition is a bacterial infection caused by Gram-positive and/or Gram-negative bacteria.

14. A method for the removal, reduction and/or prevention of Gram-negative and/or Gram- positive bacterial contamination of foodstuff, of food processing equipment, of food processing plants, of surfaces coming into contact with foodstuff, of medical devices, of surfaces in hospitals and surgeries comprising contacting said foodstuff, food processing equipment, food processing plants, surfaces coming into contact with foodstuff, medical devices, or surfaces in hospitals with a fusion protein according to claim 1.

15. A method for detecting a bacterium in a sample from a subject or in a food or feed, or environmental sample comprising contacting said sample with a fusion protein according to claim 1.

16. Use of the fusion protein according to any one of claims 1 to 4:
i) for the treatment or prevention of Gram-negative and/or Gram-positive bacterial contamination of foodstuff, of food processing equipment, of food processing plants, of surfaces coming into contact with foodstuff, of medical devices, of surfaces in hospitals and surgeries; or
ii) as a diagnostic means in food or feed or environmental diagnostics.

## Patentansprüche

1. Ein Fusionsprotein, das ein Enzym mit der Aktivität, die Zellwand von gramnegativen Bakterien und/oder grampositiven Bakterien abzubauen, und mindestens zwei Peptidabschnitte umfasst, die an den N- oder C-Terminus des Enzyms fusioniert sind, wobei die Peptidabschnitte eine Länge von 5 bis 50 Aminosäureresten haben und wobei die Peptidabschnitte ein polykationisches Peptid und ein antimikrobielles Peptid sind; und
wobei das Enzym eine Aminosäuresequenz ausgewählt aus der Gruppe bestehend aus SEQ ID NO: 1 bis 18 aufweist,
wobei mindestens 70% der Aminosäurereste des polykationischen Peptids positiv geladene Aminosäurereste sind, insbesondere Lysin- und Argininreste, und
wobei das antimikrobielle Peptid eine Aminosäuresequenz aufweist, die ausgewählt ist aus der Gruppe bestehend aus SEQ ID NO: 44 bis 63 und 78 bis 98.

2. Das Fusionsprotein nach Anspruch 1, wobei das polykationische Peptide oder das antimikrobielle Peptid eine Länge von 6 bis 39 Aminosäureresten hat.

3. Das Fusionsprotein nach einem der vorhergehenden Ansprüche, wobei die gramnegativen Bakterien ausgewählt sind aus der Gruppe bestehend aus Enterobacteriaceae, insbesondere *Escherichia, Salmonella, Shigella, Citrobacter, Edwardsiella, Enterobacter, Hafnia, Klebsiella, Morganella, Proteus, Providencia, Serratia,* und *Yersinia,* Pseudomonadaceae, insbesondere *Pseudomonas*, *Burkholderia*, *Stenotrophomonas, Shewanella, Sphingomonas* und *Comamonas, Neisseria, Moraxella, Vibrio, Aeromonas, Brucella*, *Francisella, Bordetella, Legionella, Bartonella, Coxiella, Haemophilus*, *Pasteurella, Mannheimia, Actinobacillus*, *Gardnerella,* Spirochaetaceae, insbesondere *Treponema* und *Borrelia,* Leptospiraceae, *Campylobacter, Helicobacter, Spirillum*, *Streptobacillus,* Bacteroidaceae, insbesondere *Bacteroides, Fusobacterium*, *Prevotella* und *Porphyromonas,* und *Acinetobacter,* insbesondere A. *baumanii;* und wobei die grampositiven Bakterien ausgewählt sind aus der Gruppe bestehend aus *Listeria monocytogenes, Staphylococcus aureus*, *Enterococcus faecalis, Enterococcus faecium*, *Streptococcus pneumoniae*, *Streptococcus pyogenes, Streptococcus mutans*, *Streptococcus equi*, *Clostridium difficile, Clostridium botulinum*, *Clostridium tetani, Clostridium perfringens, Bacillus anthracis, Bacillus cereus*, *Propionibacterium acnes, Mycobacterium avium*, *Mycobacterium tuberculosis*, *Corynebacterium diphteriae, Mycoplasma pneumoniae*, *Actinomyces.*

4. Das Fusionsprotein nach einem der vorhergehenden Ansprüche, wobei das Fusionsprotein eine Aminosäuresequenz gemäß SEQ ID NO: 74, 75, 127, 128, 139-141 oder 147 aufweist.

5. Ein isoliertes Nukleinsäuremolekül, das ein Fusionsprotein nach einem der Ansprüche 1 bis 4 kodiert.

6. Ein Vektor umfassend ein Nukleinsäuremolekül nach Anspruch 5.

7. Eine Wirtszelle umfassend das Nukleinsäuremolekül nach Anspruch 5 oder den Vektor nach Anspruch 6, wobei die Zelle insbesondere eine Bakterienzelle oder Hefezelle ist.

8. Das Fusionsprotein nach einem der Ansprüche 1 bis 4 zur Verwendung als Medikament, als *in vivo* Diagnostikmittel, oder zur Verwendung bei der Behandlung und oder Prävention einer Störung, Erkrankung oder eines Zustands, der mit grampositiven und/oder gramnegativen Bakterien in Zusammenhang steht, insbesondere wenn die Störung, die Erkrankung oder der Zustand von gramnegativen Bakterien von Bakteliengruppen, -familien, -gattungen oder -arten verursacht wird, die Stämme umfassen, die pathogen für Menschen oder Tiere sind, insbesondere Enterobacteriaceae, insbesondere *Escherichia,* vorzugsweise *E. coli, Salmonella, Shigella, Citrobacter, Edwardsiella, Enterobacter, Hafnia, Klebsiella,* vorzugsweise *K. pneumoniae*, *Morganella, Proteus*, *Providencia, Serratia, Yersinia,* Pseudomonadaceae, insbesondere *Pseudomonas*, vorzugsweise P. *aeruginosa, Burkholderia*, *Stenotrophomonas, Shewanella, Sphingomonas* und *Comamonas, Neisseria, Moraxella, Vibrio, Aeromonas, Brucella*, *Francisella, Bordetella, Legionella, Bartonella, Coxiella, Haemophilus*, *Pasteurella, Mannheimia, Actinobacillus*, *Gardnerella,* Spirochaetaceae, insbesondere *Treponema* und *Borrelia,* Leptospiraceae, *Campylobacter, Helicobacter, Spirillum*, *Streptobacillus*, Bacteroidaceae, insbesondere *Bacteroides, Fusobacterium*, *Prevotella* und *Porphyromonas,* und *Acinetobacter,* vorzugsweise A. *baumanii* oder von grampositiven Bakterien von Bakteriengruppen, -familien, -gattungen oder -arten verursacht wird, die Stämme umfassen, die pathogen für Menschen oder Tiere sind, insbesondere *Listeria monocytogenes, Staphylococcus aureus*, *Enterococcus faecalis, Enterococcus faecium, Streptococcus pneumoniae, Streptococcus pyogenes, Streptococcus mutans*, *Streptococcus equi*, *Clostridium difficile, Clostridium botulinum*, *Clostridium tetani, Clostridium perfringens, Bacillus anthracis*, *Bacillus cereus*, *Propionibacterium acnes, Mycobacterium avium*, *Mycobacterium tuberculosis*, *Corynebacterium diphteriae, Mycoplasma pneumoniae, Actinomyces.*

9. Verwendung eines Fusionsproteins nach einem der Ansprüche 1 bis 4 als ex vivo Diagnostikmittel, Kosmetikum, ex vivo Desinfektionsmittel oder Nahrungsmittelzusatzstoff.

10. Eine pharmazeutische Zusammensetzung umfassend ein Fusionsprotein nach Anspruch 1.

11. Ein Verfahren zur Herstellung eines Fusionsproteins nach einem der Ansprüche 1 bis 4 umfassend das Kultivieren einer Wirtszelle nach Anspruch 7 unter Bedingungen, die die Expression des Fusionsproteins erlauben.

12. Ein Fusionsprotein nach Anspruch 1 zur Verwendung in einem Verfahren zur Behandlung einer Störung, Erkrankung oder eines Zustands in einem Patienten, der von gramnegativen und/oder grampositiven Bakterien verursacht wird, wobei das Verfahren das Verabreichen des Fusionsproteins nach Anspruch 1 an den Patienten umfasst.

13. Das Fusionsprotein zur Verwendung nach Anspruch 12, wobei die Störung, die Erkrankung oder der Zustand eine bakterielle Infektion ist, die von grampositiven und/oder gramnegativen Bakterien verursacht wird.

14. Ein Verfahren zur Entfernung, Verringerung und/oder Prävention gramnegativer und/oder grampositiver bakterieller Kontamination von Nahrungsmitteln, von nahrungsmittelverarbeitenden Gerätschaften, von nahrungsmittelverarbeitenden Fabriken, von mit Nahrungsmitteln in Berührung kommenden Oberflächen, von medizinischen Geräten, von Oberflächen in Krankenhäusern und Arztpraxen, umfassend das in Kontakt bringen der Nahrungsmittel, der nahrungsmittelverarbeitenden Gerätschaften, der nahrungsmittelverarbeitenden Fabriken, der mit Nahrungsmitteln in Berührung kommenden Oberflächen, der medizinischem Geräte, der Oberflächen in Krankenhäusern mit einem Fusionsprotein nach Anspruch 1.

15. Ein Verfahren zum Nachweis eines Bakteriums in einer Probe eines Patienten oder in einem Nahrungsmittel oder Futtermittel, oder in einer Umweltprobe, umfassend das in Kontakt bringen der Probe mit einem Fusionsprotein nach Anspruch 1.

16. Verwendung eines Fusionsproteins nach einem der Ansprüche 1 bis 4:
i) zur Behandlung oder Prävention gramnegativer und/oder grampositiver bakterieller Kontamination von Nahrungsmitteln, von nahrungsmittelverarbeitenden Gerätschaften, von nahrungsmittelverarbeitenden Fabriken, von mit Nahrungsmitteln in Berührung kommenden Oberflächen, von medizinischen Geräten, von Oberflächen in Krankenhäusern und Arztpraxen; oder
ii) als Diagnostikmittel in der Nahrungsmittel- oder Futtermittel- oder Umweltdiagnostik.

## Revendications

1. Protéine de fusion composée d'une enzyme ayant une activité de dégradation de la paroi cellulaire de bactéries à Gram négatif et/ou de bactéries à Gram positif et d'au moins deux fragments peptidiques fusionnés à l'extrémité N- ou C- terminale de l'enzyme, dans laquelle les fragments peptidiques ont une longueur de 5 à 50 résidus d'acides aminés et dans laquelle les fragments peptidiques sont un peptide polycationique et un peptide antimicrobien ; et
dans laquelle l'enzyme présente une séquence d'acides aminés choisie dans le groupe constitué par SEQ ID NO: 1 à 18,
dans laquelle au moins 70 % des résidus d'acides aminés du peptide polycationique sont des résidus d'acides aminés à charge positive, en particulier des résidus lysine et/ou arginine, et
dans laquelle le peptide antimicrobien présente une séquence d'acides aminés choisie dans le groupe constitué par SEQ ID NO: 44 à 63 et 78 à 98.

2. Protéine de fusion selon la revendication 1, dans laquelle le peptide polycationique ou le peptide antimicrobien a une longueur de 6 à 39 résidus d'acides aminés.

3. Protéine de fusion selon l'une quelconque des revendications précédentes, dans laquelle les bactéries à Gram négatif sont choisies dans le groupe constitué par les Enterobacteriaceae,
en particulier *Escherichia, Salmonella, Shigella, Citrobacter, Edwardsiella, Enterobacter, Hafnia, Klebsiella, Morganella, Proteus*, *Providencia, Serratia,* et *Yersinia,* les Pseudomonadaceae, en particulier *Pseudomonas*, *Burkholderia, Stenotrophomonas, Shewanella, Sphingomonas* et *Comamonas, Neisseria, Moraxella, Vibrio, Aeromonas, Brucella*, *Francisella, Bordetella, Legionella, Bartonella, Coxiella, Haemophilus*, *Pasteurella, Mannheimia, Actinobacillus*, *Gardnerella,* les Spirochaetaceae, en particulier *Treponema* et *Borrelia,* les Leptospiraceae, *Campylobacter, Helicobacter, Spirillum*, *Streptobacillus,* les Bacteroidaceae, en particulier *Bacteroides, Fusobacterium*, *Prevotella* et *Porphyromonas,* et *Acinetobacter,* en particulier A. *baumanii ;* et dans laquelle les bactéries à Gram positif sont choisies dans le groupe constitué par *Listeria monocytogenes, Staphylococcus aureus*, *Enterococcus faecalis, Enterococcus faecium*, *Streptococcus pneumoniae*, *Streptococcus pyogenes, Streptococcus mutans, Streptococcus equi*, *Clostridium difficile, Clostridium botulinum*, *Clostridium tetani, Clostridium perfringens, Bacillus anthracis, Bacillus cereus*, *Propionibacterium acnes, Mycobacterium avium*, *Mycobacterium tuberculosis, Corynehacterium diphteriae, Mycoplasma pneumoniae, Actinomyces.*

4. Protéine de fusion selon l'une quelconque des revendications précédentes, dans laquelle ladite protéine de fusion présente une séquence d'acides aminés selon SEQ ID NO:74, 75, 127, 128, 139-141 ou 147.

5. Molécule d'acide nucléique isolée codant pour une protéine de fusion selon l'une quelconque des revendications 1 à 4.

6. Vecteur comprenant la molécule d'acide nucléique selon la revendication 5.

7. Cellule hôte comprenant la molécule d'acide nucléique selon la revendication 5 ou le vecteur selon la revendication 6, en particulier dans laquelle la cellule est une cellule bactérienne ou une cellule de levure.

8. Protéine de fusion selon l'une quelconque des revendications 1 à 4 pour son utilisation en tant que médicament, en tant que moyen de diagnostic *in vivo,* ou pour son utilisation dans le traitement et/ou la prévention d'un trouble, d'une maladie ou d'une affection associé(e) à des bactéries à Gram positif et/ou à Gram négatif, en particulier dans laquelle le trouble, la maladie ou l'affection est provoqué(e) par des bactéries à Gram négatif appartenant à des groupes, des familles, des genres ou des espèces bactérien(ne)s comprenant des souches pathogènes pour l'homme ou l'animal, en particulier les Enterobacteriaceae, en particulier *Escherichia,* notamment *E. coli, Salmonella, Shigella, Citrobacter, Edwardsiella, Enterobacter, Hafnia, Klebsiella,* notamment *K. pneumoniae, Morganella, Proteus*, *Providencia, Serratia, Yersinia,* les Pseudomonadaceae, en particulier *Pseudomonas*, notamment *P. aeruginosa, Burkholderia*, *Stenotrophomonas, Shewanella, Sphingomonas* et *Comamonas, Neisseria, Moraxella, Vibrio, Aeromonas, Brucella*, *Francisella, Bordetella, Legionella, Bartonella, Coxiella, Haemophilus*, *Pasteurella, Mannheimia, Actinobacillus*, *Gardnerella,* les Spirochaetaceae, en particulier *Treponema* et *Borrelia,* les Leptospiraceae, *Campylobacter, Helicobacter, Spirillum*, *Streptobacillus,* les Bacteroidaceae, en particulier *Bacteroides, Fusobacterium*, *Prevotella, Porphyromonas,* et *Acinetobacter,* notamment A. *baumanii* ou par des bactéries à Gram positif appartenant à des groupes, des familles, des genres ou des espèces bactérien(ne)s comprenant des souches pathogènes pour l'homme ou l'animal, en particulier, *Listeria monocytogenes, Staphylococcus aureus*, *Enterococcus faecalis, Enterococcus faecium*, *Streptococcus pneumoniae*, *Streptococcus pyogenes, Streptococcus mutans, Streptococcus equi*, *Clostridium difficile, Clostridium botulinum*, *Clostridium tetani, Clostridium perfringens, Bacillus anthracis*, *Bacillus cereus, Propionibacterium acnes, Mycobacterium avium*, *Mycobacterium tuberculosis*, *Corynehacterium diphteriae, Mycoplasma pneumoniae*, *Actinomyces.*

9. Utilisation de la protéine de fusion selon l'une quelconque des revendications 1 à 4 à titre de moyen de diagnostic *ex vivo,* de substance cosmétique, de désinfectant ex vivo ou d'additif alimentaire.

10. Composition pharmaceutique comprenant une protéine de fusion selon la revendication 1.

11. Méthode de production d'une protéine de fusion selon l'une quelconque des revendications 1 à 4 comprenant la culture d'une cellule hôte selon la revendication 7 dans des conditions supportant l'expression de ladite protéine de fusion.

12. Protéine de fusion selon la revendication 1 pour son utilisation dans une méthode destinée à traiter un trouble, une maladie ou une affection provoqué(e) par des bactéries à Gram négatif et/ou à Gram positif chez un sujet, la méthode comprenant l'administration audit sujet de ladite protéine de fusion selon la revendication 1.

13. Protéine de fusion pour son utilisation selon la revendication 12, dans laquelle le trouble, la maladie ou l'affection est une infection bactérienne provoquée par des bactéries à Gram positif et/ou à Gram négatif.

14. Méthode pour éliminer, réduire et/ou prévenir la contamination par des bactéries à Gram négatif et/ou à Gram positif de denrées alimentaires, d'un équipement de transformation des aliments, d'usines de transformation des aliments, de surfaces entrant en contact avec des denrées alimentaires, de dispositifs médicaux, de surfaces dans des hôpitaux et des blocs opératoires comprenant la mise en contact desdites denrées alimentaires, dudit équipement de transformation des aliments, desdites usines de transformation des aliments, desdites surfaces entrant en contact avec des denrées alimentaires, desdits dispositifs médicaux, desdites surfaces dans des hôpitaux avec une protéine de fusion selon la revendication 1.

15. Méthode pour détecter une bactérie dans un échantillon provenant d'un sujet ou dans un aliment ou aliment pour animaux, ou un échantillon environnemental comprenant la mise en contact dudit échantillon avec une protéine de fusion selon la revendication 1.

16. Utilisation de la protéine de fusion selon l'une quelconque des revendications 1 à 4 :
i) pour le traitement ou la prévention de la contamination par des bactéries à Gram négatif et/ou à Gram positif de denrées alimentaires, d'un équipement de transformation des aliments, d'usines de transformation des aliments, de surfaces entrant en contact avec des denrées alimentaires, de dispositifs médicaux, de surfaces dans des hôpitaux et des blocs opératoires ; ou
ii) en tant que moyen de diagnostic dans des diagnostics portant sur des aliments ou des aliments pour animaux ou l'environnement.
